(19)

Europäisches
Patentamt
European
Patent Office
Office européen
des brevets

(11) **EP 1 930 426 A1**

(12) **EUROPEAN PATENT APPLICATION**
published in accordance with Art. 153(4) EPC

(43) Date of publication:
**11.06.2008 Bulletin 2008/24**

(51) Int Cl.:
*C12N 15/00* (2006.01)   *C12N 15/09* (2006.01)
*C12Q 1/68* (2006.01)   *G01N 33/53* (2006.01)
*G01N 33/574* (2006.01)

(21) Application number: **06821799.1**

(22) Date of filing: **01.09.2006**

(86) International application number:
**PCT/JP2006/317380**

(87) International publication number:
**WO 2007/026896 (08.03.2007 Gazette 2007/10)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **02.09.2005 JP 2005255499**
**01.11.2005 JP 2005318589**

(71) Applicants:
• **TORAY INDUSTRIES, INC.**
**Tokyo 103-8666 (JP)**
• **Kyoto University**
**Kyoto-shi, Kyoto 606-8501 (JP)**

(72) Inventors:
• **KOZONO, Satoko**
**Kamakura-shi Kanagawa 248-0034 (JP)**
• **AKIYAMA, Hideo**
**Fujisawa-shi, Kanagawa 251-0033 (JP)**
• **MYOMOTO, Akira**
**Otsu-shi, Shiga 520-0842 (JP)**

• **TANAKA, Yoshinori**
**Kamakura-shi, Kanagawa 248-0036 (JP)**
• **JUNG, Giman**
**Kamakura-shi, Kanagawa 248-0034 (JP)**
• **NOBUMASA, Hitoshi**
**Otsu-shi, Shiga 520-0043 (JP)**
• **NOMURA, Osamu**
**Kamakura-shi, Kanagawa 248-0034 (JP)**
• **OGAWA, Osamu**
**Kyoto-shi, Kyoto 606-8501 (JP)**
• **NAKAMURA, Eijiro**
**Kyoto-shi, Kyoto 606-8501 (JP)**
• **TSUJIMOTO, Gozoh**
**Kyoto-shi, Kyoto 606-8501 (JP)**

(74) Representative: **Oser, Andreas et al**
**Prüfer & Partner GbR**
**Patentanwälte**
**Sohnckestrasse 12**
**81479 München (DE)**

(54) **COMPOSITION AND METHOD FOR DIAGNOSING KIDNEY CANCER AND ESTIMATING KIDNEY CANCER PATIENT S PROGNOSIS**

(57)   This invention relates to a composition, kit, DNA chip, and use thereof for detecting, diagnosing, and predicting metastasis of kidney cancer and/or for predicting the prognosis for kidney cancer, comprising one or a plurality of polynucleotides selected from the group consisting of polynucleotides, mutants thereof or fragments thereof, the expression levels of which vary in kidney cancer cells from a patient with a poor prognosis when compared with that in kidney cancer cells from a patient with a good prognosis; or antibodies or fragments thereof that bind specifically to polypeptides, mutants thereof or fragments thereof, the expression levels of which vary in the similar manner.

Fig. 2

Prediction of prognosis for kidney cancer patient/number of genes necessary for detecting metastasis of kidney cancer

EP 1 930 426 A1

**Description**

FIELD OF THE INVENTION

The present invention relates to a composition for identifying (or diagnosing) a disease that is useful for predicting the prognosis for a subject and/or for predicting or detecting metastasis of kidney cancer, to a method for predicting or identifying the prognosis for a subject and/or metastasis of kidney cancer using the composition, and to a kit for predicting the prognosis for a subject and/or metastasis of kidney cancer using the composition.

BACKGROUND OF THE INVENTION

The kidney is an important urinary organ that plays a key role in eliminating waste products from the body by filtering the blood and generating urine. The kidney is also important as an endocrine organ that produces hormones such as angiotensin, which controls the blood pressure, or erythropoietin, which is an erythropoietic factor.

Tumors that develop in the kidney are classified into: kidney cell cancer, which occurs in adults; Wilms' tumor, which occurs in children; and sarcoma, which is a rare form of tumor. Hereafter, malignant kidney cell cancer, which has the highest incidence, is referred to as "kidney cancer." In Japan, the frequency of kidney cancer development is approximately 2.5 people per 100,000 people, the male to female ratio thereof is 2 to 3:1, and men are more likely to develop kidney cancer. Among malignant urologic tumors, kidney cancer has the highest frequency after prostate cancer and bladder cancer.

As risk factors for kidney cancer, genetic factors are known. In general, smoking, excessive fat intake, and the like are known as risk factors. Also, such tumors frequently develop in long-term dialysis patients.

When the maximal diameter of the kidney tumor is not greater than 5 cm, the patients hardly have any subjective symptoms, and such tumors are often found via CT scanning at the time of physical examination. In the case of large-size tumors, hematuria, abdominal tumors, pain, or other symptoms are observed. As systemic symptoms, fever onset, weight loss, anemia, or other symptoms may occur, and erythrocytosis, elevated blood pressure, hypercalcemia, or other disease may be caused by endocrine factors. Also, spreading kidney cancer in the postcaval vein may occur in a varicose on the abdominal surface or in the testicular varicocele. Approximately 20% of kidney cancer cases are detected upon lung or bone metastasis. Kidney tumors are likely to spread in the vein and easily metastasize to other organs.

Examples of methods for detecting kidney cancer include echography, CT scanning, and angiography; however, specific biochemical markers are not yet known. Thus, examination with the use of equipment is necessary.

Also, kidney cancer can be further pathologically classified. Clear cell kidney cancer, which accounts for 90% of cases, is known to develop as a result of defects of the von-Hippel-Lindau (VHL) genes, which are cancer suppressive genes (Latif, F. et al., Science, 1993, vol. 260, pp. 1317-1320). VHL gene defects activate transcription of hypoxia-induced genes via disturbance of HIF-$\alpha$/VHF aggregation (Maxwell, P. et al., Nature, 1999, vol. 399, pp. 271-275) and also enhance the expression of genes such as VEGF and TGF$\beta$.

The main treatment for kidney cancer is surgical treatment. Regardless of the disease stage, partial or total removal of the kidney is the most common method when possible. Even if metastasis has already occurred, surgical removal of the kidney may be considered. In addition to surgical treatment, arterial embolization of the renal artery is possible. This method may be employed when surgical removal is unfeasible or before surgical removal of large tumors.

Because of the recent development of image diagnostic technologies, a very small size of kidney cancer has become detectable at an early stage. Through early-stage treatment, 90% (or more) of cancer patients can be treated. Since the treatment results for large tumors of 5 cm or greater or for metastatic tumors are poor (5-year survival rates for kidney cell cancer patients overall are approximately 50% to 60%), it is crucial to conduct high-throughput testing and diagnosis of the presence of tumors or tumor metastasis, to predict the prognosis, and to compose regimen or treatment plans.

As a method for detecting or diagnosing human kidney cancer, a method wherein differences in gene expression levels are employed is disclosed in WO 2005/024603. As proteins that are known to increase in human kidney cancer, PDE8B (WO 2004/042077), FLOT1 (WO 2004/048933), CD5 (Nagase, Y. et al., the Japanese Journal of Urology, 1991, vol. 82, pp. 1781-1789), and ECM1 (US Patent No. 6,303,765) are known, in addition to MMP2, which is considered to enhance cancer cell motility by degrading the extracellular matrix (Lein, M. et al., International Journal of Cancer, 2000, vol. 85, pp. 801-804), and TNFRSF7, whose expression level is known to increase in the case of renal disorder (Nakatsuji, T., Clinical and Experimental Medicine, 2003, vol. 2, pp. 192-196). Their specificities, however, are not relatively high, and a highly sensitive method for detecting kidney cancer based only on the expression levels of such proteins has not yet been put to clinical applications. Further, MCM3AP (JP Patent Publication (kohyo) No. 2005-520536 (A)), KRT19 (JP Patent Publication (kohyo) No. 2005-507997 (A)), SLK4 (WO 2002/06339), C5P1 (JP Patent Publication (kohyo) No. 2004-518402 (A)), FGF2 (Miyake, H. et al., 1996, Cancer Research, vol. 56, pp. 2440-2445), MMP14 (Kitagawa, Y. et al., 1999, Journal of Urology, vol. 162, pp. 905-909), ERBB2 (Freeman, M. R. et al., 1989, Cancer Research, vol.

49, pp. 6221-6225), and the like are deduced as tumor-associated markers for cancer, including kidney cancer.

DISCLOSURE OF THE INVENTION

Problems to be Solved by the Invention

As described above, gene expression markers for kidney cancer are well known; however, diagnostic markers capable of predicting metastasis and markers for predicting the prognosis are hardly known. Since existing markers have poor specificity and/or sensitivity and an effective method for detecting such markers from biological samples has not yet been established, such existing markers have not yet been clinically used in general. Thus, markers for kidney cancer with higher specificity and sensitivity have been desired.

If a plurality of effective markers for kidney cancer are discovered, remarkable progress can be expected in the treatment of kidney cancer or metastatic kidney cancer. In particular, many metastatic kidney cancer patients have poor prognoses. If the occurrence of metastasis is discovered at the time of diagnosis, more effective treatment can be provided to improve prognosis.

An object of the invention is to provide a composition, kit, or DNA chip for identifying a disease, which is useful for diagnosing kidney cancer, for diagnosing the metastasis of kidney cancer (or prognosis), and for treating kidney cancer.

Another object of the invention is to provide a method for detecting, identifying, or predicting the presence or metastasis of kidney cancer using the composition, kit, or DNA chip.

Means for Solving Problems

Markers may be searched for by a method wherein the prognosis for kidney cancer patients is observed and the gene expression levels or the protein expression levels in the kidney cancer cells from patients with good prognoses and in the kidney cancer cells from patients with poor prognoses or the amounts of metabolites of such cells are compared by some means; a method wherein the amounts of genes, proteins, or metabolites in body fluids and tissues containing the kidney cancer cells from patients with good prognoses or tissues containing the kidney cancer cells from patients with poor prognoses; or other methods.

In recent years, analysis of gene expression levels using DNA arrays has been commonly used as a method for searching for such markers. DNA arrays comprise probes having nucleotide sequences corresponding to several hundreds to several tens of thousands of genes immobilized thereon. By applying analyte samples to DNA arrays, the genes in the samples bind to probes, and the amount of binding is determined by some means. Thus, the amount of genes in the analyte samples can be determined. The genes corresponding to the probes to be immobilized on the DNA arrays can be freely selected, kidney cancer cells from patients with good prognoses and poor prognoses can be used as analyte samples, and the gene expression levels in the samples may be compared. Thus, genes that can become markers for predicting the metastasis of kidney cancer and/or the prognosis for kidney cancer patients can be deduced.

In order to overcome the aforementioned problem, we have analyzed, with the use of DNA arrays, the gene expression levels in the kidney cancer tissue from patients with good prognoses and in the kidney cancer tissue from patients with poor prognoses. Further, we have selected the genes that can discriminate the kidney cancer tissue from a patient with good prognosis from the kidney cancer tissue from a patient with poor prognosis, and/or that can determine the presence or absence of metastasis of kidney cancer by using the effect of the gene expression levels determined using DNA arrays on changes in survival rates for patients with the elapse of time after the surgery as an indication. This has led to the completion of the present invention.

Summary of the Invention

The present invention includes the following characteristics.

(1) A composition for detecting, identifying, or predicting the presence or metastasis of kidney cancer in a subject *in vitro* comprising one or more probes selected from the probes of the following group I and/or group II:

group I: polynucleotides consisting of:

(a) a polynucleotide consisting of a nucleotide sequence as shown in any of SEQ ID NOS: 1 to 10, 12 to 20, and 22 to 47, a mutant thereof, or a fragment comprising at least 15 continuous nucleotides thereof,
(b) a polynucleotide comprising a nucleotide sequence as shown in any of SEQ ID NOS: 1 to 10, 12 to 20, and 22 to 47,
(c) a polynucleotide consisting of a nucleotide sequence complementary to a nucleotide sequence as shown in any of SEQ ID NOS: 1 to 10, 12 to 20, and 22 to 47, a mutant thereof, or a fragment comprising at least 15 continuous nucleotides thereof,

(d) a polynucleotide comprising a nucleotide sequence complementary to a nucleotide sequence as shown in any of SEQ ID NOS: 1 to 10, 12 to 20, and 22 to 47, and

(e) a polynucleotide hybridizing under stringent conditions to any of the polynucleotides (a) to (d), or a fragment comprising at least 15 continuous nucleotides thereof; and

group II: antibodies, fragments thereof or chemically modified derivatives thereof consisting of:

(f) an antibody specifically binding to at least one of a polypeptide comprising an amino acid sequence encoded by a nucleotide sequence as shown in any of SEQ ID NOS: 1 to 10, 12 to 20, and 22 to 47, a polypeptide having an amino acid sequence as shown in any of SEQ ID NOS: 101 to 110, 112 to 120, and 122 to 147, a mutant of the polypeptide, and a fragment of the polypeptide; a fragment of the antibody; or a chemically modified derivative of the antibody,

(g) an antibody specifically binding to at least one of a polypeptide having an amino acid sequence as shown in any of SEQ ID NOS: 151, 153, and 155 to 160, a mutant of the polypeptide, and a fragment of the polypeptide; a fragment of the antibody; or a chemically modified derivative of the antibody, and

(h) an antibody specifically binding to at least one of a polypeptide having an amino acid sequence as shown in any of SEQ ID NOS: 161 to 190, a mutant of the polypeptide, and a fragment of the polypeptide; a fragment of the antibody; or a chemically modified derivative of the antibody.

(2) The composition according to (1) above, wherein each of the probes of group I and group II (f) is capable of detecting, identifying, or predicting the presence or metastasis of kidney cancer.

(3) The composition according to (1) above, wherein each of the probes of group II (g) and (h) is capable of detecting, identifying, or predicting the presence of kidney cancer.

(4) The composition according to (1) above, wherein the polynucleotide is DNA or RNA.

(5) The composition according to (1) above, wherein the fragments of group I are each a polynucleotide comprising at least 60 continuous nucleotides.

(6) The composition according to (1) above, wherein the fragments of group I are each a polynucleotide comprising a nucleotide sequence as shown in any of SEQ ID NOS: 51 to 60, 62 to 70, and 72 to 97 in the nucleotide sequence as shown in any of SEQ ID NOS: 1 to 10, 12 to 20, and 22 to 47 or a polynucleotide comprising a nucleotide sequence complementary thereto.

(7) The composition according to (1) above, wherein the fragments of group I are each a polynucleotide comprising a nucleotide sequence as shown in any of SEQ ID NOS: 51 to 60, 62 to 70, and 72 to 97 or a nucleotide sequence complementary thereto.

(8) The composition according to (1) above, which further comprises, as a probe, one or more polynucleotides selected from among a polynucleotide consisting of the nucleotide sequence as shown in any of SEQ ID NOS: 11, 21, and 48 to 50, a polynucleotide consisting of a complementary sequence thereof, a polynucleotide hybridizing under stringent conditions to said polynucleotide, or a fragment comprising at least 15 continuous nucleotides thereof, in addition to the probe or probes of group I.

(9) The composition according to (8) above, wherein the fragment is a polynucleotide comprising at least 60 continuous nucleotides.

(10) The composition according to (8) above, wherein the fragment is a polynucleotide comprising the nucleotide sequence as shown in any of SEQ ID NOS: 61, 71, and 98 to 100 in the nucleotide sequence as shown in any of SEQ ID NOS: 11, 21, and 48 to 50 or a polynucleotide comprising a nucleotide sequence complementary thereto.

(11) The composition according to (8) above, wherein the fragment is a polynucleotide comprising the nucleotide sequence as shown in any of SEQ ID NOS: 61, 71, and 98 to 100 or a nucleotide sequence complementary thereto.

(12) The composition according to (1) above, which further comprises an antibody that binds specifically to at least one of a polypeptide as shown in any of SEQ ID NOS: 111, 121, and 148 to 150, a mutant of the polypeptide, and a fragment of the polypeptide; a fragment of the antibody; or a chemically modified derivative of the antibody, in addition to the probe or probes of group II (f).

(13) The composition according to (1) above, which further comprises an antibody that binds specifically to at least one of a polypeptide comprising an amino acid sequence as shown in any of SEQ ID NOS: 152, 154, and 191, a mutant of the polypeptide, and a fragment of the polypeptide; a fragment of the antibody; or a chemically modified derivative of the antibody, in addition to the probe or probes of group II (g).

(14) The composition according to (1) above, which further comprises an antibody that binds specifically to at least one of a polypeptide comprising an amino acid sequence as shown in any of SEQ ID NOS: 192 to 197, a mutant of the polypeptide, and a fragment of the polypeptide; a fragment of the antibody; or a chemically modified derivative of the antibody, in addition to the probe or probes of group II (h).

(15) The composition according to any of (1), (12), (13), or (14), wherein the fragment of the polypeptide or a mutant thereof comprises an epitope having at least 7 amino acids.

(16) The composition according to any of (1), (12), (13), or (14) above, wherein each of the antibodies is a polyclonal antibody, a monoclonal antibody, a synthetic antibody, a recombinant antibody, a polyspecific antibody, or a single-chain antibody.

(17) The composition according to (1) above, which comprises at least two probes selected from the probes of group I or group II (f), (g), or (h) in combination.

(18) A kit for detecting, identifying, or predicting the presence or metastasis of kidney cancer in a subject *in vitro,* comprising one or more probes selected from the probes of group I or group II (f), (g), or (h) as defined in (1) above.

(19) The kit according to (18), which further comprises, as a probe, a polynucleotide consisting of the nucleotide sequence as shown in any of SEQ ID NOS: 11, 21, and 48 to 50, a polynucleotide consisting of a complementary sequence thereof, a polynucleotide hybridizing under stringent conditions to said polynucleotide, or a fragment comprising at least 15 continuous nucleotides thereof.

(20) The kit according to (18) or (19) above, wherein the probe is a polynucleotide consisting of the nucleotide sequence as shown in any of SEQ ID NOS: 1 to 10, 11, 12 to 20, 21, 22 to 47, and 48 to 50, a polynucleotide consisting of a complementary sequence thereof, a polynucleotide hybridizing under stringent conditions to said polynucleotide, or a fragment comprising at least 15 continuous nucleotides thereof.

(21) The kit according to any of (18) to (20) above, wherein the fragment of group I is a polynucleotide comprising at least 60 continuous nucleotides.

(22) The kit according to any of (18) to (20) above, wherein the fragment of group I is a polynucleotide comprising the nucleotide sequence as shown in any of SEQ ID NOS: 51 to 60, 61, 62 to 70, 71, 72 to 97, and 98 to 100 and comprising at least 60 continuous nucleotides in the nucleotide sequence as shown in any of SEQ ID NOS: 1 to 10, 11, 12 to 20, 21, 22 to 47, and 48 to 50 or a polynucleotide comprising a nucleotide sequence complementary thereto.

(23) The kit according to any of (18) to (20) above, wherein the fragment of group I is a polynucleotide comprising a nucleotide sequence as shown in any of SEQ ID NOS: 51 to 60, 61, 62 to 70, 71, 72 to 97, and 98 to 100 or a nucleotide sequence complementary thereto.

(24) The kit according to any of (18) to (20) above, wherein the fragment of group I is a polynucleotide comprising a nucleotide sequence as shown in any of SEQ ID NOS: 51 to 60, 61, 62 to 70, 71, 72 to 97, and 98 to 100.

(25) The kit according to (18) above, which comprises at least two or all polynucleotides each comprising a nucleotide sequence as shown in any of SEQ ID NOS: 51 to 60, 61, 62 to 70, 71, 72 to 97, and 98 to 100 or a complementary sequence of any thereof.

(26) The kit according to (18) above, which further comprises an antibody that binds specifically to at least one of a polypeptide as shown in any of SEQ ID NOS: 111, 121, and 148 to 150, a mutant of the polypeptide, and a fragment of the polypeptide; a fragment of the antibody; or a chemically modified derivative of the antibody, in addition to a probe or probes of group II (f).

(27) The kit according to (18) above, which further comprises an antibody that binds specifically to at least one of a polypeptide as shown in any of SEQ ID NOS: 152, 154, and 191, a mutant of the polypeptide, and a fragment of the polypeptide; a fragment of the antibody; or a chemically modified derivative of the antibody, in addition to a probe or probes of group II (g).

(28) The kit according to (18) above, which further comprises an antibody that binds specifically to at least one of a polypeptide as shown in any of SEQ ID NOS: 192 to 197, a mutant of the polypeptide, and a fragment of the polypeptide; a fragment of the antibody; or a chemically modified derivative of the antibody, in addition to a probe or probes of group II (h).

(29) The kit according to (18) above, wherein the probes are packaged in different containers, alone or in combination.

(30) A DNA chip for detecting, identifying, or predicting the presence or metastasis of kidney cancer in a subject *in vitro,* comprising one or more probes selected from the probes of group I (a) to (e) as defined in (1) above.

(31) The DNA chip according to (30) above, which further comprises a polynucleotide consisting of the nucleotide sequence as shown in any of SEQ ID NOS: 11, 21, and 48 to 50, a mutant of the polypeptide, and/or a fragment thereof.

(32) The DNA chip according to (30) above, which comprises at least two or all of the polynucleotides comprising a nucleotide sequence as shown in any of SEQ ID NOS: 51 to 60, 61, 62 to 70, 71, 72 to 97, and 98 to 100 or complementary sequences thereto.

(33) A method for detecting, identifying, or predicting the presence or metastasis of kidney cancer *in vitro,* comprising using a probe or probes selected from the probes of group I and/or group II (f), (g), and (h) as defined in (1) above to measure *in vitro* the presence, exisiting amount, or expression level of one or more kidney cancer-associated target nucleic acids in a biological sample from a subject.

(34) The method according to (33) above, wherein the measurement is carried out using a DNA chip.

(35) The method according to (33) above, wherein the presence or metastasis of kidney cancer is detected, identified, or predicted using changes compared with a control sample as an indication.

(36) The method according to (33) above, wherein the measurement is carried out by an immunological method.

(37) The method according to (36) above, wherein the measurement by the immunological method is carried out using an antibody of group II (f), (g), or (h), a fragment thereof, or a chemically modified derivative thereof.

(38) The method according to (37) above, wherein the antibody, fragment, or chemically modified derivative is labeled.

(39) The method according to (33) above, wherein the biological sample is a kidney tissue or a kidney cell, blood, plasma, serum, or urine.

(40) A method for detecting, identifying, or predicting the presence or metastasis of kidney cancer *in vitro,* comprising measuring *in vitro* the presence, existing amount, or expression level of one or more kidney cancer-associated target nucleic acids in a biological sample from a subject using the composition according to (1) above, the kit according to (18) above, or the DNA chip according to (30) above.

(41) A method for predicting *in vitro* a patient's prognosis and/or metastasis of kidney cancer by comparing the expression level of a target nucleic acid in a biological sample of kidney cancer cells obtained from a subject with that of the target nucleic acid in kidney cancer cells obtained from a patient population with poor prognosis and/or that of the target nucleic acid in kidney cancer cells obtained from a patient population with good prognosis using a probe or probes selected from group I as defined in (1) above, or the composition according to (1) above, the kit according to (18) above, or the DNA chip according to (30) above comprising the probe or probes, wherein the target nucleic acid can be detected by the polynucleotide contained in the composition, kit, or DNA chip or a mutant or fragment of such polynucleotide, and, when the expression level of the target nucleic acid in the subject is different from that in the patient population with good prognosis and/or that in the patient population with poor prognosis, the subject is identified to have a poor or good prognosis and/or the metastasis of kidney cancer is determined to have or have not occurred.

(42) The method according to (41) above, which involves the use of a DNA chip.

(43) The method according to (41) or (42), which comprises the steps of:

(1) measuring *in vitro* expression levels of target nucleic acids in a plurality of biological samples that are known to be kidney cancer cells obtained from a patient with a poor prognosis and/or kidney cancer cells obtained from a patient with a good prognosis using a probe or probes selected from group I as defined in (1) above, the composition of (1) above, the kit of (18) above, or the DNA chip of (30) above comprising the probe or probes;

(2) preparing a discriminant, a support vector machine, using, as training samples, the expression levels of the target nucleic acids as determined in step (1);

(3) measuring *in vitro* expression levels of the target nucleic acids in a biological sample obtained from the kidney cancer cells of the subject in the same manner as in step (1); and

(4) assigning the expression levels of the target nucleic acids identified in step (3) to the discriminant prepared in step (2) and, based on the results obtained with the use of the discriminant, predicting the prognosis for a subject and/or determining whether or not kidney cancer of the subject is metastatic or nonmetastatic, provided that the target nucleic acids can be detected by the polynucleotides, mutants thereof or fragments thereof contained in the composition, kit, or DNA chip.

(44) Use of a probe or probes selected from the probes of group I and/or group II as defined in (1) above, or of the composition of (1) above, the kit of (18) above, or the DNA chip of (30) above comprising the probe or probes, in detecting, identifying, or predicting *in vitro* the presence or metastasis of kidney cancer in a biological sample from a subject.

Definition

The terms as used herein comprise the definitions as set forth below.

The term "probe" as used herein refers to a nucleic acid, an antibody, or an equivalent thereof, which is for detecting, identifying, or predicting the presence or metastasis of kidney cancer, and which is capable of binding to a particular gene that is an kidney cancer-associated marker, or to a polypeptide encoded thereby.

The meanings of terms such as nucleotide, polynucleotide, amino acid, peptide, polypeptide, and protein, and their abbreviations are in accordance with common usage in the art.

The term "polynucleotide" as used herein refers to a nucleic acid including each of RNA and DNA. Such DNA includes cDNA, genomic DNA, and synthetic DNA. Such RNA includes total RNA, mRNA, rRNA, and synthetic RNA. The term "polynucleotide" is used interchangeably with the term "nucleic acid."

The term "cDNA" as used herein refers to a full-length DNA strand of a sequence complementary to RNA resulting from gene expression, or a DNA fragment consisting of a partial sequence thereof. cDNA can be synthesized via reverse transcriptase-polymerase chain reaction (RT-PCR) using RNA as a template and a poly T primer.

The term "gene" as used herein refers to not only double-stranded DNA but also single-stranded DNA such as a plus-strand (or a sense strand) or a complementary strand (or an antisense strand) constituting double-stranded DNA. It is not particularly limited by the length of such strand. Accordingly, the term "gene" refers to any of double-stranded DNA (including human genomic DNA), single-stranded DNA (plus-strand) (including cDNA), single-stranded DNA having a sequence complementary to the plus-strand (complementary strand), and a fragment thereof, unless otherwise specified. Such "gene" includes not only a "gene" represented by a specific nucleotide sequence (or a SEQ ID NO.) but also another "gene" encoding a protein, which has a biological function equivalent to that of a protein encoded by said gene, such as a homolog, a mutant such as a splice mutant, and a derivative. Specific examples of the "genes" encoding such homolog, mutant, or derivative include "genes" each having a nucleotide sequence which hybridizes to a sequence complementary to a specific nucleotide sequence as shown in any of SEQ ID NOS: 1 to 50 under stringent conditions as described below.

Examples of human-derived protein homologs or genes encoding the same include proteins or genes derived from other organism species corresponding to the human proteins or human genes encoding the same. Such protein homologs or gene homologs can be identified by HomoloGene (http://www.ncbi.nlm.nih.gov/homoloGene/). Specifically, a certain human amino acid or nucleotide sequence can be subjected to the BLAST programs (Karlin, S. et al., Proceedings of the National Academic Sciences, U.S.A., 1993, vol. 90, pp. 5873-5877, http://www.ncbi.nlm.nih.gov/BLAST/) to obtain the accession number of the corresponding sequence (i. e., the sequence exhibiting the highest score, E-value 0, and identity 100%). Examples of the known BLAST programs include BLASTN (gene) and BLASTX (protein). When searching for a gene, for example, the accession number obtained from the above-mentioned BLAST search is inputted into the UniGene (http://www.ncbi.nlm.nih.gov/UniGene/), and the obtained UniGeneClusterID (the number identified with "Hs.") is then inputted into the HomoloGene. From the list that shows the correlation of gene homologs between the genes of other organism species and the human genes, a gene of the other organism species can be selected as a gene homolog corresponding to the human gene represented by a given nucleotide sequence. In this procedure, the FASTA program (http://www.ddbj.nig.ac.jp/search/fasta-e.html) may be used instead of the BLAST program.

Functional regions of "genes" are not limited, and examples thereof include expression-control regions, coding regions, and exon or intron regions.

The term "transcription product" as used herein refers to messenger RNA (mRNA) which is synthesized from the DNA sequence of a gene as a template. Messenger RNA is synthesized by binding of RNA polymerase to a site called promoter, which is located upstream of the gene of interest, and subsequently by binding of ribonucleotides to the 3' end so as to be complementary to the nucleotide sequence of DNA. Such messenger RNA contains not only the gene of interest but also a full-length sequence spanning from a transcription initiation site to the terminus of a poly A sequence including expression control region, coding region, and exon or intron region.

The term "translation product" as used herein refers to a protein which is synthesized based on the information of messenger RNA synthesized via transcription regardless of modification such as splicing. During the translation process of messenger RNA, ribosome first binds to messenger RNA, and amino acids are then linked in accordance with the nucleotide sequence of messenger RNA, thereby leading to the synthesis of a protein.

The term "primer" as used herein refers to a continuous polynucleotide that specifically recognizes and amplifies RNA resulting from gene expression or a polynucleotide derived therefrom, and/or a polynucleotide complementary thereto.

The complementary polynucleotide (i.e., a complementary strand or reverse strand) refers to a polynucleotide that is basically complementary to the full-length sequence of a polynucleotide having a nucleotide sequence as shown in a given SEQ ID NO. or a partial sequence thereof (herein, conveniently referred to as a "plus strand"), on the basis of the base pairing such as A:T(U) or G:C. Such a complementary strand, however, is not limited to a sequence completely complementary to the nucleotide sequence of a plus strand of interest; that is, the complementary strand may have such a complementarity that it can hybridize to the plus strand under stringent conditions.

As used herein, the "stringent conditions" means such conditions that a probe can hybridize to a target sequence with a higher degree of detection when compared with its hybridization to other sequences (e.g., at least twice the background). Stringent conditions are dependent on the sequence of a target, varying depending on the environment where hybridization takes place. By controlling stringency of hybridization and/or washing conditions, a target sequence that is 100% complementary to the probe can be identified.

As used herein, the term "mutant" in case of a nucleic acid refers to a naturally-occurring mutant resulting from polymorphism, mutation, selective splicing during transcription, or the like, a homolog thereof, a mutant based on degeneracy of genetic cord, a mutant comprising a deletion, substitution, addition, or insertion of one or more nucleotides, preferably one or several nucleotides, in a nucleotide sequence as shown in any of SEQ ID NOS: 1 to 50 or a partial sequence thereof, a mutant having at least about 80%, at least about 85%, preferably at least about 90%, more preferably at least about 95%, at least about 97%, at least about 98%, or at least about 99% identity with said nucleotide sequence or said partial sequence thereof, or a nucleic acid mutant that hybridizes to a polynucleotide or oligonucleotide comprising said nucleotide sequence or said partial sequence thereof under the stringent conditions as defined above. On the other hand, a "mutant" in case of a protein or peptide refers to a mutant comprising a deletion, substitution, addition, or insertion of one or more amino acids, preferably one or several amino acids, in an amino acid sequence as shown in any of SEQ ID NOS: 101 to 197 or a partial sequence thereof, or a mutant having a % identity of at least about 80%, at least about 85%, at least about 90%, at least about 95%, at least about 97%, at least about 98%, or at least about 99% with said amino acid sequence or said partial sequence thereof.

The term "several" as used herein means an integer of about 10, 9, 8, 7, 6, 5, 4, 3, or 2.

As used herein, the "% identity" can be identified by using a protein or gene searching system such as BLAST or FASTA as mentioned above, with introducing a gap (Karlin, S. et al., 1993, Proceedings of the National Academic Sciences, U.S.A., vol. 90, pp. 5873-5877; Altschul, S. F. et al., 1990, Journal of Molecular Biology, vol. 215, pp. 403-410; Pearson, W. R. et al., 1988, Proceedings of the National Academic Sciences, U.S.A., vol. 85, pp. 2444-2448).

As used herein, the term "derivative" in case of a nucleic acid refers to a derivative labeled with fluorophore or the like, a derivative comprising a modified nucleotide (e.g., a nucleotide having a functional group such as halogen, alkyl (e.g., methyl), alkoxy (e.g., methoxy), thio, or carboxymethyl; or a nucleotide comprising, for example, reconstitution of a base, saturation of a double bond, deamination, or substitution of oxygen by sulfur), or the like. On the other hand, a "derivative" in case of a protein (including an antibody) refers to a derivative labeled with an enzyme, fluorophore, or radioisotope, or a chemically modified derivative, such as an acetylated, acylated, alkylated, phosphorylated, sulfated, or glycosylated derivative.

As used herein, the term "a composition for diagnosis (or detection or identification)" or "a composition for identifying the disease" refers to a composition that is directly or indirectly employed for predicting the prognosis for a kidney cancer patient and/or for diagnosing the development of kidney cancer, the degree of advancement, the presence or absence of metastasis of kidney cancer, or the degree of amelioration, or for screening for candidate substances useful for preventing, ameliorating, or treating kidney cancer. The composition comprises a nucleotide, an oligonucleotide, or a polynucleotide, which can specifically recognize and bind to a gene whose expression varies or fluctuates *in vivo*, in particularly kidney tissue, tissue associated with the development of the kidney cancer, and an antibody that can detect a protein as a translation product of the gene. Such nucleotide, oligonucleotide and polynucleotide can be effectively used as a probe for detecting the aforementioned gene that is expressed *in vivo*, in tissue, or in a cell, based on the aforementioned properties, or as a primer for amplifying the gene expressed *in vivo*. As used herein, the term "composition" refers to a composition comprising a single probe according to the present invention or a probe mixture comprising two or more different probes. Such composition can be in any form, including a solid matter (e.g., a freeze-dried product) or a solution (e.g., an aqueous solution or buffer). Also, such composition can comprise additives such as a stabilizer and a preservative as long as such additive would not influence the analysis, according to need.

As used herein, the term "biological tissue" to be detected or diagnosed refers to a sample (or specimen) obtained from a living body, in which the expression pattern of the target gene of the invention changes with the development of kidney cancer, or the amount of the target polypeptide of the present invention varies compared with a normal control sample. Examples of biological samples include kidney tissue, tissue obtained from peripheral lymph nodes, or another organ suspected of being at risk for metastasis, cells derived from such tissue, and body fluid such as blood.

The term "specifically bind(s)" as used herein means that an antibody or a fragment thereof forms an antigen-antibody complex with only the target polypeptide, or a mutant or fragment thereof, of the invention, but does not substantially form such a complex with other peptidic or polypeptidic substances. The term "substantially" as used herein means that formation of a nonspecific complex may occur, although its degree is small.

The term "epitope" as used herein refers to an antigenic or immunogenic partial amino acid region (or an antigenic determinant) of the target polypeptide of the invention or a mutant or fragment thereof. The epitope is generally composed of at least 5 amino acids, preferably at least 7 amino acids, and more preferably at least 10 amino acids.

The term "prognosis" as used herein refers to a probability of survival that is indicated by a survival curve plotted by the Kaplan-Meier method (e.g., BMJ, 1998, 317: 1572). The patients with "good prognosis" refers to patients who exhibit a high survival ratio with a significant difference of $p < 0.05$, which is obtained by classifying the kidney cancer patients in accordance with some sort of clinical information, the survival curves are plotted by the Kaplan-Meier method according to the classification, and the differences are determined by a statistical technique. Similarly, patients with "poor prognosis" refer to patients who exhibit a low survival ratio with a significant difference of $p < 0.05$, which is obtained by classifying the kidney cancer patients in accordance with some sort of clinical information, the survival curves are plotted by the Kaplan-Meier method according to the classification, and the differences are determined by a statistical technique. Since the presence or absence of cancer metastasis is a factor that influences the patient's prognosis, the prediction of the prognosis is partly correlated with the prediction of cancer metastasis.

The term "PABPN1 gene" or "PABPN1" as used herein includes a gene (or DNA) encoding the Poly(A)-Binding Protein, Nuclear 1 gene (or DNA) as shown in a given nucleotide sequence (i.e., SEQ ID NO: 1), a homolog thereof, a mutant thereof, a derivative thereof, or the like, unless it is defined by a SEQ ID NO. Specifically, it includes the PABPN1 gene (Ensembl Gene ID, ENSG00000100836) as shown in SEQ ID NO: 1 and homologs thereof derived from other organism species. The PABPN1 gene can be obtained by the method disclosed in, for example, Brais, B. et al., 1998, Nature genetics, vol. 18, pp. 164-167. The fact that variations in the PABPN1 gene expression can become an indication of metastasis of kidney cancer is not known.

The term "PINK1 gene" or "PINK1" as used herein includes a gene (or DNA) encoding the PTEN Induced Putative Kinase 1 gene (or DNA) as shown in a given nucleotide sequence (i.e., SEQ ID NO: 2), a homolog thereof, a mutant thereof, a derivative thereof, or the like, unless it is defined by a SEQ ID NO. Specifically, it includes the PINK1 gene (Ensembl Gene ID, ENSG00000180056) as shown in SEQ ID NO: 2 and homologs thereof derived from other organism species. The PINK1 gene can be obtained by the method disclosed in, for example, Unoki, M. et al., 2001, Oncogene, vol. 20, pp. 4457-4465. The fact that variations in the PINK1 gene expression can become an indication of metastasis of kidney cancer is not known.

The term "TFF2 gene" or "TFF2" as used herein includes a gene (or DNA) encoding the Trefoil Factor 2 gene (or DNA) as shown in a given nucleotide sequence (i.e., SEQ ID NO: 3), a homolog thereof, a mutant thereof, a derivative thereof, or the like, unless it is defined by a SEQ ID NO. Specifically, it includes the TFF2 gene (Ensembl Gene ID, ENSGO0000160181) as shown in SEQ ID NO: 3 and homologs thereof derived from other organism species. The TFF2 gene can be obtained by the method disclosed in, for example, Tomasetto, C. et al., 1990, EMBO Journal, vol. 9, pp. 407-414. The fact that variations in the TFF2 gene expression can become an indication of metastasis of kidney cancer is not known.

The term "EIF3S9 gene" or "EIF3S9" as used herein includes a gene (or DNA) encoding the Eukaryotic Translation Initiation Factor 3 Subunit 9 gene (or DNA) as shown in a given nucleotide sequence (i.e., SEQ ID NO: 4), a homolog thereof, a mutant thereof, a derivative thereof, or the like, unless it is defined by a SEQ ID NO. Specifically, it includes the EIF3S9 gene (Ensembl Gene ID, ENSG00000106263) as shown in SEQ ID NO: 4 and homologs thereof derived from other organism species. The EIF3S9 gene can be obtained by the method disclosed in, for example, Methot, N. et al., 1997, Journal of Biological Chemistry, vol. 272, pp. 1110-1116. The fact that variations in the EIF3S9 gene expression can become an indication of metastasis of kidney cancer is not known.

The term "MAX gene" or "MAX" as used herein includes a gene (or DNA) encoding the MAX Protein gene (or DNA) as shown in a given nucleotide sequence (i.e., SEQ ID NO: 5), a homolog thereof, a mutant thereof, a derivative thereof, or the like, unless it is defined by a SEQ ID NO. Specifically, it includes the MAX gene (Ensembl Gene ID, ENSG00000125952) as shown in SEQ ID NO: 5 and homologs thereof derived from other organism species. The MAX gene can be obtained by the method disclosed in, for example, Wagner, A. et al., 1992, Proceedings of the National Academic Sciences, U. S.A., vol. 89, pp. 3111-3115. The fact that variations in the MAX gene expression can be an indication of metastasis of kidney cancer is not known.

The term "MLL4 gene" or "MLL4" as used herein includes a gene (or DNA) encoding the Trithorax Homolog 2 gene (or DNA) as shown in a given nucleotide sequence (i.e., SEQ ID NO: 6), a homolog thereof, a mutant thereof, a derivative thereof, or the like, unless it is defined by a SEQ ID NO. Specifically, it includes the MLL4 gene (Ensembl Gene ID, ENSG00000105663) as shown in SEQ ID NO: 6 and homologs thereof derived from other organism species. The MLL4 gene can be obtained by the method disclosed in, for example, Nagase, T. et al., 1997, DNA Research, vol. 4, pp. 141-150. The fact that variations in the MLL4 gene expression can become an indication of metastasis of kidney cancer is not known.

The term "CACNB2 gene" or "CACNB2" as used herein includes a gene (or DNA) encoding the Dihydropyridine-Sensitive L-Type, Calcium Channel Beta-2 Subunit gene (or DNA) as shown in a given nucleotide sequence (i.e., SEQ ID NO: 7), a homolog thereof, a mutant thereof, a derivative thereof, or the like, unless it is defined by a SEQ ID NO. Specifically, it includes the CACNB2 gene (Ensembl Gene ID, ENSG00000165995) as shown in SEQ ID NO: 7 and homologs thereof derived from other organism species. The CACNB2 gene can be obtained by the method disclosed in, for example, Rosenfeld, M. et al., 1993, Annals of Neurology., vol. 33, pp. 113-120. The fact that variations in the CACNB2 gene expression can become an indication of metastasis of kidney cancer is not known.

The term "ZMYND11 gene" or "ZMYND11" as used herein includes a gene (or DNA) encoding the Adenovirus 5 E1A-Binding Protein gene (or DNA) as shown in a given nucleotide sequence (i.e., SEQ ID NO: 8), a homolog thereof, a mutant thereof, a derivative thereof, or the like, unless it is defined by a SEQ ID NO. Specifically, it includes the ZMYND11 gene (Ensembl Gene ID, ENSG00000015171) as shown in SEQ ID NO: 8 and homologs thereof derived from other organism species. The ZMYND11 gene can be obtained by the method disclosed in, for example, Hateboer, G et al., 1995, EMBO Journal, vol. 14, pp. 3159-3169. The fact that variations in the ZMYND11 gene expression can become an indication of metastasis of kidney cancer is not known.

The term "BAT2 gene" or "BAT2" as used herein includes a gene (or DNA) encoding the Adenovirus 5 E1A-Binding Protein gene (or DNA) as shown in a given nucleotide sequence (i.e., SEQ ID NO: 9), a homolog thereof, a mutant thereof, a derivative thereof, or the like, unless it is defined by a SEQ ID NO. Specifically, it includes the BAT2 gene (Ensembl Gene ID, ENSG00000111960) as shown in SEQ ID NO: 9 and homologs thereof derived from other organism species. The BAT2 gene can be obtained by the method disclosed in, for example, Banerji, J. et al., 1990, Proceedings of the National Academic Sciences, U.S.A., vol. 87, pp. 2374-2378. The fact that variations in the BAT2 gene expression can become an indication of metastasis of kidney cancer is not known.

The term "NRBP gene" or "NRBP" as used herein includes a gene (or DNA) encoding the Nuclear Receptor Binding Protein gene (or DNA) as shown in a given nucleotide sequence (i.e., SEQ ID NO: 10), a homolog thereof, a mutant thereof, a derivative thereof, or the like, unless it is defined by a SEQ ID NO. Specifically, it includes the NRBP gene (Ensembl Gene ID, ENSG00000115216) as shown in SEQ ID NO: 10 and homologs thereof derived from other organism species. The NRBP gene can be obtained by the method disclosed in, for example, Hooper, J. D.. et al., 2000, Genomics, vol. 66, pp. 113-118. The fact that variations in the NRBP gene expression can become an indication of metastasis of kidney cancer is not known.

The term "MCM3AP gene" or "MCM3AP" as used herein includes a gene (or DNA) encoding the 80 KDa MCM3-Associated Protein gene (or DNA) as shown in a given nucleotide sequence (i.e., SEQ ID NO: 11), a homolog thereof, a mutant thereof, a derivative thereof, or the like, unless it is defined by a SEQ ID NO. Specifically, it includes the MCM3AP gene (Ensembl Gene ID, ENSG00000160294) as shown in SEQ ID NO: 11 and homologs thereof derived from other organism species. The MCM3AP gene can be obtained by the method disclosed in, for example, Takei, Y. et al., 1998, Journal of Biological Chemistry, vol. 273, pp. 22177-22180. The possibility that variations in the MCM3AP gene expression may become an indication of metastasis of kidney cancer is presumed in JP Patent Publication (kohyo) No. 2005-520536 (A), although it has not practically been verified.

The term "COL4A1 gene" or "COL4A1" as used herein includes a gene (or DNA) encoding the Collagen Alpha 1(IV) Chain gene (or DNA) as shown in a given nucleotide sequence (i.e., SEQ ID NO: 12), a homolog thereof, a mutant thereof, a derivative thereof, or the like, unless it is defined by a SEQ ID NO. Specifically, it includes the COL4A1 gene (Ensembl Gene ID, ENSG00000139825) as shown in SEQ ID NO: 12 and homologs thereof derived from other organism species. The COL4A1 gene can be obtained by the method disclosed in, for example, Solomon, E. et al., 1985, Proceedings of the National Academic Sciences, U.S.A., vol. 82, pp. 3330-3334. The fact that variations in the COL4A1 gene expression can become an indication of metastasis of kidney cancer is not known.

The term "MKNK1 gene" or "MKNK1" as used herein includes a gene (or DNA) encoding the MAP Kinase-Interacting Serine/Threonine Kinase 1 gene (or DNA) as shown in a given nucleotide sequence (i.e., SEQ ID NO: 13), a homolog thereof, a mutant thereof, a derivative thereof, or the like, unless it is defined by a SEQ ID NO. Specifically, it includes the MKNK1 gene (Ensembl Gene ID, ENSG00000079277) as shown in SEQ ID NO: 13 and homologs thereof derived from other organism species. The MKNK1 gene can be obtained by the method disclosed in, for example, Fukunaga, R. et al., 1997, EMBO Journal, vol. 16, pp. 1921-1933. The fact that variations in the MKNK1 gene expression can become an indication of metastasis of kidney cancer is not known.

The term "BBC3 gene" or "BBC3" as used herein includes a gene (or DNA) encoding the BCL2 Binding Component 3 gene (or DNA) as shown in a given nucleotide sequence (i.e., SEQ ID NO: 14), a homolog thereof, a mutant thereof, a derivative thereof, or the like, unless it is defined by a SEQ ID NO. Specifically, it includes the BBC3 gene (Ensembl Gene ID, ENSG00000105327) as shown in SEQ ID NO: 14 and homologs thereof derived from other organism species. The BBC3 gene can be obtained by the method disclosed in, for example, Yu, J. et al., 2001, Molecular Cell, vol. 7, pp. 673-682. The fact that variations in the BBC3 gene expression can become an indication of metastasis of kidney cancer is not known.

The term "FLJ10359 gene" or "FLJ10359" as used herein includes a gene (or DNA) encoding the Protein BAP28 gene (or DNA) as shown in a given nucleotide sequence (i.e., SEQ ID NO: 15), a homolog thereof, a mutant thereof, a derivative thereof, or the like, unless it is defined by a SEQ ID NO. Specifically, it includes the FLJ10359 gene (Ensembl Gene ID, ENSG00000119285) as shown in SEQ ID NO: 15 and homologs thereof derived from other organism species.

The term "DPT gene" or "DPT" as used herein includes a gene (or DNA) encoding the Dermatopontin gene (or DNA) as shown in a given nucleotide sequence (i.e., SEQ ID NO: 16), a homolog thereof, a mutant thereof, a derivative thereof, or the like, unless it is defined by a SEQ ID NO. Specifically, it includes the DPT gene (Ensembl Gene ID, ENSG00000143196) as shown in SEQ ID NO: 16 and homologs thereof derived from other organism species. The DPT gene can be obtained by the method disclosed in, for example, Superti-Fruga, A. et al., 1993, Genomics, vol. 17, pp. 463-467. The fact that variations in the DPT gene expression can become an indication of metastasis of kidney cancer is not known.

The term "C10orf76 gene" or "C10orf76" as used herein includes a gene (or DNA) encoding the gene (or DNA) as shown in a given nucleotide sequence (i.e., SEQ ID NO: 17), a homolog thereof, a mutant thereof, a derivative thereof, or the like, unless it is defined by a SEQ ID NO. Specifically, it includes the C10orf76 gene (Ensembl Gene ID, ENSG00000120029) as shown in SEQ ID NO: 17 and homologs thereof derived from other organism species.

The term "DIA1 gene" or "DIA1" as used herein includes a gene (or DNA) encoding the NADH-Cytochrome B5 Reductase gene (or DNA) as shown in a given nucleotide sequence (i.e., SEQ ID NO: 18), a homolog thereof, a mutant thereof, a derivative thereof, or the like, unless it is defined by a SEQ ID NO. Specifically, it includes the DIA1 gene (Ensembl Gene ID, ENSG00000100243) as shown in SEQ ID NO: 18 and homologs thereof derived from other organism species. The DIA1 gene can be obtained by the method disclosed in, for example, Du, M. et al., 1997, Biochemical Biophysical Research Communication, vol. 235, pp. 779-783. The fact that variations in the DIA1 gene expression can become an indication of metastasis of kidney cancer is not known.

The term "PBK gene" or "PBK" as used herein includes a gene (or DNA) encoding the T-LAK Cell-Originated Protein Kinase gene (or DNA) as shown in a given nucleotide sequence (i.e., SEQ ID NO: 19), a homolog thereof, a mutant thereof, a derivative thereof, or the like, unless it is defined by a SEQ ID NO. Specifically, it includes the PBK gene (Ensembl Gene ID, ENSG00000168078) as shown in SEQ ID NO: 19 and homologs thereof derived from other organism species. The PBK gene can be obtained by the method disclosed in, for example, Gaudet, S. et al., 2000, Proceedings of the National Academic Sciences, U.S.A., vol. 97, pp. 5167-5172. The fact that variations in the PBK gene expression can become an indication of metastasis of kidney cancer is not known.

The term "PRKD2 gene" or "PRKD2" as used herein includes a gene (or DNA) encoding the Protein Kinase C, D2 Type gene (or DNA) as shown in a given nucleotide sequence (i.e., SEQ ID NO: 20), a homolog thereof, a mutant thereof, a derivative thereof, or the like, unless it is defined by a SEQ ID NO. Specifically, it includes the PRKD2 gene (Ensembl Gene ID, ENSG00000105287) as shown in SEQ ID NO: 20 and homologs thereof derived from other organism species. The PRKD2 gene can be obtained by the method disclosed in, for example, Sturany, S. et al., 2001, Journal of Biological Chemistry, vol. 276, pp. 3310-3318. The fact that variations in the PRKD2 gene expression can become an indication of metastasis of kidney cancer is not known.

The term "KRT19 gene" or "KRT19" as used herein includes a gene (or DNA) encoding the Keratin, Type I Cytoskeletal 19 gene (or DNA) as shown in a given nucleotide sequence (i.e., SEQ ID NO: 21), a homolog thereof, a mutant thereof, a derivative thereof, or the like, unless it is defined by a SEQ ID NO. Specifically, it includes the KRT19 gene (Ensembl Gene ID, ENSG00000171345) as shown in SEQ ID NO: 21 and homologs thereof derived from other organism species. The KRT19 gene can be obtained by the method disclosed in, for example, Bader, B. et al., 1986, EMBO Journal, vol. 5, pp. 1865-1875. The fact that variations in the KRT19 gene expression can become an indication of kidney cancer is deduced in JP Patent Publication (kohyo) No. 2005-507997 (A), although it has not yet been verified.

The term "FLJ23436 gene" or "FLJ23436" as used herein includes a gene (or DNA) encoding the gene (or DNA) as shown in a given nucleotide sequence (i.e., SEQ ID NO: 22), a homolog thereof, a mutant thereof, a derivative thereof, or the like, unless it is defined by a SEQ ID NO. Specifically, it includes the FLJ23436 gene (Ensembl Gene ID, ENSG00000169957) as shown in SEQ ID NO: 22 and homologs thereof derived from other organism species. The FLJ23436 gene can be obtained by the method disclosed in, for example, Beausoleil, S. A. et al., 2004, Proceedings of the National Academic Sciences, U.S.A., vol. 101, pp. 12130-12135. The fact that variations in the FLJ23436 gene expression can become an indication of metastasis of kidney cancer is not known.

The term "NPHS2 gene" or "NPHS2" as used herein includes a gene (or DNA) encoding the Podocin gene (or DNA) as shown in a given nucleotide sequence (i.e., SEQ ID NO: 23), a homolog thereof, a mutant thereof, a derivative thereof, or the like, unless it is defined by a SEQ ID NO. Specifically, it includes the NPHS2 gene (Ensembl Gene ID, ENSG00000116218) as shown in SEQ ID NO: 23 and homologs thereof derived from other organism species. The NPHS2 gene can be obtained by the method disclosed in, for example, Kestila, M. et al., 1998, Molecular Cell, vol. 1, pp. 575-582. The fact that variations in the NPHS2 gene expression can become an indication of metastasis of kidney cancer is not known.

The term "C3orf14 gene" or "C3orf14" as used herein includes a gene (or DNA) encoding the HT021 gene (or DNA) as shown in a given nucleotide sequence (i.e., SEQ ID NO: 24), a homolog thereof, a mutant thereof, a derivative thereof, or the like, unless it is defined by a SEQ ID NO. Specifically, it includes the C3orf14 gene (Ensembl Gene ID, ENSG00000114405) as shown in SEQ ID NO: 24 and homologs thereof derived from other organism species.

The term "AGTR2 gene" or "AGTR2" as used herein includes a gene (or DNA) encoding the Type-2 Angiotensin II Receptor gene (or DNA) as shown in a given nucleotide sequence (i.e., SEQ ID NO: 25), a homolog thereof, a mutant thereof, a derivative thereof, or the like, unless it is defined by a SEQ ID NO. Specifically, it includes the AGTR2 gene (Ensembl Gene ID, ENSG00000180772) as shown in SEQ ID NO: 25 and homologs thereof derived from other organism species. The AGTR2 gene can be obtained by the method disclosed in, for example, Koike, G et al., 1994, Biochemical Biophysical Research Communication, vol. 203, pp. 1842-1850. The fact that variations in the AGTR2 gene expression can become an indication of metastasis of kidney cancer is not known.

The term "HTR1F gene" or "HTR1F" as used herein includes a gene (or DNA) encoding the 5-Hydroxytryptamine IF Receptor gene (or DNA) as shown in a given nucleotide sequence (i.e., SEQ ID NO: 26), a homolog thereof, a mutant thereof, a derivative thereof, or the like, unless it is defined by a SEQ ID NO. Specifically, it includes the HTR1F gene (Ensembl Gene ID, ENSG00000179097) as shown in SEQ ID NO: 26 and homologs thereof derived from other organism species. The HTR1F gene can be obtained by the method disclosed in, for example, Lovenberg, T. W. et al., 1993, Proceedings of the National Academic Sciences, U.S.A., vol. 90, pp. 2184-2188. The fact that variations in the HTR1F gene expression can become an indication of metastasis of kidney cancer is not known.

The term "KIF3B gene" or "KIF3B" as used herein includes a gene (or DNA) encoding the Kinesin-Like Protein KIF3B gene (or DNA) as shown in a given nucleotide sequence (i.e., SEQ ID NO: 27), a homolog thereof, a mutant thereof, a derivative thereof, or the like, unless it is defined by a SEQ ID NO. Specifically, it includes the KIF3B gene (Ensembl Gene ID, ENSG00000101350) as shown in SEQ ID NO: 27 and homologs thereof derived from other organism species. The KIF3B gene can be obtained by the method disclosed in, for example, Nagase, T. et al., 1997, DNA Research, vol. 4, pp. 141-150. The fact that variations in the KIF3B gene expression can become an indication of metastasis of kidney cancer is not known.

The term "DCN gene" or "DCN" as used herein includes a gene (or DNA) encoding the Decorin gene (or DNA) as shown in a given nucleotide sequence (i.e., SEQ ID NO: 28), a homolog thereof, a mutant thereof, a derivative thereof, or the like, unless it is defined by a SEQ ID NO. Specifically, it includes the DCN gene (Ensembl Gene ID, ENSG00000011465) as shown in SEQ ID NO: 28 and homologs thereof derived from other organism species. The DCN gene can be obtained by the method disclosed in, for example, Danielson, K. G et al., 1993, Genomics, vol. 15, pp. 146-160. The fact that variations in the DCN gene expression can become an indication of metastasis of kidney cancer is not known.

The term "STK22C gene" or "STK22C" as used herein includes a gene (or DNA) encoding the Testis-Specific Serine/Threonine Kinase 22C gene (or DNA) as shown in a given nucleotide sequence (i.e., SEQ ID NO: 29), a homolog thereof, a mutant thereof, a derivative thereof, or the like, unless it is defined by a SEQ ID NO. Specifically, it includes the STK22C gene (Ensembl Gene ID, ENSG00000162526) as shown in SEQ ID NO: 29 and homologs thereof derived from other organism species. The STK22C gene can be obtained by the method disclosed in, for example, Visconti, P. E. et al., 2001, Genomics, vol. 77, pp. 163-170. The fact that variations in the STK22C gene expression can become an indication of metastasis of kidney cancer is not known.

The term "DKFZp566C0424 gene" or "DKFZp566C0424" as used herein includes a gene (or DNA) encoding the Putative MAPK Activating Protein PM20 gene (or DNA) as shown in a given nucleotide sequence (i.e., SEQ ID NO: 30), a homolog thereof, a mutant thereof, a derivative thereof, or the like, unless it is defined by a SEQ ID NO. Specifically, it includes the DKFZp566C0424 gene (ENSG00000055070) as shown in SEQ ID NO: 30 and homologs thereof derived from other organism species. The DKFZp566C0424 gene can be obtained by the method disclosed in, for example, Visconti, P. E. et al., 2001, Genomics, vol. 77, pp. 163-170. The fact that variations in the DKFZp566C0424 gene expression can become an indication of metastasis of kidney cancer is not known.

The term "CNR1 gene" or "CNR1" as used herein includes a gene (or DNA) encoding the Cannabinoid Receptor 1 gene (or DNA) as shown in a given nucleotide sequence (i.e., SEQ ID NO: 31), a homolog thereof, a mutant thereof, a derivative thereof, or the like, unless it is defined by a SEQ ID NO. Specifically, it includes the CNR1 gene (Ensembl Gene ID, ENSG00000118432) as shown in SEQ ID NO: 31 and homologs thereof derived from other organism species. The CNR1 gene can be obtained by the method disclosed in, for example, Matsuda, L.A. et al., 1990, Nature, vol. 346, pp. 561-564. The fact that variations in the CNR1 gene expression can become an indication of metastasis of kidney cancer is not known.

The term "HOMER3 gene" or "HOMER3" as used herein includes a gene (or DNA) encoding the HOMER, Neuronal Immediate Early Gene, 3 gene (or DNA) as shown in a given nucleotide sequence (i.e., SEQ ID NO: 32), a homolog thereof, a mutant thereof, a derivative thereof, or the like, unless it is defined by a SEQ ID NO. Specifically, it includes the HOMER3 gene (Ensembl Gene ID, ENSG00000051128) as shown in SEQ ID NO: 32 and homologs thereof derived from other organism species. The HOMER3 gene can be obtained by the method disclosed in, for example, Xiao, B. et al., 2001, Neuron, vol. 21, pp. 707-716. The fact that variations in the HOMER3 gene expression can become an indication of metastasis of kidney cancer is not known.

The term "GPR2 gene" or "GPR2" as used herein includes a gene (or DNA) encoding the C-C Chemokine Receptor Type 10 gene (or DNA) as shown in a given nucleotide sequence (i.e., SEQ ID NO: 33), a homolog thereof, a mutant thereof, a derivative thereof, or the like, unless it is defined by a SEQ ID NO. Specifically, it includes the GPR2 gene (Ensembl Gene ID, ENSG00000177032) as shown in SEQ ID NO: 33 and homologs thereof derived from other organism species. The GPR2 gene can be obtained by the method disclosed in, for example, Marchese, A. et al., 1994, Genomics, vol. 23, pp. 609-618. The fact that variations in the GPR2 gene expression can become an indication of metastasis of kidney cancer is not known.

The term "FLJ12442 gene" or "FLJ12442" as used herein includes a gene (or DNA) encoding the gene (or DNA) as shown in a given nucleotide sequence (i.e., SEQ ID NO: 34), a homolog thereof, a mutant thereof, a derivative thereof, or the like, unless it is defined by a SEQ ID NO. Specifically, it includes the FLJ12442 gene (Ensembl Gene ID, ENSG00000168268) as shown in SEQ ID NO: 34 and homologs thereof derived from other organism species. The FLJ12442 gene can be obtained by the method disclosed in, for example, Ota, T. et al., 2004, Nature Genetics, vol, 36, pp. 40-45. The fact that variations in the FLJ12442 gene expression can become an indication of metastasis of kidney cancer is not known.

The term "XLKD1 gene" or "XLKD1" as used herein includes a gene (or DNA) encoding the Extracellular Link Domain Containing 1 gene (or DNA) as shown in a given nucleotide sequence (i.e., SEQ ID NO: 35), a homolog thereof, a mutant thereof, a derivative thereof, or the like, unless it is defined by a SEQ ID NO. Specifically, it includes the XLKD1 gene (Ensembl Gene ID, ENSG00000133800) as shown in SEQ ID NO: 35 and homologs thereof derived from other organism species. The XLKD1 gene can be obtained by the method disclosed in, for example, Banerji, S. et al., 1999, Journal of Cellular Biology, vol. 144, pp. 1789-801. The fact that variations in the XLKD1 gene expression can become an indication of metastasis of kidney cancer is not known.

The term "CASKIN2 gene" or "CASKIN2" as used herein includes a gene (or DNA) encoding the CASK-Interacting Protein 2 gene (or DNA) as shown in a given nucleotide sequence (i.e., SEQ ID NO: 36), a homolog thereof, a mutant thereof, a derivative thereof, or the like, unless it is defined by a SEQ ID NO. Specifically, it includes the CASKIN2 gene (Ensembl Gene ID, ENSG00000177303) as shown in SEQ ID NO: 36 and homologs thereof derived from other organism species. The CASKIN2 gene can be obtained by the method disclosed in, for example, Strausberg, R. L. et al., 2002, Proceedings of the National Academic Sciences, U.S.A., vol. 99, pp. 16899-16903. The fact that variations in the CASKIN2 gene expression can become an indication of metastasis of kidney cancer is not known.

The term "COL5A2 gene" or "COL5A2" as used herein includes a gene (or DNA) encoding the Collagen Alpha 2(V) Chain Protein gene (or DNA) as shown in a given nucleotide sequence (i.e., SEQ ID NO: 37), a homolog thereof, a mutant thereof, a derivative thereof, or the like, unless it is defined by a SEQ ID NO. Specifically, it includes the COL5A2 gene (Ensembl Gene ID, ENSG00000179877) as shown in SEQ ID NO: 37 and homologs thereof derived from other organism species. The COL5A2 gene can be obtained by the method disclosed in, for example, Burgeson, R. E. et al., 2002, Proceedings of the National Academic Sciences, U.S.A., vol. 73, pp. 2579-2583. The fact that variations in the COL5A2 gene expression can become an indication of metastasis of kidney cancer is not known.

The term "BRD3 gene" or "BRD3" as used herein includes a gene (or DNA) encoding the Bromodomain-Containing Protein 3 gene (or DNA) as shown in a given nucleotide sequence (i.e., SEQ ID NO: 38), a homolog thereof, a mutant thereof, a derivative thereof, or the like, unless it is defined by a SEQ ID NO. Specifically, it includes the BRD3 gene (Ensembl Gene ID, ENSG00000169925) as shown in SEQ ID NO: 38 and homologs thereof derived from other organism species. The BRD3 gene can be obtained by the method disclosed in, for example, Nomura, N. L. et al., 1994, DNA Research, vol. 1, pp. 223-229. The fact that variations in the BRD3 gene expression can become an indication of metastasis of kidney cancer is not known.

The term "ATP6VOA4 gene" or "ATP6VOA4" as used herein includes a gene (or DNA) encoding the ATPase, H+ Transporting, Lysosomal V0 Subunit A ISOFORM 4 gene (or DNA) as shown in a given nucleotide sequence (i.e., SEQ ID NO: 39), a homolog thereof, a mutant thereof, a derivative thereof, or the like, unless it is defined by a SEQ ID NO. Specifically, it includes the ATP6V0A4gene (Ensembl Gene ID, ENSG00000105929) as shown in SEQ ID NO: 39 and homologs thereof derived from other organism species. The ATP6VOA4 gene can be obtained by the method disclosed in, for example, Smith, A. N. et al., 2000, Nature Genetics, vol. 26, pp. 71-75. The fact that variations in the ATP6VOA4 gene expression can become an indication of metastasis of kidney cancer is not known.

The term "PRODH2 gene" or "PRODH2" as used herein includes a gene (or DNA) encoding the Nephrin gene (or DNA) as shown in a given nucleotide sequence (i.e., SEQ ID NO: 40), a homolog thereof, a mutant thereof, a derivative thereof, or the like, unless it is defined by a SEQ ID NO. Specifically, it includes the PRODH2 gene (Ensembl Gene ID, ENSG00000161270) as shown in SEQ ID NO: 40 and homologs thereof derived from other organism species. The PRODH2 gene can be obtained by the method disclosed in, for example, Chakravarti, A. et al., 2002, Proceedings of the National Academic Sciences, U.S.A., vol. 99, pp. 4755-4756. The fact that variations in the PRODH2 gene expression can become an indication of metastasis of kidney cancer is not known.

The term "EPHB2 gene" or "EPHB2" as used herein includes a gene (or DNA) encoding the Ephrin Type-B Receptor 2 gene (or DNA) as shown in a given nucleotide sequence (i.e., SEQ ID NO: 41), a homolog thereof, a mutant thereof, a derivative thereof, or the like, unless it is defined by a SEQ ID NO. Specifically, it includes the EPHB2 gene (Ensembl Gene ID, ENSG00000133216) as shown in SEQ ID NO: 41 and homologs thereof derived from other organism species. The EPHB2 gene can be obtained by the method disclosed in, for example, Chan, J. et al., 1991, Oncogene, vol. 6, pp. 1057-1061. The fact that variations in the EPHB2 gene expression can become an indication of metastasis of kidney cancer is not known.

The term "LYAR gene" or "LYAR" as used herein includes a gene (or DNA) encoding the Hypothetical Protein FLJ20425 gene (or DNA) as shown in a given nucleotide sequence (i.e., SEQ ID NO: 42), a homolog thereof, a mutant thereof, a derivative thereof, or the like, unless it is defined by a SEQ ID NO. Specifically, it includes the LYAR gene (Ensembl Gene ID, ENSG00000145220) as shown in SEQ ID NO: 42 and homologs thereof derived from other organism species. The LYAR gene can be obtained by the method disclosed in, for example, Su, L. et al., 1993, Genes Development, vol. 7, pp. 735-748. The fact that variations in the LYAR gene expression can become an indication of metastasis of kidney cancer is not known.

The term "COX6B gene" or "COX6B" as used herein includes a gene (or DNA) encoding the Cytochrome C Oxidase Polypeptide VIB gene (or DNA) as shown in a given nucleotide sequence (i.e., SEQ ID NO: 43), a homolog thereof, a mutant thereof, a derivative thereof, or the like, unless it is defined by a SEQ ID NO. Specifically, it includes the COX6B gene (Ensembl Gene ID, ENSG00000126267) as shown in SEQ ID NO: 43 and homologs thereof derived from other organism species. The COX6B gene can be obtained by the method disclosed in, for example, Taanman, J-W. et al., 1989, Nucleic Acids Research, vol. 17, pp. 1766. The fact that variations in the COX6B gene expression can become an indication of metastasis of kidney cancer is not known.

The term "PRH1 gene" or "PRH1" as used herein includes a gene (or DNA) encoding the Salivary Acidic Proline-Rich Phosphoprotein 1/2 gene (or DNA) as shown in a given nucleotide sequence (i.e., SEQ ID NO: 44), a homolog thereof, a mutant thereof, a derivative thereof, or the like, unless it is defined by a SEQ ID NO. Specifically, it includes the PRH1 gene (Ensemb1 Gene ID, ENSG00000176621) as shown in SEQ ID NO: 44 and homologs thereof derived from other organism species. The PRH1 gene can be obtained by the method disclosed in, for example, Oppenheim, F. G. et al., 1971, Biochemistry, vol. 10, pp. 4233-4238. The fact that variations in the PRH1 gene expression can become an indication of metastasis of kidney cancer is not known.

The term "LAPTM5 gene" or "LAPTM5" as used herein includes a gene (or DNA) encoding the Lysosomal-Associated Multitransmembrane Protein gene (or DNA) as shown in a given nucleotide sequence (i.e., SEQ ID NO: 45), a homolog thereof, a mutant thereof, a derivative thereof, or the like, unless it is defined by a SEQ ID NO. Specifically, it includes the LAPTM5 gene (Ensembl Gene ID, ENSG00000162511) as shown in SEQ ID NO: 45 and homologs thereof derived from other organism species. The LAPTM5 gene can be obtained by the method disclosed in, for example, Adra, C. N. et al., 1996, Genomics, vol. 35, pp. 328-337. The fact that variations in the LAPTM5 gene expression can become an indication of metastasis of kidney cancer is not known; however, the possibility that LAPTM5 may become a marker for kidney cancer is presumed in WO 2005-24603.

The term "RPS6KA4 gene" or "RPS6KA4" as used herein includes a gene (or DNA) encoding the Ribosomal Protein S6 Kinase, 90KDA, Polypeptide 4 gene (or DNA) as shown in a given nucleotide sequence (i.e., SEQ ID NO: 46), a homolog thereof, a mutant thereof, a derivative thereof, or the like, unless it is defined by a SEQ ID NO. Specifically, it includes the RPS6KA4 gene (Ensembl Gene ID, ENSG00000162302) as shown in SEQ ID NO: 46 and homologs thereof derived from other organism species. The RPS6KA4 gene can be obtained by the method disclosed in, for example, Pierrat, B. et al., 1998, Journal of Biological Chemistry, vol. 273, pp. 29661-29671. The fact that variations in the RPS6KA4 gene expression can become an indication of metastasis of kidney cancer is not known.

The term "GCC2 gene" or "GCC2" as used herein includes a gene (or DNA) encoding the GRIP and Coiled-Coil Domain Containing 2 gene (or DNA) as shown in a given nucleotide sequence (i.e., SEQ ID NO: 47), a homolog thereof, a mutant thereof, a derivative thereof, or the like, unless it is defined by a SEQ ID NO. Specifically, it includes the GCC2 gene (Ensembl Gene ID, ENSG00000144055) as shown in SEQ ID NO: 47 and homologs thereof derived from other organism species. The GCC2 gene can be obtained by the method disclosed in, for example, Eichmuller, S. et al., 2001, Proceedings of the National Academic Sciences, U.S.A., vol. 98, pp. 629-634. The fact that variations in the GCC2 gene expression can become an indication of metastasis of kidney cancer is not known.

The term "FGF2 gene" or "FGF2" as used herein includes a gene (or DNA) encoding the Heparin-Binding Growth Factor 2 gene (or DNA) as shown in a given nucleotide sequence (i.e., SEQ ID NO: 48), a homolog thereof, a mutant thereof, a derivative thereof, or the like, unless it is defined by a SEQ ID NO. Specifically, it includes the FGF2 gene (Ensembl Gene ID, ENSG00000138685) as shown in SEQ ID NO: 48 and homologs thereof derived from other organism species. The FGF2 gene can be obtained by the method disclosed in, for example, Abraham, J. et al., 1986, Science, vol. 233, pp. 545-548. The fact that variations in the FGF2 gene expression can become an indication of metastasis of kidney cancer is known as disclosed in, for example, Miyake, H. et al., 1996, Cancer Research, vol. 56, pp. 2440-2445.

The term "MMP14 gene" or "MMP14" as used herein includes a gene (or DNA) encoding the Matrix Metalloproteinase-14 gene (or DNA) as shown in a given nucleotide sequence (i.e., SEQ ID NO: 49), a homolog thereof, a mutant thereof, a derivative thereof, or the like, unless it is defined by a SEQ ID NO. Specifically, it includes the MMP14 gene (Ensembl Gene ID, ENSG00000157227) as shown in SEQ ID NO: 49 and homologs thereof derived from other organism species. The MMP14 gene can be obtained by the method disclosed in, for example, Sato, H. et al., 1994, Nature, vol. 370, pp. 61-65. The fact that variations in the MMP 14 gene expression can become an indication of metastasis of kidney cancer is known as disclosed in, for example, Kitagawa, Y. et al., 1999, Journal of Urology, vol. 162, pp. 905-909

The term "ERBB2 gene" or "ERBB2" as used herein includes a gene (or DNA) encoding the Receptor Protein-Tyrosine Kinase ERBB-2 gene (or DNA) as shown in a given nucleotide sequence (i.e., SEQ ID NO: 50), a homolog thereof, a mutant thereof, a derivative thereof, or the like, unless it is defined by a SEQ ID NO. Specifically, it includes the ERBB2 gene (Ensembl Gene ID, ENSG00000141736) as shown in SEQ ID NO: 50 and homologs thereof derived from other organism species. The ERBB2 gene can be obtained by the method disclosed in, for example, Yang-Feng, T. L. et al., 1985, Citogenetic Cell Genetics, vol. 40, pp. 784. The fact that variations in the ERBB2 gene expression can become an indication of metastasis of kidney cancer is known as disclosed in, for example, Freeman, M. R. et al., 1989, Cancer Research, vol. 49, pp. 6221-6225.

Other genes or polypeptides described herein are adequately described in the following description.

Effects of the Invention

The present invention provides a composition for diagnosing a disease, which is useful for diagnosing, detecting, identifying, or predicting the presence or metastasis of kidney cancer and for treating kidney cancer, and a method for diagnosing, detecting, identifying, or predicting the presence or metastasis of kidney cancer using said composition. Thus, the present invention provides remarkable effects by providing a rapid and simple method for detecting, identifying, or predicting the presence or metastasis of kidney cancer with high specificity and high efficiency of prediction.

Some of the markers for kidney cancer of the present invention are found in biological samples, such as blood taken from patients with kidney cancer. Since the above-mentioned markers are not or almost not found in healthy persons, the presence or amount of the markers can be used as the indication to easily detect kidney cancer in the blood, for example.

The present invention enables effective diagnosis for managing the prognosis for patients with kidney cancer by using the probes for detecting kidney cancer of the present invention in combination with the probes for detecting metastasis of kidney cancer of the present invention.

BRIEF DESCRIPTION OF THE DRAWINGS

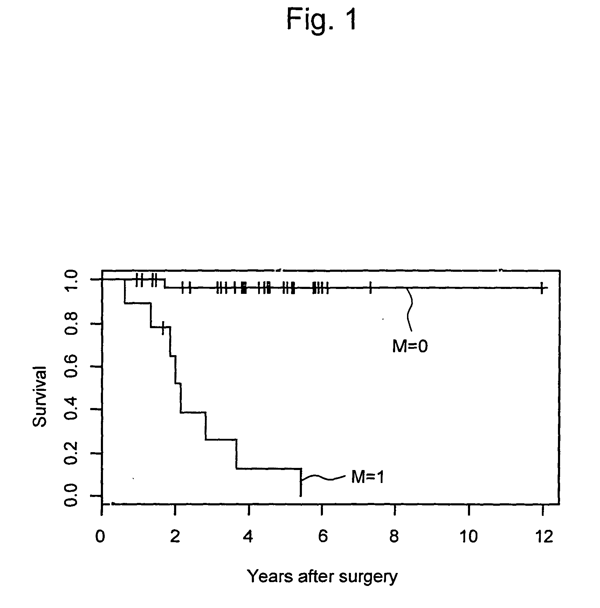

Fig. 1 shows the survival curves prepared by the Kaplan-Meier method concerning a group of patients in which metastasis to organs other than the kidney was not diagnosed at the time of surgery (M = 0) and concerning a group of patients in which metastasis was diagnosed (M = 1). The vertical axis indicates a survival rate, and the horizontal axis indicates years after surgery. The 5-year survival was 96% (M = 0) and 13% (M = 1), respectively.

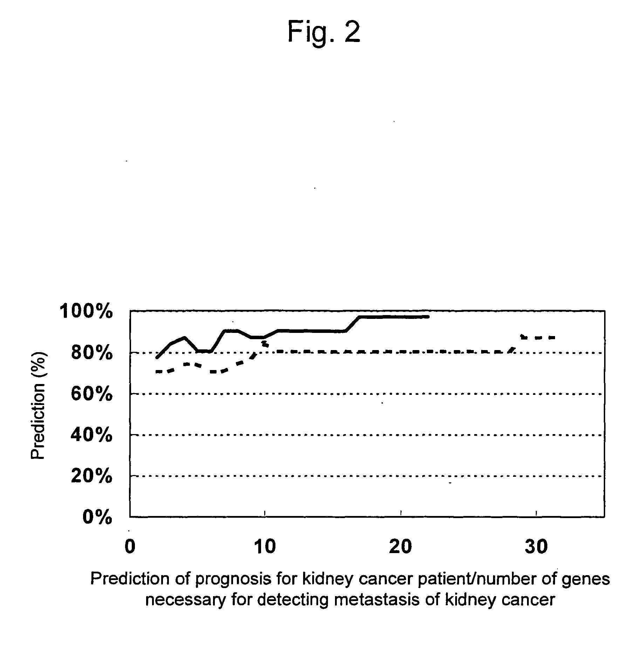

Fig. 2 shows the probability of predicting patients with good prognoses when the polynucleotides shown in SEQ ID NOS: 1 to 19 and 48 corresponding to the genes indicated in Table 1 are used in combination as a DNA chip, and the probability of predicting patients with poor prognoses when the polynucleotides shown in SEQ ID NOS: 20 to 47, 49, and 50 corresponding to the genes are used in combination as a DNA chip. The vertical axis indicates the probability of predicting patients with good prognoses or poor prognoses, and the horizontal axis indicates the total number of genes required for predicting patients with good prognoses or poor prognoses. The solid line indicates the probability of predicting patients with good prognoses, and the broken line indicates the probability of predicting patients with poor prognoses.

PREFERRED EMBODIMENTS OF THE INVENTION

Hereafter, the present invention is described in more detail.

1. Kidney cancer-associated markers

1.1 Kidney cancer-associated target nucleic acids

Examples of target nucleic acids as markers associated with metastasis of kidney cancer for detecting, identifying, or predicting the presence or metastasis of kidney cancer, for predicting the prognosis for a kidney cancer patient, or for identifying the presence or absence of cells to which kidney cancer has metastasized using the composition, kit, or DNA chip of the present invention include human genes each comprising a nucleotide sequence as shown in any of SEQ ID NOS: 1 to 50 (i.e., PABPN1, PINK1, TFF2, EIF3S9, MAX, MLL4, CACNB2, ZMYND11, BAT2, NRBP, MCM3AP, COL4A1, MKNK1, BBC3, FLJ10359, DPT, C10orf76, DIA1, PBK, PRKD2, KRT19, FLJ23436, NPHS2, C3orf14, AGTR2, HTR1F, KIF3B, DCN, STK22C, DKFZp566C0424, CNR1, HOMER3, GPR2, FLJ12442, XLKD1, CASKIN2, COL5A2, BRD3, ATP6VOA4, PRODH2, EPHB2, LYAR, COX6B, PRH1, LAPTM5, RPS6KA4, GCC2, FGF2, MMP14, and ERBB2, respectively), homologs thereof, transcription products or cDNAs thereof, mutants thereof, and derivatives thereof. The terms "gene," "homolog," "transcription product," "cDNA," "mutant," and "derivative" are as defined above. The preferred target nucleic acids are human genes, each of which comprises a nucleotide sequence as shown in any of SEQ ID NOS: 1 to 50, and transcription products or cDNAs thereof, preferably transcription products or cDNAs.

According to the present invention, the expression levels of said genes, a target of the prediction of the prognosis for kidney cancer patients and/or a target of the prediction of the metastasis of kidney cancer, significantly change (i.e., increase or decrease) in the kidney cancer tissues from the patients with poor prognoses, when compared with the kidney cancer tissues from patients with good prognoses. Table 1 shows the Ensemble Gene ID numbers and the GenBank accession numbers of the genes collectively. Genes shown in Table 1 are the genes that recognize kidney cancer tissue from a patient with good prognosis and the genes that recognize kidney cancer tissue from a patient with poor prognosis.

Table 1

| SEQ ID NO: | Ensemble Gene ID | GenBank Acc. No. | Genes | SEQ ID NO: | Ensemble Gene ID | GenBank Acc. No. | Genes |
|---|---|---|---|---|---|---|---|
| 1 | ENSG00000100836 | NM_004643 | PABPN1 | 26 | ENSG00000179097 | NM_000866 | HTR1F |
| 2 | ENSG00000180056 | NM_032409 | PINK1 | 27 | ENSG00000101350 | NM_004798 | KIF3B |
| 3 | ENSG00000160181 | NM_005423 | TFF2 | 28 | ENSG00000011465 | NM_133506 | DCN |
| 4 | ENSG00000106263 | NM_003751 | EIF3S9 | 29 | ENSG00000162526 | NM_052841 | STK22C |
| 5 | ENSG00000125952 | NM_002382 | MAX | 30 | ENSG00000055070 | BX640985 | DKFZp566C0424 |
| 6 | ENSG00000105663 | NM_014727 | MLL4 | 31 | ENSG00000118432 | NM_016083 | CNR1 |
| 7 | ENSG00000165995 | NM_000724 | CACNB2 | 32 | ENSG00000051128 | NM_004838 | HOMER3 |
| 8 | ENSG00000015171 | NM_006624 | ZMYND11 | 33 | ENSG00000177032 | NM_016602 | GPR2 |
| 9 | ENSG00000111960 | NM_004638 | BAT2 | 34 | ENSG00000168268 | NM_022908 | FLJ12442 |
| 10 | ENSG00000115216 | NM_013392 | NRBP | 35 | ENSG00000133800 | NM_006691 | XLKD1 |
| 11 | ENSG00000160294 | NM_003906 | MCM3AP | 36 | ENSG00000177303 | NM_020753 | CASKIN2 |
| 12 | ENSG00000139825 | NM_001845 | COL4A1 | 37 | ENSG00000179877 | NM_000393 | COL5A2 |
| 13 | ENSG00000079277 | NM_003684 | MKNK1 | 38 | ENSG00000169925 | NM_007371 | BRD3 |
| 14 | ENSG00000105327 | NM_014417 | BBC3 | 39 | ENSG00000105929 | NM_020632 | ATP6VOA4 |
| 15 | ENSG00000119285 | NM_018072 | FLJ10359 | 40 | ENSG00000161270 | NM_021232 | PRODH2 |
| 16 | ENSG00000143196 | NM_001937 | DPT | 41 | ENSG00000133216 | NM_004442 | EPHB2 |
| 17 | ENSG00000120029 | NM_024541 | C10orf76 | 42 | ENSG00000145220 | NM_017816 | LYAR |
| 18 | ENSG00000100243 | NM_007326 | DIA1 | 43 | ENSG00000126267 | NM_001863 | COX6B |
| 19 | ENSG00000168078 | NM_018492 | PBK | 44 | ENSG00000176621 | NM_006250 | PRH2 |
| 20 | ENSG00000105287 | NM_016457 | PRKD2 | 45 | ENSG00000162511 | NM_006762 | LAPTM5 |
| 21 | ENSG00000171345 | NM_002276 | KRT19 | 46 | ENSG00000162302 | NM_003942 | RPS6KA4 |
| 22 | ENSG00000169957 | NM_024671 | FLJ23436 | 47 | ENSG00000144055 | NM_181453, NM_014635 | GCC2 |
| 23 | ENSG00000116218 | NM_014625 | NPHS2 | 48 | ENSG00000138685 | NM_002006 | FGF2 |
| 24 | ENSG00000114405 | NM_020685 | C3orf14 | 49 | ENSG00000157227 | NM_004995 | MMP14 |
| 25 | ENSG00000180772 | NM_000686 | AGTR2 | 50 | ENSG00000141736 | NM_004448 | ERBB2 |

The 1st target nucleic acids are the PABPN1 gene, homologs thereof, transcription products or cDNAs thereof, mutants thereof, or derivatives thereof.

The 2nd target nucleic acids are the PINK1 gene, homologs thereof, transcription products or cDNAs thereof, mutants thereof, or derivatives thereof.

The 3rd target nucleic acids are the TFF2 gene, homologs thereof, transcription products or cDNAs thereof, mutants thereof, or derivatives thereof.

The 4th target nucleic acids are the EIF3S9 gene, homologs thereof, transcription products or cDNAs thereof, mutants thereof, or derivatives thereof.

The 5th target nucleic acids are the MAX gene, homologs thereof, transcription products or cDNAs thereof, mutants thereof, or derivatives thereof.

The 6th target nucleic acids are the MLL4 gene, homologs thereof, transcription products or cDNAs thereof, mutants thereof, or derivatives thereof.

The 7th target nucleic acids are the CACNB2 gene, homologs thereof, transcription products or cDNAs thereof, mutants thereof, or derivatives thereof.

The 8th target nucleic acids are the ZMYND11 gene, homologs thereof, transcription products or cDNAs thereof, mutants thereof, or derivatives thereof.

The 9th target nucleic acids are the BAT2 gene, homologs thereof, transcription products or cDNAs thereof, mutants thereof, or derivatives thereof.

The 10th target nucleic acids are the NRBP gene, homologs thereof, transcription products or cDNAs thereof, mutants thereof, or derivatives thereof.

The 11th target nucleic acids are the MCM3AP gene, homologs thereof, transcription products or cDNAs thereof, mutants thereof, or derivatives thereof.

The 12th target nucleic acids are the COL4A1 gene, homologs thereof, transcription products or cDNAs thereof, mutants thereof, or derivatives thereof.

The 13th target nucleic acids are the MKNK1 gene, homologs thereof, transcription products or cDNAs thereof, mutants thereof, or derivatives thereof.

The 14th target nucleic acids are the BBC3 gene, homologs thereof, transcription products or cDNAs thereof, mutants thereof, or derivatives thereof.

The 15th target nucleic acids are the FLJ10359 gene, homologs thereof, transcription products or cDNAs thereof, mutants thereof, or derivatives thereof.

The 16th target nucleic acids are the DPT gene, homologs thereof, transcription products or cDNAs thereof, mutants thereof, or derivatives thereof.

The 17th target nucleic acids are the C10orf76 gene, homologs thereof, transcription products or cDNAs thereof, mutants thereof, or derivatives thereof.

The 18th target nucleic acids are the DIA1 gene, homologs thereof, transcription products or cDNAs thereof, mutants thereof, or derivatives thereof.

The 19th target nucleic acids are the PBK gene, homologs thereof, transcription products or cDNAs thereof, mutants thereof, or derivatives thereof.

The 20th target nucleic acids are the PRKD2 gene, homologs thereof, transcription products or cDNAs thereof, mutants thereof, or derivatives thereof.

The 21st target nucleic acids are the KRT19 gene, homologs thereof, transcription products or cDNAs thereof, mutants thereof, or derivatives thereof.

The 22nd target nucleic acids are the FLJ23436 gene, homologs thereof, transcription products or cDNAs thereof, mutants thereof, or derivatives thereof.

The 23rd target nucleic acids are the NPHS2 gene, homologs thereof, transcription products or cDNAs thereof, mutants thereof, or derivatives thereof.

The 24th target nucleic acids are the C3orf14 gene, homologs thereof, transcription products or cDNAs thereof, mutants thereof, or derivatives thereof.

The 25th target nucleic acids are the AGTR2 gene, homologs thereof, transcription products or cDNAs thereof, mutants thereof, or derivatives thereof.

The 26th target nucleic acids are the HTR1F gene, homologs thereof, transcription products or cDNAs thereof, mutants thereof, or derivatives thereof.

The 27th target nucleic acids are the KIF3B gene, homologs thereof, transcription products or cDNAs thereof, mutants thereof, or derivatives thereof.

The 28th target nucleic acids are the DCN gene, homologs thereof, transcription products or cDNAs thereof, mutants thereof, or derivatives thereof.

The 29th target nucleic acids are the STK22C gene, homologs thereof, transcription products or cDNAs thereof, mutants thereof, or derivatives thereof.

The 30th target nucleic acids are the DKFZp566C0424 gene, homologs thereof, transcription products or cDNAs thereof, mutants thereof, or derivatives thereof.

The 31 st target nucleic acids are the CNR1 gene, homologs thereof, transcription products or cDNAs thereof, mutants thereof, or derivatives thereof.

The 32nd target nucleic acids are the HOMER3 gene, homologs thereof, transcription products or cDNAs thereof, mutants thereof, or derivatives thereof.

The 33rd target nucleic acids are the GPR2 gene, homologs thereof, transcription products or cDNAs thereof, mutants thereof, or derivatives thereof.

The 34th target nucleic acids are the FLJ12442 gene, homologs thereof, transcription products or cDNAs thereof, mutants thereof, or derivatives thereof.

The 35th target nucleic acids are the XLKD1 gene, homologs thereof, transcription products or cDNAs thereof, mutants thereof, or derivatives thereof.

The 36th target nucleic acids are the CASKIN2 gene, homologs thereof, transcription products or cDNAs thereof, mutants thereof, or derivatives thereof.

The 37th target nucleic acids are the COL5A2 gene, homologs thereof, transcription products or cDNAs thereof, mutants thereof, or derivatives thereof.

The 38th target nucleic acids are the BRD3 gene, homologs thereof, transcription products or cDNAs thereof, mutants thereof, or derivatives thereof.

The 39th target nucleic acids are the ATP6VOA4 gene, homologs thereof, transcription products or cDNAs thereof, mutants thereof, or derivatives thereof.

The 40th target nucleic acids are the PRODH2 gene, homologs thereof, transcription products or cDNAs thereof, mutants thereof, or derivatives thereof.

The 41st target nucleic acids are the EPHB2 gene, homologs thereof, transcription products or cDNAs thereof, mutants thereof, or derivatives thereof.

The 42nd target nucleic acids are the LYAR gene, homologs thereof, transcription products or cDNAs thereof, mutants thereof, or derivatives thereof.

The 43rd target nucleic acids are the COX6B gene, homologs thereof, transcription products or cDNAs thereof, mutants thereof, or derivatives thereof.

The 44th target nucleic acids are the PRH1 gene, homologs thereof, transcription products or cDNAs thereof, mutants thereof, or derivatives thereof.

The 45th target nucleic acids are the LAPTM5 gene, homologs thereof, transcription products or cDNAs thereof, mutants thereof, or derivatives thereof.

The 46th target nucleic acids are the RPS6KA4 gene, homologs thereof, transcription products or cDNAs thereof, mutants thereof, or derivatives thereof.

The 47th target nucleic acids are the GCC2 gene, homologs thereof, transcription products or cDNAs thereof, mutants thereof, or derivatives thereof.

The 48th target nucleic acids are the FGF2 gene, homologs thereof, transcription products or cDNAs thereof, mutants thereof, or derivatives thereof.

The 49th target nucleic acids are the MMP14 gene, homologs thereof, transcription products or cDNAs thereof, mutants thereof, or derivatives thereof.

The 50th target nucleic acids are the ERBB2 gene, homologs thereof, transcription products or cDNAs thereof, mutants thereof, or derivatives thereof.

1.2 <u>Kidney cancer-associated target polypeptide (1)</u>

Examples of target polypeptides as markers associated with the kidney cancer for detecting, identifying, or predicting the presence or metastasis of kidney cancer, for predicting the prognosis for a kidney cancer patient, or for identifying the presence or absence of cells to which kidney cancer has metastasized using the composition or kit of the present invention include polypeptides encoded by PABPN1, PINK 1, TFF2, EIF3S9, MAX, MLL4, CACNB2, ZMYND11, BAT2, NRBP, MCM3AP, COL4A1, MKNK1, BBC3, FLJ10359, DPT, C10orf76, DIA1, PBK, PRKD2, KRT19, FLJ23436, NPHS2, C3orf14, AGTR2, HTR1F, KIF3B, DCN, STK22C, DKFZp566C0424, CNR1, HOMER3, GPR2, FLJ12442, XLKD1, CASKIN2, COL5A2, BRD3, ATP6VOA4, PRODH2, EPHB2, LYAR, COX6B, PRH1, LAPTM5, RPS6KA4, GCC2, FGF2, MMP14, and ERBB2 genes, such as human polypeptides each comprising an amino acid sequence as shown in SEQ ID NOS: 101 to 150, homologs thereof, mutants thereof, or derivatives thereof. The terms "polypeptide," "homolog," "mutant," and "derivative" are as defined above. Examples of preferable target polypeptides are human polypeptides each comprising an amino acid sequence as shown in SEQ ID NOS: 101 to 110, 112 to 120, and 122 to 147.

According to the present invention, the expression levels of the polypeptides, which are a target of the prediction of the prognosis for kidney cancer patients and/or a target of the prediction of the metastasis of kidney cancer, significantly change (i.e., increase or decrease) in the kidney cancer tissues from the patients with poor prognoses, when compared with the kidney cancer tissues from patients with good prognoses, as with the expression levels of the corresponding genes and the transcription products thereof. Alternatively, the levels of such polypeptides in the blood significantly change (i.e., increase or decrease) in the kidney cancer patients with poor prognoses, when compared with the kidney cancer patients with good prognoses.

1.3 Kidney cancer-associated target polypeptide (2)

Other kidney cancer-associated target polypeptides as markers for detecting kidney cancer *in vitro* using the composition or kit of the present invention are polypeptides comprising the amino acid sequences as shown in SEQ ID NOS: 151 to 197, preferably SEQ ID NOS: 151, 153, 155 to 160, or SEQ ID NOS: 161 to 190, mutants thereof, or fragments thereof.

Polypeptides as shown in SEQ ID NOS: 151 to 160 and 191 and in SEQ ID NOS: 161 to 190, and 192 to 197 of the present invention are shown in Tables 2 and 3 with the gene names, protein numbers (GenBank names and accession numbers), and properties. The listed polypeptides are detected specifically in, for example, blood plasma of patients with kidney cancer, whereas they are not detected or are much lower than the detectable level in blood plasmas of healthy persons.

Table 2

| SEQ ID NO: | Gene name | Protein No. | Properties |
|---|---|---|---|
| 151 | AAK2 | P54646 | AMP kinase 2 |
| 152 | SLK4 | Q8IW52 | SLIT and NTRK-like protein 4 precursor |
| 153 | SP4L | Q8TC36 | SPerm-associated antigen 4-like protein (spermary and spermatogenesis-associated gene 4 protein) |
| 154 | C5P1 | Q96SZ6 | CDK5 regulatory subunit-associated protein 1 (CDK5 activator-binding protein C42) (CGI-05) |
| 155 | SI8A | Q92185 | $\alpha$-N-acetyl-neuraminide $\alpha$-2,8-sialyltransferase (EC 2.4.99.8) (ganglioside GD3 synthase) |
| 156 | KAP2 | P13861 | cAMP-dependent protein kinase type II-$\alpha$ regulatory chain |
| 157 | F262 | 060825 | 6-Phosphofructo-2-kinase/fructose-2,6-biphosphatase 2 (6PF-2-K/Fru-2,6-P2ASE heart isozyme) (PFK-2/FBPase-2)[6-phosphofructo-2-kinase (EC 2.7.1.105); containing fructose-2,6-biphosphatase (EC 3.1.3.46)] |
| 158 | TCPH | Q99832 | T-complex protein 1, eta subunit (TCP-1-eta) (CCT-eta) (HIV-1 Nef interacting protein) |
| 159 | GB11 | P29992 | Interacting protein-binding protein G(y), $\alpha$ subunit ($\alpha$-11) |
| 160 | CT67 | Q9H4Z3 | Protein C20orf67 |
| 191 | CEGT | Q16739 | Ceramide glucosyltransferase (EC 2.4.1.80) (glucosyltransferase) (GCS) (UDP-glucose) |

Table 3

| SEQ ID NO: | Gene name | Protein No. | Properties |
|---|---|---|---|
| 161 | AMHR2 | Q16671 | Anti-muellerian hormone type II receptor precursor (EC 2.7.1.37) (AMH type II receptor) (MIS type II receptor) (MISRII) (MRII) |
| 162 | APOL3 | O95236 | Apolipoprotein L3 (apolipoprotein L-III) (ApoL-III) (TNF-inducible protein CG12-1) |
| 163 | DEDD2 | Q8WXF8 | DNA-binding death effector domain-containing protein 2 (DED-containing protein FLAME-3) |

(continued)

| SEQ ID NO: | Gene name | Protein No. | Properties |
|---|---|---|---|
| 164 | HEXB | P07686 | β-Hexosaminidase β chain precursor (EC 3.2.1.52) (N-acetyl-β-glucosaminidase) |
| 165 | HNRPK | P61978 | Heterogeneous nuclear ribonucleoprotein K (hnRNP K) |
| 166 | KPNA1 | P52294 | Importin α-1 subunit (karyopherin α-1 subunit) (SRP1-β) (RAG protein 2) (nucleoprotein interactor 1) (NPI-1) |
| 167 | LASP1 | Q14847 | LIM and SH3 domain protein (LASP-1)(MLN 50) |
| 168 | LIAS | O43766 | Lipoic acid synthetase, mitochondrial precursor (Lip-syn) (lipoic acid synthetase) (HUSSY-01) |
| 169 | MAGEC3 | Q8TD91 | Melanoma-associated antigen C3 (MAGE-C3 antigen) |
| 170 | NFATC4 | Q14934 | Nuclear factor of activated T cells, cytoplam 4 (T cell transcription factor NFAT3) (NF-ATc4) (NF-AT3) |
| 171 | OLFM3 | Q96PB7 | Noelin 3 precursor (olfactomedin 3) (optimedin) (UNQ1924/PR04399) |
| 172 | POLR3F | Q9H1D9 | DNA-dependent RNA polymerase III 39 kDa polypeptide (EC 2.7.7.6) |
| 173 | PPP3CB | NP_066955 | Serine/threonine protein phosphatase 2B catalyst subunit, β isoform (EC 3.1.3.16) |
| 174 | SF1 | Q15637 | Splicing factor 1 (zinc finger protein 162) (transcription factor ZFM1) |
| 175 | SLC24A2 | Q9UI40 | Sodium/potassium/calcium exchanger 2 precursor (Na(+)/K(+)/Ca (2+)-exchange protein 2) |
| 176 | SLC26A4 | 043511 | Pendrin (sodium-independent chlorine/iodine transporter) |
| 177 | SLCO1A2 | P46721 | Solute carrier organic anion transporter family member 1A2 |
| 178 | TPM2 | P07951 | Tropomyosin β chain (tropomyosin 2) (β-tropomyosin) |
| 179 | UCHL5 | Q9Y5K5 | Ubiquitin carboxyl terminal hydrolase isozyme L5 (EC 3.4.19.12) (UCH-L5) |
| 180 | UGT8 | Q16880 | 2-Hydroxyacyl sphingosine 1-β-galactosyl transferase precursor (EC 2.4.1.45) (UDP- galactosylceramide transferase) |
| 181 | ZNF140 | P52738 | Zinc finger protein 140 |
| 182 | ZNF274 | Q96GC6 | Zinc finger protein 274 (zinc finger protein SP2114) (zinc finger protein HFB 101) |
| 183 | MID2 | Q9UJV3 | Midline 2 |
| 184 | LIPE | Q05469 | Lipase, hormone-sensitive |
| 185 | HDAC6 | Q9UBN7 | Histone deacetylase 6 |
| 186 | ACO2 | Q99798 | Aconitase 2, mitochondria |
| 187 | APLP1 | P51693 | Amyloid β(A4) precursor-like protein 1 |
| 188 | NUMB | P49757 | Numb homolog (Drosophila) |
| 189 | ARHGEF7 | Q14155 | Rho guanine nucleotide exchange factor (GEF)7 |
| 190 | GNAQ | P50148 | Guanine nucleotide-binding protein (G protein), q polypeptide |
| 191 | MMP2 | P08253 | 72 kDa type IV collagenase precursor (EC 3.4.24.24) (72 kDa gelatinase) |
| 192 | TNFRSF7 | P26842 | Tumor necrosis factor receptor superfamily member 7 precursor (CD27L receptor) |

(continued)

| SEQ ID NO: | Gene name | Protein No. | Properties |
|---|---|---|---|
| 193 | PDE8B | 095263 | High-affinity cAMP-specific and IBMX-nonsensitive 3',5'-cyclic hosphodiesterase 8B |
| 194 | FLOT1 | 075955 | Flotillin 1 |
| 195 | CD5 | P06127 | T-cell surface glycoprotein CD5 [precursor] |
| 196 | ECM1 | Q16610 | Extracellular matrix protein 1 |

According to the present invention, the levels of the target polypeptides for detecting kidney cancer in a biological sample such as blood are significantly or remarkably high in a subject with kidney cancer, when compared with healthy persons.

2. Probes for diagnosing kidney cancer

According to the present invention, the probes for detecting, identifying, or predicting the presence and/or metastasis of kidney cancer or for predicting the subject's prognosis after surgery are selected from the probes of group I and/or group II:

group I: polynucleotides consisting of:

(a) a polynucleotide consisting of a nucleotide sequence as shown in any of SEQ ID NOS: 1 to 10, 12 to 20, and 22 to 47, a mutant thereof, or a fragment comprising at least 15 continuous nucleotides thereof,
(b) a polynucleotide comprising a nucleotide sequence as shown in any of SEQ ID NOS: 1 to 10, 12 to 20, and 22 to 47,
(c) a polynucleotide consisting of a nucleotide sequence complementary to a nucleotide sequence as shown in any of SEQ ID NOS: 1 to 10, 12 to 20, and 22 to 47, a mutant thereof, or a fragment comprising at least 15 continuous nucleotides thereof,
(d) a polynucleotide comprising a nucleotide sequence complementary to a nucleotide sequence as shown in any of SEQ ID NOS: 1 to 10, 12 to 20, and 22 to 47, and
(e) a polynucleotide hybridizing under stringent conditions to any of the polynucleotides (a) to (d), or a fragment comprising at least 15 continuous nucleotides thereof; and

group II: antibodies, fragments thereof or chemically modified derivatives thereof consisting of:

(f) an antibody specifically binding to at least one of a polypeptide encoded by a nucleotide sequence as shown in any of SEQ ID NOS: 1 to 10, 12 to 20, and 22 to 47 or a polypeptide having an amino acid sequence as shown in any of SEQ ID NOS: 101 to 110, 112 to 120, and 122 to 147, a mutant thereof, and a fragment thereof, a fragment of the antibody, or a chemically modified derivative of the antibody or fragment,
(g) an antibody specifically binding to at least one of a polypeptide having an amino acid sequence as shown in any of SEQ ID NOS: 151, 153, and 155 to 160, a mutant thereof, and a fragment thereof, a fragment of the antibody, or a chemically modified derivative of the antibody, and
(h) an antibody specifically binding to at least one of a polypeptide having an amino acid sequence as shown in any of SEQ ID NOS: 161 to 190, a mutant thereof, and a fragment thereof, a fragment of the antibody, or a chemically modified derivative of the antibody.

All of the above-described probes can bind to any of the kidney cancer-associated markers described in Sections 1.1 to 1.3 above, and they can be used to detect, identify or predict the presence or metastasis of kidney cancer. For example, any probe of group I and group II (f) enables detection, identification, or prediction of the presence or metastasis of kidney cancer. Also, any probe of group II (g) and (h) enables detection, identification, or prediction of the presence of kidney cancer.
According to the present invention, the nucleic acid probe includes DNA or RNA, and the antibody probe includes, for example, a polyclonal antibody, a monoclonal antibody, a fragment thereof, a synthetic antibody, a recombinant antibody, a polyspecific antibody, and a single-chain antibody.
We have now found that the probes as described in group I and/or group II could be used for detecting, identifying, or predicting the presence and/or metastasis of kidney cancer for the first time.

3. Composition for diagnosing kidney cancer and/or for predicting the prognosis or metastasis of kidney cancer

3.1 Nucleic acid composition

According to the present invention, the nucleic acid composition for detecting, identifying, or predicting the presence and metastasis of kidney cancer, for predicting the prognosis for a kidney cancer patient, and for identifying the presence or absence of to which kidney cancer has metastasized using the composition or kit of the present invention comprises one or more probes of group I as described in Section 2 above. Such composition enables qualitative and/or quantitative measurements of the presence, expression level, or amount of human derived PABPN1, PINK1, TFF2, EIF3S9, MAX, MLL4, CACNB2, ZMYND11, BAT2, NRBP, MCM3AP, COL4A1, MKNK1, BBC3, FLJ10359, DPT, C10orf76, DIA1, PBK, PRKD2, KRT19, FLJ23436, NPHS2, C3orf14, AGTR2, HTR1F, KIF3B, DCN, STK22C, DKFZp566C0424, CNR1, HOMER3, GPR2, FLJ12442, XLKD1, CASKIN2, COL5A2, BRD3, ATP6VOA4, PRODH2, EPHB2, LYAR, COX6B, PRH1, LAPTM5, RPS6KA4, GCC2, FGF2, MMP14, and ERBB2 genes, homologs thereof, transcription products or cDNA thereof, mutants thereof, or derivatives thereof, as target nucleic acids for predicting the prognosis or metastasis of kidney cancer.
The expression levels of said target nucleic acids significantly change (i.e., increase or decrease) in the kidney cancer tissue from a patient with poor prognosis, when compared with the kidney cancer tissue from a patient with good prognosis. Accordingly, the composition of the present invention can be effectively used for measuring and comparing the expression levels of the target nucleic acids both in the kidney cancer tissue from a patient with good prognosis and in the kidney cancer tissue from a patient with poor prognosis.
The compositions usable in the present invention include a combination of one or more polynucleotides selected from: polynucleotides comprising the nucleotide sequences as shown in SEQ ID NOS: 1 to 50 as observed in the body tissue of a patient with kidney cancer, and polynucleotides complementary thereto; polynucleotides hybridizing under stringent conditions to DNA consisting of nucleotide sequences complementary to said nucleotide sequences, and polynucleotides complementary thereto; and polynucleotides comprising at least 15 continuous nucleotides in the nucleotide sequences of said polynucleotides.
Specifically, the composition of the present invention can comprise one or more polynucleotides or fragments thereof set forth below:

(1) polynucleotides each consisting of a nucleotide sequence as shown in any of SEQ ID NOS: 1 to 50, mutants thereof, or fragments comprising at least 15 continuous nucleotides thereof;
(2) polynucleotides each comprising a nucleotide sequence as shown in any of SEQ ID NOS: 1 to 50;
(3) polynucleotides each consisting of a nucleotide sequence as shown in any of SEQ ID NOS: 1 to 10, 12 to 20, and 22 to 47, mutants thereof, or fragments comprising at least 15 continuous nucleotides thereof;
(4) polynucleotides each comprising a nucleotide sequence as shown in any of SEQ ID NOS: 1 to 10, 12 to 20, and 22 to 47;
(5) polynucleotides each consisting of a nucleotide sequence complementary to a nucleotide sequence as shown in any of SEQ ID NOS: 1 to 10, 12 to 20, and 22 to 47, mutants thereof, or fragments comprising at least 15 continuous nucleotides thereof;
(6) polynucleotides each comprising a nucleotide sequence complementary to a nucleotide sequence as shown in any of SEQ ID NOS: 1 to 10, 12 to 20, and 22 to 47;
(7) polynucleotides each hybridizing under stringent conditions to DNA consisting of a nucleotide sequence complementary to a nucleotide sequence as shown in any of SEQ ID NOS: 1 to 10, 12 to 20, and 22 to 47, or fragments comprising at least 15 continuous nucleotides thereof;
(8) polynucleotides each hybridizing under stringent conditions to DNA consisting of a nucleotide sequence as shown in any of SEQ ID NOS: 1 to 10, 12 to 20, and 22 to 47 or fragments comprising at least 15 continuous nucleotides thereof;
(9) a polynucleotide consisting of the nucleotide sequence as shown in any of SEQ ID NOS: 1 to 11, 21, and 48 to 50, mutants thereof, or fragments comprising at least 15 continuous nucleotides thereof;
(10) polynucleotides comprising a nucleotide sequence as shown in any of SEQ ID NOS: 11, 21, and 48 to 50;
(11) polynucleotides consisting of a nucleotide sequence complementary to a nucleotide sequence as shown in SEQ ID NOS: 11, 21, and 48 to 50, mutants thereof, or fragments comprising at least 15 continuous nucleotides thereof;
(12) polynucleotides comprising a nucleotide sequence complementary to the nucleotide sequence as shown in any of SEQ ID NOS: 11, 21, and 48 to 50;
(13) polynucleotides each hybridizing under stringent conditions to DNA consisting of a nucleotide sequence complementary to the nucleotide sequence as shown in any of SEQ ID NOS: 11, 21, and 48 to 50 or fragments comprising at least 15 continuous nucleotides thereof; and

(14) polynucleotides each hybridizing under stringent conditions to DNA consisting of a nucleotide sequence as shown in any of SEQ ID NOS: 11, 21, and 48 to 50 or fragments comprising at least 15 continuous nucleotides thereof.

Fragments of the polynucleotide as described in (1) to (14) above can include, but are not limited to, nucleotide sequences of, for example, continuous 15 to all nucleotides, 15 to 5000 nucleotides, 15 to 4500 nucleotides, 15 to 4000 nucleotides, 15 to 3500 nucleotides, 15 to 3000 nucleotides, 15 to 2500 nucleotides, 15 to 2000 nucleotides, 15 to 1500 nucleotides, 15 to 1000 nucleotides, 15 to 900 nucleotides, 15 to 800 nucleotides, 15 to 700 nucleotides, 15 to 600 nucleotides, 15 to 500 nucleotides, 15 to 400 nucleotides, 15 to 300 nucleotides, 15 to 250 nucleotides, 15 to 200 nucleotides, 15 to 150 nucleotides, 15 to 140 nucleotides, 15 to 130 nucleotides, 15 to 120 nucleotides, 15 to 110 nucleotides, 15 to 100 nucleotides, 15 to 90 nucleotides, 15 to 80 nucleotides, 15 to 70 nucleotides, 15 to 60 nucleotides, 15 to 50 nucleotides, 15 to 40 nucleotides, 15 to 30 nucleotides or 15 to 25 nucleotides, 25 to all nucleotides, 25 to 1000 nucleotides, 25 to 900 nucleotides, 25 to 800 nucleotides, 25 to 700 nucleotides, 25 to 600 nucleotides, 25 to 500 nucleotides, 25 to 400 nucleotides, 25 to 300 nucleotides, 25 to 250 nucleotides, 25 to 200 nucleotides, 25 to 150 nucleotides, 25 to 140 nucleotides, 25 to 130 nucleotides, 25 to 120 nucleotides, 25 to 110 nucleotides, 25 to 100 nucleotides, 25 to 90 nucleotides, 25 to 80 nucleotides, 25 to 70 nucleotides, 25 to 60 nucleotides, 25 to 50 nucleotides or 25 to 40 nucleotides, 50 to all nucleotides, 50 to 1000 nucleotides, 50 to 900 nucleotides, 50 to 800 nucleotides, 50 to 700 nucleotides, 50 to 600 nucleotides, 50 to 500 nucleotides, 50 to 400 nucleotides, 50 to 300 nucleotides, 50 to 250 nucleotides, 50 to 200 nucleotides, 50 to 150 nucleotides, 50 to 140 nucleotides, 50 to 130 nucleotides, 50 to 120 nucleotides, 50 to 110 nucleotides, 50 to 100 nucleotides, 50 to 90 nucleotides, 50 to 80 nucleotides, 50 to 70 nucleotides or 50 to 60 nucleotides, 60 to all nucleotides, 60 to 1000 nucleotides, 60 to 900 nucleotides, 60 to 800 nucleotides, 60 to 700 nucleotides, 60 to 600 nucleotides, 60 to 500 nucleotides, 60 to 400 nucleotides, 60 to 300 nucleotides, 60 to 250 nucleotides, 60 to 200 nucleotides, 60 to 150 nucleotides, 60 to 140 nucleotides, 60 to 130 nucleotides, 60 to 120 nucleotides, 60 to 110 nucleotides, 60 to 100 nucleotides, 60 to 90 nucleotides, and 60 to 80 nucleotides or 60 to 70 nucleotides, and so on.

According to an embodiment of the invention, fragments of polynucleotides each comprising the nucleotide sequence as shown in any of SEQ ID NOS: 1 to 10, 12 to 20, and 22 to 47 preferably comprise a nucleotide sequence as shown in any of SEQ ID NOS: 51 to 60, 62 to 70, and 72 to 97, a complementary sequence thereof, or a partial sequence comprising at least 15 continuous nucleotides thereof.

The composition of the present invention includes the following polynucleotide or polynucleotides, for example.

(1) a polynucleotide comprising at least 15 continuous nucleotides in each of the nucleotide sequence as shown in any of SEQ ID NOS: 1 to 10, 12 to 20, and 22 to 47 or complementary sequences thereof;

(2) a polynucleotide comprising at least 60 continuous nucleotides in each of the nucleotide sequence as shown in any of SEQ ID NOS: 1 to 10, 12 to 20, and 22 to 47 or complementary sequences thereof;

(3) a polynucleotide comprising at least 15 continuous nucleotides in the nucleotide sequence as shown in any of SEQ ID NOS: 11, 21, and 48 to 50 or a complementary sequence thereof;

(4) a polynucleotide comprising at least 60 continuous nucleotides in the nucleotide sequence as shown in any of SEQ ID NOS: 11, 21, and 48 to 50 or a complementary sequence thereof;

(5) a polynucleotide comprising a nucleotide sequence as shown in any of SEQ ID NOS: 51 to 60, 62 to 70, and 72 to 97 in the nucleotide sequence as shown in SEQ ID NOS: 1 to 10, 12 to 20, and 22 to 47, and comprising at least 60 continuous nucleotides;

(6) a polynucleotide comprising a sequence complementary to a nucleotide sequence as shown in any of SEQ ID NOS: 51 to 60, 62 to 70, and 72 to 97 in the sequences complementary to the nucleotide sequence as shown in any of SEQ ID NOS: 1 to 10, 12 to 20, and 22 to 47, and comprising at least 60 continuous nucleotides;

(7) a polynucleotide comprising the nucleotide sequence as shown in any of SEQ ID NOS: 61, 71, and 98 to 100 in the nucleotide sequence as shown in any of SEQ ID NOS: 11, 21, and 48 to 50, and comprising at least 60 continuous nucleotides; and

(8) a polynucleotide comprising a sequence complementary to the nucleotide sequence as shown in any of SEQ ID NOS: 61, 71, and 98 to 100 in the sequence complementary to the nucleotide sequence as shown in any of SEQ ID NOS: 11, 21, and 48 to 50, and comprising at least 60 continuous nucleotides.

The polynucleotides or fragments thereof as used in the invention may be DNA or RNA.

Polynucleotides in the compositions of the present invention can be prepared by common techniques such as recombinant DNA technology, PCR, or a method of using an automatic DNA/RNA synthesizer.

Recombinant DNA technology or PCR can include the use of the techniques as disclosed in, for example, Ausubel. et al., Current Protocols in Molecular Biology, John Willey & Sons, US (1993); or Sambrook et al., Molecular Cloning: A Laboratory Manual, Cold Spring Harbor Laboratory Press, US (1989).

The human-derived PABPN1, PINK1, TFF2, EIF3S9, MAX, MLL4, CACNB2, ZMYND11, BAT2, NRBP, MCM3AP, COL4A1, MKNK1, BBC3, FLJ10359, DPT, C10orf76, DIA1, PBK, PRKD2, KRT19, FLJ23436, NPHS2, C3orf14, AGTR2, HTR1F, KIF3B, DCN, STK22C, DKFZp566C0424, CNR1, HOMER3, GPR2, FLJ12442, XLKD1, CASKIN2, COL5A2, BRD3, ATP6VOA4, PRODH2, EPHB2, LYAR, COX6B, PRH1, LAPTM5, RPS6KA4, GCC2, FGF2, MMP14, and ERBB2 genes are known, and the methods for obtaining the same are also known. Thus, these genes can be cloned in order to prepare polynucleotides as the compositions of the present invention.

Polynucleotides constituting the compositions of the present invention may be chemically synthesized using an automatic DNA synthesizer. Such synthesis is generally carried out by the phosphoramidite method, which enables the automatic synthesis of a single-stranded DNA of at most about 100 nucleotides. The automatic DNA synthesizer is commercially available from, for example, Polygen, ABI, or Applied BioSystems.

Alternatively, the polynucleotides of the present invention can be prepared by cDNA cloning. Total RNA is extracted from a tissue of a living body, such as kidney tissue, in which the target gene or genes of the present invention is/are expressed, the extracted total RNA is applied to the oligo dT cellulose column to obtain poly A(+) RNA, cDNA library is prepared therefrom by RT-PCR, and the target cDNA clones can be obtained from the resulting library by a screening method such as hybridization screening, expression screening, or antibody screening. If necessary, the cDNA clones may be amplified by PCR. Probes or primers can be selected and synthesized from any sequences comprising 15 to 100 continuous nucleotides in the nucleotide sequences as shown in SEQ ID NOS: 1 to 50. The cDNA cloning technique is described in, for example, Sambrook, J. & Russel, D., Molecular Cloning, A LABORATORY MANUAL, Cold Spring Harbor Laboratory Press, January 15, 2001, vol. 1: 7.42 to 7.45, vol. 2: 8.9 to 8.17.

3.2 Antibody composition

The compositions of the present invention can comprise, as probe for diagnosing kidney cancer, the antibodies described in group II of Section 2 above, fragments thereof, or chemically-modified derivatives thereof. Such compositions are useful for detecting, identifying, or predicting *in vitro* the presence and/or metastasis of kidney cancer in a subject afflicted with kidney cancer. In the present invention, prediction of metastasis can lead to prediction of good or poor prognosis for the subject after surgery.

(A) The first example of the antibody composition of the present invention is a composition comprising: one or more antibodies against polypeptides having the amino acid sequence as shown in any of SEQ ID NOS: 101 to 110, 112 to 120, and 122 to 147 of group II (f), fragments thereof, or chemically modified derivatives thereof.
The composition of the present invention can further comprise one or more antibodies against a polypeptide having the amino acid sequence as shown in any of SEQ ID NOS: 111, 121, and 148 to 150, a mutant of the polypeptide, or a fragment of the polypeptide; a fragment of the antibody; or a chemically modified derivative of the antibody. Use of such antibodies in combination can improve the accuracy for predicting the prognosis.
In order to detect polypeptides encoded by the PABPN1, PINK1, TFF2, EIF3S9, MAX, MLL4, CACNB2, ZMYND11, BAT2, NRBP, MCM3AP, COL4A1, MKNK1, BBC3, FLJ10359, DPT, C10orf76, DIA1, PBK, PRKD2, KRT19, FLJ23436, NPHS2, C3orf14, AGTR2, HTR1F, KIF3B, DCN, STK22C, DKFZp566C0424, CNR1, HOMER3, GPR2, FLJ12442, XLKD1, CASKIN2, COL5A2, BRD3, ATP6VOA4, PRODH2, EPHB2, LYAR, COX6B, PRH1, LAPTM5, RPS6KA4, GCC2, FGF2, MMP14, and ERBB2 genes, as markers for predicting the prognosis or metastasis of kidney cancer, homologs thereof, mutants thereof, or derivatives thereof, at least 1, and preferably at least 2, antibodies against such polypeptides can be used in combination in the present invention.
(B) The second example of the antibody composition of the present invention is a composition comprising: one or more antibodies against polypeptides having the amino acid sequence as shown in any of SEQ ID NOS: 151 to 160 and 191 and SEQ ID NOS: 161 to 190, and 192 to 197 of group II (g) and (h), respectively, fragments thereof, or chemically modified derivatives thereof.

Such polypeptides are encoded by the genes shown in Tables 1, 2, and 3. The nucleotide sequences of such genes can be easily obtained based on the gene names shown in the tables by accessing the NCBI website.

The above-mentioned polypeptides can be obtained by the recombinant DNA technology. For example, the cDNA clones obtained in the above-described manner are incorporated into an expression vector, which is then transformed or transfected into prokaryotic or eukaryotic host cells, and the resulting prokaryotic or eukaryotic host cells are cultured. Thus, polypeptides of interest can be obtained from the cells or culture supernatants. Vectors and expression systems are commercially available from Novagen, Takara Shuzo, Daiichi Pure Chemicals, Qiagen, Stratagene, Promega, Roche Diagnostics, Invitrogen, Genetics Institute, or Amersham Bioscience.

Examples of host cells that can be used include prokaryotic cells such as bacteria (e.g., *E. coli* or *Bacillus subtilis*), yeast (e.g., *Saccharomyces cerevisiae*), insect cells (e.g., Sf cells), and mammalian cells (e.g., COS, CHO, and BHK cells).

Vectors can comprise, in addition to DNA encoding each of the aforementioned polypeptides, regulatory elements such as promoter, enhancer, polyadenylation signal, ribosome-binding site, replication origin, terminator, and selection marker. Moreover, in order to facilitate the purification of a polypeptide, a peptidic label may be added to the C- or N-terminus of the polypeptide to form a fusion polypeptide. Examples of representative peptidic labels include, but are not limited to, (histidine)$_{6-10}$ repeat, FLAG, myc peptide, and GFP polypeptide. The recombinant DNA techniques are described in Sambrook, J. & Russel, D. (*supra*).

When the polypeptides of the present invention are produced without the addition of a peptidic label, the polypeptides can be purified by, for example, ultrafiltration, salting-out, gel filtration, or ion-exchange chromatography. In addition thereto, affinity chromatography, HPLC, hydrophobic chromatography, isoelectric chromatography or the like may be carried out in combination. When the protein has a peptidic label, such as histidine repeat, FLAG, myc, or GFP, affinity chromatography suitable for each peptidic label can be carried out in accordance with conventional techniques. Construction of such an expression vector that facilitates isolation or purification is preferable. When the expression vector is constructed so as to express in the form of the fusion polypeptide of a polypeptide with a peptidic label, and such vector is used to prepare the polypeptide by genetic engineering techniques, isolation or purification of the polypetide is easy.

According to the present invention, the mutants of the above polypeptides are a mutant comprising a deletion, substitution, addition, or insertion of one or more amino acids, preferably one or several amino acids, in each of the amino acid sequences as shown in SEQ ID NOS: 101 to 197 or partial sequences thereof; or alternatively a mutant having an identity of about 80% or higher, about 85% or higher, preferably about 90% or higher, more preferably about 95% or higher, about 97% or higher, about 98% or higher, or about 99% or higher with said amino acid sequence or a partial sequence thereof, as defined above. Examples of such mutants include naturally-occurring mutants, such as a homolog of a mammalian species other than human, a mutant thereof based on human polymorphism or splicing mutation, or the like.

According to the present invention, a fragment of the polypeptide or mutant thereof consists of at least 5, at least 7, at least 10, at least 15, preferably at least 20, at least 25, more preferably at least 30, at least 40, or at least 50 continuous amino acid residues in the amino acid sequence of the polypeptide, and has a single epitope or a plurality of epitomes. Such a fragment can immunospecifically bind to the antibody of the present invention or a fragment thereof. The polypeptide may be cleaved or fragmented by an enzyme that is present in the body, such as protease or peptidase, thereby being present as fragments.

The thus-obtained antibody that recognizes the polypeptide can specifically bind to the polypeptide via an antigen-binding site of the antibody. Specifically, a polypeptide having an amino acid sequence as shown in any of SEQ ID NOS: 101 to 197, a fragment thereof, a mutant thereof, or a fusion polypeptide can be used as an immunogen to produce immunoreactive antibodies.

More specifically, the polypeptide, a fragment thereof, a mutant thereof, or a fusion polypeptide comprises an antigenic determinant or epitope that elicits antibody formation, which antigen determinant or epitope may have a linear structure or a higher-order (or disconnected) structure. Such antigen determinant or epitope can be identified by any epitope analysis known in the art, such as phage display or reverse immunogenetics.

Antibodies of any aspect are elicited by the polypeptides of the present invention. If all, part, or an epitope of the polypeptide is isolated, a polyclonal or monoclonal antibody can be prepared in accordance with conventional techniques. An example of the method for preparing an antibody is described in Kennet et al. (ed.), Monoclonal Antibodies, Hybridomas: A New Dimension in Biological Analyses, Plenum Press, New York, 1980.

A polyclonal antibody can be prepared by immunizing an animal such as a bird (e.g., a chicken) or a mammalian (e.g., a rabbit, goat, horse, sheep, or mouse) with the polypeptide of the invention. The antibody of interest can be purified from the blood of an immunized animal via an appropriate combination of techniques such as ammonium sulfate fractionation, ion-exchange chromatography, and affinity chromatography.

A monoclonal antibody can be obtained by the technique comprising producing hybridoma cell lines that produce monoclonal antibodies specific for each relevant polypeptide in a mouse in accordance with conventional techniques. One method for producing such hybridoma cell lines comprises immunizing an animal with an enzyme polypeptide of the invention, removing spleen cells from the immunized animal, fusing the spleen cells with a myeloma cell line to produce hybridoma cells therefrom, and identifying a hybridoma cell line that produces a monoclonal antibody binding to the enzyme of interest. A monoclonal antibody can be recovered by a conventional technique.

The antibody of the present invention is not particularly limited, provided that such antibody can bind specifically to the target polypeptide of the present invention or a fragment thereof. The antibody usable in the invention is a monoclonal or polyclonal antibody, preferably monoclonal antibody. Examples of other antibodies include a recombinant antibody, a synthetic enzyme, a polyspecific antibody (e.g., a bispecific antibody), a single-stranded antibody, and an antibody fragment. Specific examples of such antibodies include Fab, F(ab')$_2$, scFv, Fv, and dsFv. The globulin type of the antibody of the present invention is not particularly limited, as long as the antibody has the aforementioned properties. It may be any of IgG, IgM, IgA, IgE, and IgD.

Preparation of monoclonal antibody

(1) Immunization and collection of antibody-producing cell

The immunogen which is a target polypeptide is administered to a mammalian animal such as rat, mouse (e.g., the inbred mouse strain Balb/c), or rabbit. The dose of the immunogen is appropriately determined depending on, for example, the type of an animal to be immunized or the route of administration, and it is about 50 to 200 μg per animal. Immunization is primarily performed by injecting an immunogen subcutaneously or intraperitoneally. The intervals of immunization are not particularly limited. After the primary immunization, boost immunization is carried out 2 to 10 times, preferably 3 or 4 times, at the intervals of several days to several weeks, and preferably at the intervals of 1 to 4 weeks. After the primary immunization, the antibody titer of the blood serum of the immunized animal is repeatedly measured by, for example, enzyme-linked immuno sorbent assay (ELISA). When the antibody titer reached a plateau, the immunogen is injected intravenously or intraperitoneally to complete the final immunization. The antibody-producing cells are recovered 2 to 5 days, preferably 3 days, after the final immunization. Examples of antibody-producing cells include spleen cells, lymph node cells, and peripheral blood cells, preferably spleen cells or regional lymph node cells.

(2) Cell fusion

Hybridoma cell lines that produce monoclonal antibodies specific for target polypeptides are prepared. Such hybridomas can be produced and identified via conventional techniques. The method for producing such hybridoma cell lines comprises immunizing an animal with a protein of the invention, removing spleen cells from the immunized animal, fusing the spleen cells with a myeloma cell line, producing hybridoma cells therefrom, and identifying a hybridoma cell line that produces a monoclonal antibody binding to the enzyme of interest. Myeloma cell lines to be fused with antibody-producing cells can be commercially available established cell lines of animals such as mice. Preferably, cell lines to be used have drug selectivity; namely, they cannot survive in the HAT selection medium (containing hypoxanthine, aminopterin, and thymidine) in an unfused state, while they can survive only in a state fused with antibody-producing cells. The established cells are preferably derived from an animal of the same species with the animal to be immunized. A specific example of the myeloma cell line is the strain P3X63-Ag.8 (ATCC TIB9), which is a BALB/c mouse-derived hypoxanthine guanine phosphoribosyl-transferase (HGPRT) deficient cell line.

Subsequently, the myeloma cell lines are fused with the antibody-producing cells. Cell fusion is carried out in a serum-free medium for animal cell culture, such as DMEM or RPMI-1640 medium, by mixing the antibody-producing cells with the myeloma cell lines at about 1:1 to 20:1 in the presence of a cell fusion accelerator. As the cell fusion accelerator, polyethylene glycol having an average molecular weight of 1,500 to 4,000 daltons can be used at a concentration of about 10 to 80%, for example. Optionally, an auxiliary agent, such as dimethyl sulfoxide, can be used in combination in order to enhance the fusion efficiency. Further, the antibody-producing cells can be fused with the myeloma cell lines by using a commercially available cell fusion apparatus utilizing electric stimulus (e.g., electroporation).

(3) Selection and cloning of hybridomas

The hybridomas of interest are selected from the fused cells. To this end, the cell suspension is adequately diluted in, for example, a fetal bovine serum-containing RPMI-1640 medium, the suspension is aliquoted into each well of a microtiter plate at about two million cells/well, a selection medium to each well, and culture is thereafter carried out while appropriately exchanging the selection medium with the same fresh medium. The culture temperature is 20°C to 40°C, preferably about 37°C. When the myeloma cell is an HGPRT-deficient strain or thymidine kinase-deficient strain, a hybridoma of a cell having an ability to produce an antibody and a myeloma cell line can selectively be cultured and grown in the selection medium containing hypoxanthine, aminopterin, and thymidine (i.e., the HAT medium). As a result, cells grown about 14 days after the initiation of culture in the selection medium can be obtained as the hybridoma.

Subsequently, whether or not the culture supernatant of the grown hybridoma contains the antibody of interest is screened for. Screening of hybridomas can be carried out in accordance with conventional techniques, without particular limitation. For example, the culture supernatant in the well containing the grown hybridomas is partially sampled and then subjected to enzyme immuno assay (EIA) or ELISA or radio immuno assay (RIA). The fused cells are cloned using the limiting dilution method or the like, and monoclonal antibody-producing cells, i.e. hybridomas, are established in the end. The hybridoma of the present invention is stable during the culture in a basic medium, such as RPMI-1640 or DMEM, as described below, and the hybridoma can produce and secrete a monoclonal antibody that reacts specifically with a target polypeptide.

(4) Recovery of antibody

Monoclonal antibody can be recovered by conventional techniques. Specifically, a monoclonal antibody can be collected from the established hybridoma by the conventional cell culture technique, the ascites development, or the like. According to the cell culture technique, hybridoma is cultured in an animal cell culture medium, such as 10% fetal bovine serum-containing RPMI-1640 medium, MEM medium, or a serum-free medium, under common culture conditions (e.g., 37°C, 5% $CO_2$) for 2 to 10 days, and the antibody is obtained from the culture supernatant. In the case of the ascites development, about 10 millions of myeloma-derived hybridomas cells are administered intraperitoneally to an animal of the same species as the mammal from which the myeloma cell is derived, so as to allow the hybridoma cells to grow in a large quantity. After one to two weeks, the ascites or blood serum is collected from said animal.

When the purification of an antibody is required in the above-described method for collecting the antibody, the conventional techniques, such as salting out by ammonium sulfate, ion-exchange chromatography, affinity chromatography, and gel filtration chromatography, may be appropriately selected or combined to obtain the purified monoclonal antibody.

Prep-aration of polyclonal antibody

When polyclonal antibodies are prepared, an animal such as rabbit is immunized in the same manner as described above, the antibody titer is measured 6 to 60 days after the final immunization by enzyme immunoassay (EIA or ELISA) or radio immunoassay (RIA), and blood is taken on the day the maximal antibody titer is measured, in order to obtain antiserum. Thereafter, the reactivity of the polyclonal antibodies in the antiserum is assayed by ELISA or the like.

Chemically modified derivative

The antibody of the present invention or a fragment thereof may be a chemically modified derivative. Examples include derivatives labeled with an enzyme, fluorophore, or radioisotope, and chemically modified derivatives, such as an acetylated, acylated, alkylated, phosphorylated, sulfated, or glycosylated derivatives.

Examples of the labels for use in enzyme immunoassay include enzymes such as peroxidase (POD), alkaline phosphatase, β-galactosidase, urease, catalase, glucose oxidase, lactate dehydrogenase, amylase, and biotin-avidin complexes. Examples of the labels for use in fluorescence immunoassay include fluorescent substances or fluorophores, such as fluorescein isothiocyanate, tetramethylrhodamine isothiocyanate, substituted rhodamine isothiocyanate, dichlorotriazine isothiocyanate, Alexa, and AlexaFluoro. Examples of the labels for use in radio immunoassay include radioactive isotopes, such as tritium, iodine ($^{131}$I, $^{125}$I, $^{123}$I, and $^{121}$I), phosphorus ($^{32}$P), sulfur ($^{35}$S), and metals (e.g., $^{68}$Ga, $^{67}$Ga, $^{68}$Ge, $^{54}$Mn, $^{99}$Mo, $^{99}$Tc, and $^{133}$Xe). Examples of the labels for use in luminescent immunoassay include luminescent molecules, luminescent substances, or bioluminescent substances, such as an NADH-, FMNH2-, luciferase system, luminol-hydrogen peroxide-POD system, acridinium ester system, or dioxetane compound system.

Alternatively, an avidin-biotin system or streptavidin-biotin system can also be used optionally. In such a case, biotin may be bound to the antibodies of the present invention or fragments thereof, for example. A label can be bound to an antibody by conventional techniques, such as the glutaraldehyde method, the maleimide method, the pyridyl disulfide method, or the periodic acid method in the case of enzyme immunoassay. In radioimmunoassay, the binding of label and antibody can be carried out in accordance with the conventional techniques, such as the chloramine-T method and Bolton-Hunter method.

4. Kit for diagnosis of kidney cancer and/or prediction of the prognosis or metastasis of kidney cancer

4.1 Nucleic acid kit

The present invention also provides a kit for detecting, identifying, or predicting *in vitro* the presence, metastasis, or prognosis of kidney cancer, comprising one or more polynucleotides of group I described in Section 2 above, mutants thereof, and/or fragments thereof.

The kit of the present invention can comprise nucleic acid probes selected from group I as shown below. Such probes may be packaged in suitable containers, alone or in combination.

The kit of the present invention can comprise at least one of a polynucleotide comprising the nucleotide sequence as shown in any of SEQ ID NOS: 1 to 10, 12 to 20, and 22 to 47, a polynucleotide comprising a complementary sequence thereof, a polynucleotide hybridizing under stringent conditions to said polynucleotide, or a fragment thereof.

The kit of the present invention can further comprise at least one of a polynucleotide comprising the nucleotide sequence as shown in any of SEQ ID NOS: 11, 21, and 48 to 50, a polynucleotide comprising a complementary sequence thereof, a polynucleotide hybridizing under stringent conditions to said polynucleotide, or a fragment thereof.

A fragment of the polynucleotide, which can be contained in the kit of the invention, is, for example, at least one DNA selected from the following groups (1) to (5):

(1) DNA comprising at least 15 continuous nucleotides in the nucleotide sequence as shown in any of SEQ ID NOS: 1 to 10, 11, 12 to 20, 21, 22 to 47, and 48 to 50 or a complementary sequence thereof;
(2) DNA comprising at least 60 continuous nucleotides in the nucleotide sequence as shown in any of SEQ ID NOS: 1 to 10, 11, 12 to 20, 21, 22 to 47, and 48 to 50 or a complementary sequence thereof;
(3) DNA comprising the nucleotide sequence as shown in any of SEQ ID NOS: 51 to 60, 61, 62 to 70, 71, 72 to 97, and 98 to 100 or a complementary sequence thereof and comprising at least 60 continuous nucleotides in the nucleotide sequence as shown in any of SEQ ID NOS: 1 to 10, 11, 12 to 20, 21, 22 to 47, and 48 to 50 or a complementary sequence thereof;
(4) DNA consisting of the nucleotide sequence as shown in any of SEQ ID NOS: 51 to 60, 61, 62 to 70, 71, 72 to 97, and 98 to 100; or
(5) DNA comprising a nucleotide sequence complementary to the nucleotide sequence as shown in any of SEQ ID NOS: 51 to 60, 61, 62 to 70, 71, 72 to 97, and 98 to 100.

According to a preferable embodiment, the polynucleotide is a polynucleotide consisting of a nucleotide sequence as shown in any of SEQ ID NOS: 1 to 10, 12 to 20, and 22 to 47, a polynucleotide consisting of a complementary sequence thereof, a polynucleotide hybridizing under stringent conditions to said polynucleotide, or a fragment comprising at least 15 continuous nucleotides thereof.

According to another preferable embodiment, the kit of the present invention can further comprise, in addition to the above polynucleotide, a polynucleotide consisting of the nucleotide sequence as shown in any of SEQ ID NOS: 11, 21, and 48 to 50, a polynucleotide consisting of a complementary sequence thereof, a polynucleotide hybridizing under stringent conditions to said polynucleotide, or a fragment comprising at least 15 continuous nucleotides thereof.

According to a preferable embodiment, the fragment can be a polynucleotide comprising at least 15, preferably at least 60 continuous nucleotides.

According to another preferable embodiment, the fragment is a polynucleotide comprising a nucleotide sequence as shown in any of SEQ ID NOS: 51 to 60, 61, 62 to 70, 71, 72 to 97, and 98 to 100 and comprising at least 60 continuous nucleotides in the nucleotide sequence as shown in any of SEQ ID NOS: 1 to 10, 11, 12 to 20, 21, 22 to 47, and 48 to 50, respectively, or a polynucleotide comprising a nucleotide sequence complementary thereto.

According to another preferable embodiment, the fragment is a polynucleotide comprising a nucleotide sequence as shown in any of SEQ ID NOS: 51 to 60, 61, 62 to 70, 71, 72 to 97, and 98 to 100.

According to another preferable embodiment, the fragment is a polynucleotide comprising a nucleotide sequence complementary to a nucleotide sequence as shown in any of SEQ ID NOS: 51 to 60, 61, 62 to 70, 71, 72 to 97, and 98 to 100.

According to another preferable embodiment, the fragment is a polynucleotide consisting of a nucleotide sequence as shown in any of SEQ ID NOS: 51 to 60, 61, 62 to 70, 71, 72 to 97, and 98 to 100.

Specific examples of the aforementioned combinations include: a polynucleotide comprising the nucleotide sequence as shown in SEQ ID NO: 1 or 2 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 3 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 4 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 5 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 6 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 7 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 8 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 9 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 10 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 11 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 12 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 13 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 14 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 15 or a complementary sequence thereof and/or a fragment thereof; a polynucleotide comprising a nucleotide sequence as shown in any of SEQ ID NOS: 1 to 16 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 17 or a complementary sequence thereof, and/or a fragment thereof; a polynucleotide comprising a nucleotide sequence as shown in any of SEQ ID NOS: 1 to 18 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 19 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 20 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 21 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 22 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 23 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 24 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 25 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 26 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 27 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 28 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 29 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 30 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 31 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 32 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 33 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 34 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 35 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 36 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 37 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 38 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 39 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 40 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 41 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 42 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 43 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 44 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 45 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 46 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 47 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 48 or a complementary sequence thereof, a polynucleotide hybridizing under stringent conditions to such polynucleotide, and/or a fragment thereof; a polynucleotide comprising a nucleotide sequence as shown in any of SEQ ID NOS: 1 to 49 or a complementary sequence thereof, a polynucleotide hybridizing under stringent conditions to such polynucleotide, and/or a fragment thereof; and a polynucleotide comprising a nucleotide sequence as shown in any of SEQ ID NOS: 1 to 50 or a complementary sequence thereof, a polynucleotide hybridizing under stringent conditions to such polynucleotide, and/or a fragment thereof.

According to a more preferable embodiment, the kit of the present invention can comprise at least two to all polynucleotides comprising the nucleotide sequence as shown in any of SEQ ID NOS: 51 to 97 and 98 to 100 or a complementary sequence thereof.

A specific example of another combination is a polynucleotide comprising (or consisting of) the nucleotide sequence as shown in any of SEQ ID NOS: 1 to 19 and 48 or a complementary sequence thereof, and/or a fragment thereof.

A further specific example of another combination is a polynucleotide comprising (or consisting of) the nucleotide sequence as shown in any of SEQ ID NOS: 20 to 47, 49, and 50 or a complementary sequence thereof, and/or a fragment thereof.

In the present invention, the size of fragments of the polynucleotides is, for example, continuous 15 to all nucleotides, 15 to 5000 nucleotides, 15 to 4500 nucleotides, 15 to 4000 nucleotides, 15 to 3500 nucleotides, 15 to 3000 nucleotides, 15 to 2500 nucleotides, 15 to 2000 nucleotides, 15 to 1500 nucleotides, 15 to 1000 nucleotides, 15 to 900 nucleotides, 15 to 800 nucleotides, 15 to 700 nucleotides, 15 to 600 nucleotides, 15 to 500 nucleotides, 15 to 400 nucleotides, 15 to 300 nucleotides, 15 to 250 nucleotides, 15 to 200 nucleotides, 15 to 150 nucleotides, 15 to 140 nucleotides, 15 to 130 nucleotides, 15 to 120 nucleotides, 15 to 110 nucleotides, 15 to 100 nucleotides, 15 to 90 nucleotides, 15 to 80 nucleotides, 15 to 70 nucleotides, 15 to 60 nucleotides, 15 to 50 nucleotides, 15 to 40 nucleotides, 15 to 30 nucleotides or 15 to 25 nucleotides; 25 to all nucleotides, 25 to 1000 nucleotides, 25 to 900 nucleotides, 25 to 800 nucleotides, 25 to 700 nucleotides, 25 to 600 nucleotides, 25 to 500 nucleotides, 25 to 400 nucleotides, 25 to 300 nucleotides, 25 to 250 nucleotides, 25 to 200 nucleotides, 25 to 150 nucleotides, 25 to 140 nucleotides, 25 to 130 nucleotides, 25 to 120 nucleotides, 25 to 110 nucleotides, 25 to 100 nucleotides, 25 to 90 nucleotides, 25 to 80 nucleotides, 25 to 70 nucleotides, 25 to 60 nucleotides, 25 to 50 nucleotides or 25 to 40 nucleotides; 50 to all nucleotides, 50 to 1000 nucleotides, 50 to 900 nucleotides, 50 to 800 nucleotides, 50 to 700 nucleotides, 50 to 600 nucleotides, 50 to 500 nucleotides, 50 to 400 nucleotides, 50 to 300 nucleotides, 50 to 250 nucleotides, 50 to 200 nucleotides, 50 to 150 nucleotides, 50 to 140 nucleotides, 50 to 130 nucleotides, 50 to 120 nucleotides, 50 to 110 nucleotides, 50 to 100 nucleotides, 50 to 90 nucleotides, 50 to 80 nucleotides, 50 to 70 nucleotides or 50 to 60 nucleotides; 60 to all nucleotides, 60 to 1000 nucleotides, 60 to 900 nucleotides, 60 to 800 nucleotides, 60 to 700 nucleotides, 60 to 600 nucleotides, 60 to 500 nucleotides, 60 to 400 nucleotides, 60 to 300 nucleotides, 60 to 250 nucleotides, 60 to 200 nucleotides, 60 to 150 nucleotides, 60 to 140 nucleotides, 60 to 130 nucleotides, 60 to 120 nucleotides, 60 to 110 nucleotides, 60 to 100 nucleotides, 60 to 90 nucleotides, or 60 to 80 nucleotides or 60 to 70, in the nucleotide sequence of each polynucleotide.

It should be noted that the above combinations that constitute the kit of the present invention are exemplary, and any other combinations fall within the scope of the present invention.

The kit of the present invention can comprise, in addition to the polynucleotides of the present invention, mutants thereof, or fragments thereof as described above, known or novel polynucleotides that enable detection of kidney cancer or prediction of metastasis or prognosis of kidney cancer.

4.2 Antibody kit

The present invention also provides a kit for detecting, identifying, or predicting *in vitro* the presence, metastasis, or prognosis of kidney cancer, comprising one or more of the antibodies of group II described in Section 2 above, fragments thereof, chemically modified derivatives thereof, the antibodies described in Section 3.2 above, fragments thereof, or chemically modified derivatives thereof.

The kit of the present invention can comprise, as probes, antibodies from groups II (f), (g), and (h), fragments thereof, or chemically modified derivatives thereof. These probes can be packaged in suitable containers, alone or in combination.

Examples of probe combinations are as follows.

In order to detect polypeptides encoded by the PABPN1, PINK1, TFF2, EIF3S9, MAX, MLL4, CACNB2, ZMYND11, BAT2, NRBP, MCM3AP, COL4A1, MKNK1, BBC3, FLJ10359, DPT, C10orf76, DIA1, PBK, PRKD2, KRT19, FLJ23436, NPHS2, C3orf14, AGTR2, HTR1F, KIF3B, DCN, STK22C, DKFZp566C0424, CNR1, HOMER3, GPR2, FLJ12442, XLKD1, CASKIN2, COL5A2, BRD3, ATP6VOA4, PRODH2, EPHB2, LYAR, COX6B, PRH1, LAPTM5, RPS6KA4, GCC2, FGF2, MMP14, and ERBB2 genes as markers for predicting the prognosis of kidney cancer or as markers for metastasis of kidney cancer, homologs thereof, mutants thereof, or derivatives thereof, the first example comprises one or more, and preferably two or more antibodies against such polypeptides, mutants thereof, or fragments thereof.

Specifically, the probes comprised in the kit are: one or more antibodies that bind specifically to at least one of polypeptides each comprising the amino acid sequence as shown in any of SEQ ID NOS: 101 to 110, 112 to 120, and 122 to 147, mutants thereof, or fragments thereof; fragments thereof; or chemically modified derivatives.

The kit can further comprise an antibody against a polypeptide having the amino acid sequence as shown in any of SEQ ID NOS: 111, 121, and 148 to 150, a fragment thereof, or a chemically modified derivative thereof. Use of such antibodies in combination can improve the accuracy for predicting metastasis or prognosis after surgery.

The second example includes one or more antibodies against the polypeptides encoded comprising the amino acid sequences as shown in SEQ ID NOS: 151, 153, and 155 to 160 and in SEQ ID NOS: 161 to 190 of group II (g) and (h) as kidney cancer markers, fragments thereof, or chemically modified derivatives thereof.

The kit can further comprise an antibody against a polypeptide having the amino acid sequence as shown in any of SEQ ID NOS: 152, 154, and 191, and 192 to 197, a fragment thereof, or a chemically modified derivative thereof. Use of such antibodies in combination can improve the accuracy for detecting kidney cancer.

The antibodies comprised in the kit of the present invention can be present singly or in the form of a mixture. Alternatively, the antibodies may be bound onto a solid-phase carrier or may be in the free form. Further, the kit of the present invention can comprise a labeled secondary antibody, a carrier, a washing buffer, a sample diluent, a substrate for enzyme, a reaction terminator, a marker (target) polypeptide(s) as purified standard(s), instructions, and so on.

5. DNA chip

The present invention further provides a DNA chip for detecting kidney cancer or predicting the prognosis or metastasis of kidney cancer using the same polynucleotide(s) as the polynucleotide(s) comprised in the composition and/or the kit of the present invention as described in Sections 3 and 4 above, a mutant(s) thereof, or a fragment(s) thereof, alone or in combination, preferably in combination.

A substrate of the DNA chip is not particularly limited, provided that the substrate can comprise DNAs immobilized thereon. Examples of the substrate include a glass slide, a silicon chip, a polymer chip, and a nylon membrane. Such substrates may be subjected to surface treatment, for example, poly-L-lysine coating or introduction of a functional group such as an amino or carboxyl group.

DNA can be immobilized on a substrate by any common techniques without particular limitation. Examples of such techniques include a method wherein DNA is spotted using a high-density dispenser, called spotter or arrayer, a method of spraying DNA on a substrate using an apparatus (i.e., inkjet), which jets fine droplets from a nozzle by a piezoelectric element, and a method of synthesizing nucleotides successively on a substrate. When the high-density dispenser is used, for example, different gene solutions are first placed into each well of a multi-well plate, and the solutions are taken out of the plate using a pin (i.e., needle) and are successively spotted on the substrate. According to the inkjet technique, genes are jetted through a nozzle, and the genes are arrayed on the substrate at a high speed. In the DNA synthesis on the substrate, a nucleotide on the substrate is protected with a functional group, which is capable of leaving from the substrate by light, and light is selectively applied only to a nucleotide at a specific position by using a mask, thereby deprotecting the functional group. Thereafter, nucleotides are added to the reaction mixture, which nucleotides are coupled to the nucleotides on the substrate, and this step is repeated.

Polynucleotides to be immobilized are the polynucleotides of the present invention as described above.

Examples of such polynucleotides can comprise one or more of the following polynucleotides or fragments thereof:

(1) polynucleotides each consisting of a nucleotide sequence as shown in any of SEQ ID NOS: 1 to 50, mutants thereof, or fragments comprising at least 15 continuous nucleotides thereof;

(2) polynucleotides each comprising a nucleotide sequence as shown in any of SEQ ID NOS: 1 to 50;

(3) polynucleotides each consisting of a nucleotide sequence as shown in any of SEQ ID NOS: 1 to 10, 12 to 20, and 22 to 47, mutants thereof, or fragments comprising at least 15 continuous nucleotides thereof;

(4) polynucleotides each comprising a nucleotide sequence as shown in any of SEQ ID NOS: 1 to 10, 12 to 20, and 22 to 47, mutants thereof, or fragments comprising at least 15 continuous nucleotides thereof;

(5) polynucleotides each consisting of a nucleotide sequence complementary to a nucleotide sequence as shown in any of SEQ ID NOS: 1 to 10, 12 to 20, and 22 to 47, mutants thereof, or fragments comprising at least 15 continuous nucleotides thereof;

(6) polynucleotides each comprising a nucleotide sequence complementary to a nucleotide sequence as shown in any of SEQ ID NOS: 1 to 10, 12 to 20, and 22 to 47;

(7) polynucleotide each hybridizing under stringent conditions to DNA consisting of a nucleotide sequence complementary to a nucleotide sequence as shown in any of SEQ ID NOS: 1 to 10, 12 to 20, and 22 to 47 or fragments comprising at least 15 continuous nucleotides thereof;

(8) polynucleotides each hybridizing under stringent conditions to DNA consisting of a nucleotide sequence as shown in any of SEQ ID NOS: 1 to 10, 12 to 20, and 22 to 47 or fragments comprising at least 15 continuous nucleotides thereof;

(9) a polynucleotide consisting of the nucleotide sequence as shown in any of SEQ ID NOS: 11, 21, and 48 to 50, mutants thereof, or fragments comprising at least 15 continuous nucleotides thereof;

(10) polynucleotides comprising a nucleotide sequence as shown in any of SEQ ID NOS: 11, 21, and 48 to 50, mutants thereof, or fragments comprising at least 15 continuous nucleotides thereof;

(11) polynucleotides consisting of a nucleotide sequence complementary to a nucleotide sequence as shown in SEQ ID NOS: 11, 21, and 48 to 50, mutants thereof, or fragments comprising at least 15 continuous nucleotides thereof;

(12) polynucleotides comprising a nucleotide sequence complementary to the nucleotide sequence as shown in any of SEQ ID NOS: 11, 21, and 48 to 50;

(13) polynucleotides each hybridizing under stringent conditions to DNA consisting of a nucleotide sequence complementary to a nucleotide sequence as shown in any of SEQ ID NOS: 11, 21, and 48 to 50 or fragments comprising at least 15 continuous nucleotides thereof;

(14) polynucleotides each hybridizing under stringent conditions to DNA consisting of a nucleotide sequence as shown in any of SEQ ID NOS: 11, 21, and 48 to 50 or fragments comprising at least 15 continuous nucleotides thereof;

(15) a polynucleotide comprising at least 15 continuous nucleotides in a nucleotide sequence as shown in any of SEQ ID NOS: 1 to 10, 12 to 20, and 22 to 47 or a complementary sequence thereof;

(16) a polynucleotide comprising at least 60 continuous nucleotides in a nucleotide sequence as shown in any of SEQ ID NOS: 1 to 10, 12 to 20, and 22 to 47 or a complementary sequence thereof;

(17) a polynucleotide comprising at least 15 continuous nucleotides in a nucleotide sequence as shown in any of SEQ ID NOS: 11, 21, and 48 to 50 or a complementary sequence thereof;

(18) a polynucleotide comprising at least 60 continuous nucleotides in a nucleotide sequence as shown in any of SEQ ID NOS: 11, 21, and 48 to 50 or a complementary sequence thereof;

(19) a polynucleotide comprising a nucleotide sequence as shown in any of SEQ ID NOS: 51 to 60, 62 to 70, and 72 to 97 and at least 60 continuous nucleotides in a nucleotide sequence as shown in any of SEQ ID NOS: 1 to 10, 12 to 20, and 22 to 47;

(20) a polynucleotide comprising a nucleotide sequence comprising a sequence complementary to a nucleotide sequence as shown in any of SEQ ID NOS: 51 to 60, 62 to 70, and 72 to 97 and at least 60 continuous nucleotides in a nucleotide sequence complementary to a nucleotide sequence as shown in any of SEQ ID NOS: 1 to 10, 12 to 20, and 22 to 47;

(21) a polynucleotide comprising a nucleotide sequence as shown in any of SEQ ID NOS: 61, 71, and 98 to 100 and at least 60 continuous nucleotides in a nucleotide sequence as shown in any of SEQ ID NOS: 11, 21, and 48 to 50; and

(22) a polynucleotide comprising a sequence complementary to a nucleotide sequence as shown in any of SEQ ID NOS: 61, 71, and 98 to 100 and at least 60 continuous nucleotides in a nucleotide sequence as shown in any of SEQ ID NOS: 11, 21, and 48 to 50.

According to a preferable embodiment, the DNA chip of the present invention can include at least 2 or all polynucleotides each comprising a nucleotide sequence as shown in any of SEQ ID NOS: 51 to 60, 61, 62 to 70, 71, 72 to 97, and 98 to 100 or a complementary sequence thereof.

According to the present invention, the polynucleotides to be immobilized may be any of genomic DNA, cDNA, RNA, synthetic DNA, and synthetic RNA, or alternatively they may be single-stranded or double-stranded.

Examples of DNA chips that can detect and determine the expression levels of the target gene, RNA, or cDNA include the Gene Chip Human Genome U133 Plus 2.0 Array (Affymetrix), the Whole human genome oligo microarray (Agilent), the IntelliGene® HS Human Expression CHIP (Takara Bio), and a polymethylmethacrylate DNA chip substrate having a concave-convex structure (JP Patent Publication (kokai) No. 2004-264289 A).

DNA microarrays can be prepared by, for example, a method wherein probes that have been prepared in advance are immobilized on a solid-phase surface. In this method, polynucleotides into which functional groups have been introduced are synthesized, and oligonucleotides or polynucleotides are spot-deposited on the surface of a surface-treated solid-phase support, followed by covalent binding to the surface (e.g., J. B. Lamture et al., Nucleic. Acids. Research, 1994, vol. 22, pp. 2121-2125; Z. Guo et al., Nucleic. Acids. Research, 1994, vol. 22, pp. 5456-5465). In general, the polynucleotides are covalently bound to the surface-treated solid-phase support via a spacer or crosslinker. The method wherein fine pieces of polyacrylamide gel are aligned on the glass surface and synthetic polynucleotides are covalently bound thereto is also known (G. Yershov et al., Proceedings of the National Academic Sciences, U.S.A., 1996, vol. 94, p. 4913). As a further method, a microelectrode array is prepared on silica microarray, a reaction site is formed on the electrode by providing a permeable layer of streptavidin-containing agarose, this site is positively charged to immobilize the biotinylated polynucleotides thereon, and the charge at the site is regulated. This enables performance of hybridization at a high speed under stringent condition (R. G Sosnowski et al., Proceedings of the National Academic Sciences, U.S.A., 1997, vol. 94, pp. 1119-1123).

6. Method for detecting and identifying the presence of kidney cancer and/or for predicting the prognosis or metastasis of kidney cancer

The present invention provides a method for predicting *in vitro* the subject's prognosis and/or the presence or absence of metastasis of kidney cancer comprising comparing the expression level of the target nucleic acids in a biological sample of kidney cancer cells from a subject with the expression level of the target nucleic acids in the kidney cancer cells from patients with poor prognoses and/or the expression level of the target nucleic acids in the kidney cancer cells from patients with good prognoses, by using the composition, kit, or DNA chip of the present invention, or combinations thereof, wherein the target nucleic acids can be detected by the polynucleotides in the composition, kit, or DNA chip, mutants thereof, or fragments thereof, and, when the expression level of the target nucleic acids in the subject is changed compared with that in the patients with good prognoses or poor prognoses and/or that in the patients with poor prognoses, the subject is determined to have poor or good prognosis, and/or the metastasis of kidney cancer is determined to have or have not occurred.

The present invention also provides use of the composition, kit, or DNA chip of the present invention for detecting *in vitro* the kidney cancer cells suspected of being at risk for metastasis in an analyte sample from a subject.

The above-described method of the present invention involves the use of the composition, kit, or DNA chip of the present invention comprising the polynucleotides of the present invention, mutants thereof, or fragments thereof alone or in any possible combination.

In the method for predicting, detecting, identifying, or (genetically) diagnosing the metastasis of kidney cancer and/or for predicting the prognosis for kidney cancer patients of the present invention, the polynucleotides comprised in the composition, kit, or DNA chip of the present invention, mutants thereof, or fragments thereof can be used as primers or detection probes (searchers). When used as primers, for example, primers comprising generally 15 to 50' nucleotides, preferably 15 to 30 nucleotides, and more preferably 18 to 25 nucleotides can be used. When used as detection probes, for example, polynucleotides comprising 15 to all nucleotides, preferably 25 to 1000 nucleotides, more preferably 25 to 100 nucleotides can be used. It should be understoodthat the number of nucleotides should not be limited to the specific ranges.

The polynucleotides, mutants thereof, or fragments thereof that are comprised in the composition or kit of the present invention can be used as primers or probes in accordance with the conventional techniques in known methods for specifically detecting a given gene, such as Northern blotting, RT-PCR, *in situ* hybridization, or Southern hybridization. As to samples to be tested (or analytes), the whole or part of the kidney tissue or the body tissue suspected of being at risk for having kidney cancer cells of a subject may be removed by biopsy or another means, or the samples may be removed from the body tissue excised by surgery, depending on types of detection methods. Further, total RNA prepared therefrom in accordance with the conventional techniques may be used, or various polynucleotides including cDNA or poly A(+) RNA prepared from the RNA may be used.

Alternatively, the expression levels of nucleic acids such as the gene, RNA, or cDNA of the present invention in the body tissue can be detected or quantified using a DNA chip (including a DNA microarray). In this case, the composition or kit of the present invention can be used as a DNA array probe (e.g., the Human Genome U133 Plus 2.0 Array (Affymetrix) uses a polynucleotide probe having 25 nucleotides). Such a DNA array may be hybridized to the labeled DNA or RNA, which is prepared from RNA removed from the body tissue, and a complex of the probe with the labeled DNA or RNA resulting from such hybridization may be detected using the labeled DNA or RNA as an indication to evaluate the presence or absence of the expression of the genes associated with kidney cancer metastasis or genes associated with prognosis for the cancer patient or the expression levels thereof in the body tissue. In the method of the present invention, a DNA chip is preferably used. This enables the simultaneous evaluation of the presence or absence of the expression of a plurality of genes, or the simultaneous evaluation of the expression levels of the genes, in a single biological sample.

The composition, kit, or DNA chip of the present invention is useful for predicting the prognosis for a kidney cancer patient and/or predicting, identifying, or detecting metastasis of kidney cancer (e.g., diagnosis of affection or degree of affection). Specifically, prognosis for a kidney cancer patient and/or metastasis of kidney cancer can be predicted using the composition, kit, or DNA chip in the following manner. That is, the body tissue of the subject having kidney cancer cells can be subjected to the assay of the expression levels of the genes that are detected with the use of such diagnostic composition. In this case, the term "differences in gene expression levels" refers to not only the presence or absence of the expression but also the case where differences in gene expression levels between the body tissue comprising kidney cancer cells from a patient with a good prognosis and the body tissue comprising kidney cancer cells from a patient with a poor prognosis are statistically significant (p value of < 0.05). For example, the expression of the PABPN1 gene is induced/decreased in kidney cancer cells from a patient with poor prognosis. Thus, its expression is increased/decreased in kidney cancer tissue from a subject with poor prognosis. If the differences between such expression level and the expression level in the normal tissue are significant, the subject is suspected of being at risk for kidney cancer metastasis and is also predicted to have poor prognosis.

A method for detecting kidney cancer (cells) using the composition, kit, or DNA chip of the present invention comprises: removing the whole or part of the body tissue from a subject via biopsy or recovering it from the body tissue excised by surgery; detecting the genes contained therein using a polynucleotide or polynucleotides selected from the polynucleotides of the present invention, mutants thereof, or fragments thereof; measuring the expression levels of said genes; and predicting metastasis of kidney cancer, diagnosing the presence or absence of metastasis of kidney cancer or a degree thereof, and/or predicting the prognosis for a kidney cancer patient. Also, the method for predicting metastasis of kidney cancer according to the present invention can detect, identify, or predict amelioration or the degree of amelioration of the disease when a therapeutic agent is administered to an kidney cancer bearing patient, for example.

The method of the present invention can comprise, for example, the following steps (a), (b), and (c) of:

(a) bringing a biological sample of a subject into contact with a polynucleotide or polynucleotides comprised in the composition, kit, or DNA chip of the present invention;
(b) measuring the expression level of the target nucleic acid(s) in the biological sample using the polynucleotide or polynucleotides as the probe; and
(c) predicting the prognosis for kidney cancer patients and/or identifying the presence or absence of kidney cancer (cells) suspected of being at risk for metastasis in the biological sample on the basis of the results obtained in step (b).

Examples of biological samples used in the method of the present invention include the body tissues of a subject, for example, samples prepared from kidney tissue and peripheral tissue thereof, tissue suspected of being at risk for having the metastasis of kidney cancer, and the like. Specifically, an RNA containing sample prepared from such tissue or a sample containing a polynucleotide prepared therefrom may be prepared by removing the whole or part of the body tissue from the subject via biopsy, or recovering the sample from the body tissue excised by surgery to prepare the sample therefrom in accordance with conventional techniques.

The term "subject" as used herein refers to a mammalian animal. Examples thereof include, but are not limited to, human, monkey, mouse, and rat, preferably human.

In the method of the present invention, the above-mentioned steps may be varied depending on types of biological samples used as analytes.

When RNA is used as the analyte, for example, detection of kidney cancer (cells) can comprise the following steps (a), (b), and (c) of:

(a) allowing RNA prepared from a biological sample of a subject or a complementary polynucleotide (cDNA) transcribed therefrom to bind to a polynucleotide comprised in the composition, kit, or DNA chip of the present invention;
(b) measuring the RNA prepared from the biological sample bound to the polynucleotide or a complementary polynucleotide transcribed from the RNA using the above polynucleotide as a probe; and
(c) identifying the presence or absence of kidney cancer (cells) from a patient with a poor prognosis based on the results obtained in step (b).

In order to detect, identify, or diagnose the metastatic kidney cancer (cells) by the method of the present invention, for example, various hybridization techniques can be employed. Examples of the hybridization techniques that can be employed include Northern blotting, quantitative Southern blotting, RT-PCR, DNA chip analysis, *in situ* hybridization, and Southern hybridization.

When Northern blotting is employed, the diagnostic composition of the present invention can be used as a probe to detect and assay the presence or absence of gene expression in RNA or the expression level thereof. Specifically, the diagnostic composition (specifically a complementary strand) of the present invention is labeled with a radioisotope (e.g., $^{32}$P, $^{33}$P, or $^{35}$S) or a fluorophore, the resultant is hybridized to the RNA obtained from a body tissue of a subject that has been transferred onto a nylon membrane or the like in accordance with any of the conventional techniques, the resulting double-strand of the diagnostic composition (i.e., DNA) and RNA can be measured by detecting a signal derived from a label (a radioisotope or fluorophore) of the diagnostic composition using a radio detector (e.g., BAS-1800 II, Fuji Photo Film, Japan) or a fluorescent detector (STORM 860, Amersham Bioscience).

When the quantitative RT-PCR is employed, the diagnostic composition of the present invention can be used as a primer to detect and assay the presence or absence of the gene expression in RNA or the expression level thereof. Specifically, cDNA is prepared from RNA of a body tissue of a subject in accordance with a conventional technique, a pair of primers prepared from the diagnostic composition of the present invention (i.e., a forward strand and a reverse strand, both bound to the cDNA) is hybridized to cDNA to perform PCR with the use of cDNA as a template in accordance with the conventional technique, thereby amplifying the target gene regions, and the resulting double-stranded DNA is detected. Double-stranded DNA can be detected by a method wherein PCR is carried out using a primer that has been labeled with a radioisotope or fluorophore in advance, a method wherein the PCR product is electrophoresed on agarose gel, and double-stranded DNA is detected by staining the same with ethidium bromide or the like, or a method wherein the resulting double-stranded DNA is transferred to a nylon membrane or the like in accordance with a conventional technique, and the resultant is subjected to hybridization to the labeled diagnostic composition as a probe to detect the substance of interest.

When the DNA array analysis is employed, a DNA chip comprising the diagnostic composition of the present invention as a DNA probe (single-stranded or double-stranded) bound to a substrate is used. A substrate comprising genes immobilized thereon is generally referred to as DNA chip or DNA array. Examples of the DNA array include a DNA macroarray and a DNA microarray. As used herein, the term "DNA chip" refers to such DNA arrays.

Hybridization conditions are not particularly limited. For example, hybridization is carried out in 3 to 4×SSC and 0.1% to 0.5% SDS at 30°C to 50°C for 1 to 24 hours, more preferably in 3.4×SSC and 0.3% SDS at 40°C to 45°C for 1 to 24 hours, followed by washing. Washing is continuously carried out, for example, with a solution containing 2×SSC and 0.1% SDS, with a solution of 1×SSC, and with a solution of 0.2xSSC at room temperature. The term "1×SSC" refers to an aqueous solution containing 150 mM sodium chloride and 15 mM sodium citrate (pH 7.2). Preferably, a complementary strand remains hybridized to the target (+) strand even if it is washed under such conditions. Specific examples of such complementary strand include a strand consisting of the nucleotide sequence completely complementary to the nucleotide sequence of the target (+) strand, and a strand consisting of a nucleotide sequence having at least 80% identity with said strand.

When PCR is carried out under stringent hybridization conditions using polynucleotide fragments obtained from the composition or kit of the present invention as primers, for example, a PCR buffer comprising 10 mM Tris-HCl (pH 8.3), 50 mM KCl, and 1 to 2 mM MgCl$_2$ is used, and the treatment is carried out at a temperature, Tm-(5 to 10°C) which is calculated from the primer sequence, for about 15 seconds to 1 minute. The Tm value can be calculated, for example, by the equation Tm = 2 × (the number of adenine residues + the number of thymine residues) + 4 × (the number of guanine residues + the number of cytosine residues).

Another example of the "stringent conditions" for hybridization is described in, for example, Sambrook, J. & Russel, D., Molecular Cloning, A LABORATORY MANUAL, Cold Spring Harbor Laboratory Press, January 15, 2001, vol. 1: 7.42 to 7.45, vol. 2: 8.9 to 8.17, and such conditions can be employed in the present invention.

The present invention also provides a method for predicting the prognosis for a kidney cancer patient by assaying the expression levels of target nucleic acids or genes in the biological sample from a subject using one or more probes of group I and/or group II, the composition, kit, or DNA chip of the present invention, or combinations thereof and using a discriminant, i.e., the support vector machine (SVM), using the gene expression levels in the kidney cancer tissue from a patient with good prognosis and the kidney cancer tissue from a patient with poor prognosis as the training samples, and/or a method for determining whether or not the biological sample contains metastatic kidney cancer cells.

The present invention further provides a method for detecting, identifying, or predicting the metastasis of kidney cancer, the method comprising the steps of:

(1) measuring *in vitro* expression levels of target nucleic acids in a plurality of biological samples that are known to be the tissue which contains kidney cancer cells obtained from a patient with a poor prognosis and/or the tissue which contains kidney cancer cells obtained from a patient with a good prognosis using a probe or probes selected from group I as defined above, the composition, the kit, or the DNA chip comprising the probe or probes;

(2) preparing a discriminant, a support vector machine, using, as training samples, the expression levels of the target nucleic acids determined in step (1);

(3) measuring *in vitro* expression levels of the target nucleic acids in a biological sample obtained from the kidney cancer cells of the subject in the same manner as in step (1); and

(4) assigning the expression levels of the target nucleic acids identified in step (3) to the discriminant prepared in step (2) and, based on the results obtained with the use of the discriminant, predicting the prognosis for a subject and/or determining whether or not kidney cancer of the subject is metastatic or nonmetastatic, provided that the target nucleic acids can be detected by the polynucleotides, mutants thereof, or fragments thereof contained in the composition, kit, or DNA chip.

Alternatively, the method of the present invention can comprise the following steps (a), (b), and (c) of:

(a) measuring the expression levels of the target genes in the biological samples that are known to be the tissue containing the kidney cancer cells from a patient with good prognosis or the tissue containing the kidney cancer cells from a patient with poor prognosis using the composition for diagnosis (detection), kit, or DNA chip of the present invention;
(b) preparing a discriminant (i.e., support vector machine) by assigning the expression levels determined in step (a) into the equations 1 to 5 below; and
(c) assaying the expression levels of the target genes in the biological sample from a subject using the composition for diagnosis (detection), kit, or DNA chip of the present invention, and assigning the determined values into the discriminant prepared in step (b), in order to predict the prognosis for a kidney cancer patient and/or determine whether or not the biological sample contains metastatic kidney cancer cells based on the obtained results.

SVM is a learning machine that was proposed in the framework of a statistical learning theory made to solve a two-class classification problem, by V. Vapnik of AT&T in 1995 (The Nature of Statistical Leaning Theory, Springer, 1995). SVM is a linear classifier but it can solve nonlinear problems in combination with the Kernel method as described below. Among many hyperplanes that classify training samples of different classes, the hyperplane that maximizes the minimum distance from the hyperplane to the training sample may be defined as the classification plane to classify a new test sample in the most accurate manner.

SVM can only solve linear problems. As a method for solving substantially nonlinear problems, a method wherein a feature vector is nonlinearly transformed into a higher-dimensional feature, and linear classification is then performed, is known. This becomes equivalent to the use of a nonlinear model in an original space. High-dimensional mapping, however, requires an enormous computational effort and reduces a generalization capability. According to SVM, the classification function depends exclusively on the inner product of the inputted pattern. Accordingly, if the inner product could be calculated, the optimal classification function could be constructed. The formula that represents the inner product of two elements in a nonlinearly mapped space only by the input in original spaces is referred to as the Kernel formula. An optimal classification function, i.e. a discriminant, can be formed only by the Kernel formula without computation of features in the actually mapped space while performing high-dimensional mapping (e.g., Hideki Asou et al., Toukei kagaku no furontia 6 (Frontier of statistical science 6), "Pataan ninshiki to gakushu no toukeigaku (Statistics of pattern recognition and learning)," pp. 107-138, Iwanami Shoten Publishers, Tokyo, Japan, date of publication, April 11, 2003).

Examples of the computation of a discriminant that can be used in the method of the present invention are shown below.

In order to identify SVM, the expression levels of the target gene in biological samples that are known to be a kidney cancer cell-containing tissue from a patient with good prognosis or kidney cancer tissue from a patient with poor prognosis is provided as training samples, and a constant of the classification function can be identified in the following manner.

The training sample $x_i$ is assumed to belong to either a group of kidney cancer cell-containing tissue from a patient with a good prognosis or kidney cancer tissue from a patient with a poor prognosis, which groups are classified into (+) or (-). When training samples can be linearly separated by the hyperplane, the classification function is, for example, as follows:

[equation 1]

$$f(x) = \sum_{i=1}^{n} w_i \cdot x_i + b$$

where w represents a weighting factor, b represents a bias constant, and x represents a sample variable.

This function, however, has a restriction:

$$[\text{equation 2}]$$

$$y_i\left(w^T x_i + b\right) \geq 1 - \xi_i$$

$$\xi_i \geq 0, i = 1, \cdots, n$$

where T represents an inner product, y represents a sample class, and ζ represents a slack variable. Thus, the Lagrange's method of unidentified multipliers may be used to regress to the following optimization problem using the Lagurange multiplier α.

$$[\text{equation 3}]$$

$$\max_\alpha \sum_{i=1}^{n} \alpha_i - \frac{1}{2} \sum_{i,j=1}^{n} \alpha_i \alpha_j y_i y_j x_i^T x_j$$

$$[\text{equation 4}]$$

$$0 \leq \alpha_i \leq C, \sum_{i=1}^{n} \alpha_i y_i = 0$$

where C represents a restriction parameter identified by an experiment.

If the above problem is dissolved, the following formula is consequently obtained.

$$[\text{equation 5}]$$

$$w = \sum_{i=1}^{n} \alpha_i y_i x_i$$

$$b = -\frac{1}{2}\left(w^T x_A + w^T x_B\right)$$

Thus, two groups of training samples are necessary in order to prepare a SVM discriminant for classifying unknown samples. According to the present invention, such training samples are, for example, a set of samples obtained from patients of "the expressed genes $(x_1,x_2,..x_i,..,x_n)$ obtained from the kidney cancer tissue of patients with good prognoses or poor prognoses" and a set of samples obtained from the patients of "the expressed genes $(x_1,x_2,..x_i,...x_n)$ obtained from the kidney cancer tissue of patients with poor prognoses." The number (n) of the expressed genes concerning such sets varies depending on the design of the experiment. The expression levels of each gene yield significant difference, relatively small difference, or no difference between the two groups regardless of the type of experiment. In order to improve the accuracy of the SVM discriminant, distinctive differences are required between 2 groups of training samples. Thus, it is necessary to selectively extract and use genes that exhibit different expression levels between 2 groups from among gene sample sets.

Examples of methods for extracting genes that exhibit different expression levels between 2 groups include a t-test which is a parametric analysis for detecting different means and an U-test of Mann-Whitney which is a non-parametric analysis. As a method that utilizes survival analysis, the plots of the survival curve obtained by the Kaplan-Meier method are analyzed by the log-rank test or the Wilcoxon test. Also, a method wherein a survival curve is used as a regression model and analyzed by the Cox proportional hazards model is particularly useful to deduce whether or not a given variable is associated with survival. Specifically, the Cox proportional hazards model indicates how sufficiently a variety of variables describe the regression model concerning a plurality of survival curves plotted by the Kaplan-Meier method of the group of patients classified in accordance with a given category.

In the method of the present invention, for example, any combination of one or more of the aforementioned polynucleotides as shown in any of SEQ ID NOS: 1 to 10, 12 to 20, and 22 to 47 and one or more of polynucleotides as shown in any of SEQ ID NOS: 11, 21, and 48 to 50 may be used. Also, the fact that the expression levels of the 50 types of target genes in tissue containing kidney cancer cells from a patient with a good prognosis are significantly different from those in tissue containing kidney cancer cells from a patient with poor prognosis, and that such expression levels vary in tissue containing kidney cancer cells from a patient with poor prognosis, are used as indications to determine the expression levels of the 50 types of genes. Thus, the prediction of the prognosis for a kidney cancer patient and/or the distinction of the metastasis of kidney cancer can be performed at the probability of 85% or higher, 89% or higher, 90% or higher, preferably 92% or higher, more preferably 93% or higher, and further preferably 94% or higher (Fig. 2).

The present invention further provides a method for predicting the prognosis of kidney cancer or for detecting, identifying, or predicting the metastasis of kidney cancer, comprising measuring *in vitro* the expression levels of the polypeptides or the blood levels (or existing amounts) of the polypeptides in tissue containing kidney cancer cells from a patient with a good prognosis and in tissue containing kidney cancer cells from a patient with a poor prognosis, by using one or more antibodies against respective polypeptides encoded by the aforementioned 50 types of genes (e.g., those as shown in SEQ ID NOS: 1 to 50) or fragments thereof (e.g., as shown in SEQ ID NOS: 51 to 100), such as polypeptides consisting of the respective amino acid sequences as shown in SEQ ID NOS: 101 to 150, or fragments thereof.

The present invention also provides a method for detecting, identifying, or predicting kidney cancer, comprising measuring *in vitro* the expression levels of the polypeptides between kidney cancer tissue and non-cancerous tissue or the blood levels of the polypeptides (or the existing amounts), by using one or more, for example, 2 or more, 3 ore more, or 5 or more to all antibodies against the respective polypeptides encoded by the aforementioned 47 genes (e.g., those as shown in SEQ ID NOS: 151 to 160, and 191 and those as shown in SEQ ID NOS: 161 to 190, and 192 to 197) or fragments thereof.

Specifically, the above mentioned measurement can be carried out by an immunological method.

Examples of immunological assay techniques include enzyme immunoassay (ELISA or EIA), fluorescence immunoassay, radio immunoassay (RIA), luminescent immunoassay, immunonephelometry, latex agglutination assay, latex turbidimetry, hemagglutination, particle agglutination, and Western blotting.

When the above method is carried out by an immunoassay technique using a label, the antibody of the present invention may be immobilized, or a component in the sample may be immobilized to subject such substance to an immunological reaction.

Examples of solid-phase supports that can be used include insoluble supports in the form of beads, microplate, test tube, stick, or specimen (test strip) comprising a polystyrene, polycarbonate, polyvinyltoluene, polypropyrene, polyethylene, polyvinyl chloride, nylon, polymethacrylate, latex, gelatin, agarose, cellulose, sepharose, glass, metal, ceramic, or magnetic material.

The samples can be immobilized on the support in accordance with a conventional technique by binding the antibody of the present invention or a sample component to the solid-phase support by physical adsorption, chemical binding, or a combination thereof.

The present invention is intended to easily detect the reaction between the antibody of the present invention and the target polypeptide in the sample. To this end, the antibody of the present invention is labeled to directly detect the reaction of interest. Alternatively, a labeled secondary antibody is used to indirectly detect the reaction. In the method of detection according to the present invention, the latter indirect detection technique (e.g., the sandwich technique) is preferably employed from the viewpoint of sensitivity.

Examples of label substances that can be used for enzyme immunoassay include enzymes such as peroxidase (POD), alkaline phosphatase, β-galactosidase, urease, catalase, glucose oxidase, lactate dehydrogenase, amylase, and a biotin-avidin complex. Examples of label substances that can be used for fluorescence immunoassay include fluorescent substances such as fluorescein isothiocyanate, tetramethylrhodamine isothiocyanate, substituted rhodamine isothiocyanate, dichlorotriazine isothiocyanate, Alexa, or AlexaFluoro and fluorophores. Examples of label substances that can be used for radio immunoassay include radioactive isotopes, such as tritium, iodine ($^{131}$I, $^{125}$I, $^{123}$I, and $^{121}$I), phosphorus ($^{32}$P and $^{33}$P), sulfur ($^{35}$S), and metals (e.g., $^{68}$Ga, $^{67}$Ga, $^{68}$Ge, $^{54}$Mn, $^{99}$Mo, $^{99}$Tc, and $^{133}$Xe). Examples of label substances that can be used for luminescent immunoassay include luminescent molecules such as an NADH-, FMNH2-, luciferase system, luminol-hydrogen peroxide-POD system, acridinium ester system, or dioxetane compound system.

Also, an avidin-biotin system or streptavidin-biotin system may be used optionally. In such a case, the antibody of the invention or a fragment thereof may be bound, for example, to biotin.

A label can be bound to the antibody in case of enzyme immunoassay, for example, via a conventional technique, such as the glutaraldehyde method, the maleimide method, the pyridyl sulfide method, or the periodic acid method. Radio immunoassay can be carried out in accordance with a conventional technique, such as the chloramine-T method or Bolton-Hunter method. Such assay techniques can be carried out in accordance with conventional techniques (Current protocols in Protein Sciences, 1995, John Wiley & Sons Inc., Current protocols in Immunology, 2001, John Wiley & Sons Inc.). When the antibody of the present invention is directly labeled, for example, a component in the sample is immobilized and brought into contact with the labeled antibody of the present invention to form a complex of the marker polypeptide and the antibody of the present invention. The unbound labeled antibody is separated by washing, and the amount of the target polypeptide in the sample can be determined based on the amount of the bound labeled antibody or the unbound labeled antibody.

When the labeled secondary antibody is used, for example, the antibody of the present invention is allowed to react with the sample (the primary reaction), then with the labeled secondary antibody (the secondary reaction). The primary reaction and the secondary reaction may be carried out in the reverse order, concurrently, or separately. The primary and secondary reactions result in the formation of a complex of immobilized target polypeptide/the antibody of the invention/ labeled secondary antibody or a complex of the immobilized antibody of the invention/ target polypeptide/ labeled secondary antibody. The unbound labeled secondary antibody is separated by washing, and the amount of target polypeptide in the sample can be determined based on the amount of the bound labeled secondary antibody or of the unbound labeled secondary antibody.

In the enzyme immunoassay, specifically, the enzyme label is allowed to react with a substrate under optimal conditions, and the amount of the reaction product is assayed by an optical method or the like. In the fluorescence immunoassay, the fluorescent intensity from a fluorescent label is assayed. In the radio immunoassay, the radioactivity from radioactive label is assayed. In the luminescent immunoassay, the luminescent level from a luminescent reaction system is assayed.

In the method of the present invention, the generation of immune-complex aggregates in immunonephelometry, latex agglutination assay, latex turbidimetry, hemagglutination, particle agglutination, or the like is assayed by optically measuring the transmitted beam or scattered beam. When visually assayed, a solvent, such as a phosphate, glycine, Tris, or Good's buffer, can be used. Further, a reaction accelerator such as polyethylene glycol or an inhibitor of nonspecific reaction may be added to the reaction system.

The above-mentioned antibody or a fragment thereof includes, for example, a polyclonal antibody, a monoclonal antibody, a synthetic antibody, a recombinant antibody, a polyspecific antibody (including a bispecific antibody), a single chain antibody, an Fab fragment, and an $F(ab')_2$ fragment. The polyclonal antibody can be prepared as a specific antibody by a so-called absorption method, which comprises binding the antibody to an affinity column to which a purified polypeptide has been bound.

The measurement can comprise the steps of: bringing an antibody labeled with a common enzyme or fluorophore or a fragment thereof into contact with a tissue section or homogenized tissue or body fluid (such as blood, blood serum, blood plasma, or urine); and qualitatively or quantitatively measuring an antigen-antibody complex. Detection is carried out by, for example, a method wherein the presence and level of a target polypeptide are measured by immunoelectron microscopy, or a method wherein the level of a target polypeptide is assayed by a conventional method, such as ELISA or a fluorescent antibody method. Thus, kidney cancer can be detected. Further, when the expression level of a target polypeptide is decreased in tissue containing kidney cancer cells from a patient with good prognosis or in tissue containing kidney cancer cells from a patient with poor prognosis, or when the blood level of such polypeptides is significantly varied in the subject afflicted with kidney cancer with poor prognosis from the subject afflicted with kidney cancer with good prognosis, the subject can be identified to have poor prognosis and/or the metastasis of kidney cancer. In other words, when the expression level or amount of the existing target polypeptide is significantly varied from normal values, the subject is identified to have kidney cancer or have kidney cancer with poor prognosis and/or metastasis. The term "significantly" as used herein means that the identified values are statistically significant.

EXAMPLES

The present invention will be described in more detail with reference to the examples set forth below; however, it is contemplated that the technical scope of the present invention is not limited to the examples.

<Example 1>

(1) Clinical and pathological findings concerning subjects

Informed consent was obtained from 31 Japanese patients with kidney cancer, and the kidney tissues were excised from them at the time of the surgical excision of kidney cancer. The excised tissue was visually and/or histopathologically inspected to identify the kidney cancer tissue, and the kidney cancer tissue was immediately frozen and stored in liquid nitrogen.

(2) Extraction of total RNA and preparation of cDNA

The tissue in the kidney cancer lesion of the kidney tissue obtained from an kidney cancer patient was used as a sample. Total RNA was prepared from the tissue using a Trizol reagent (Invitrogen) in accordance with the manufacturer's recommended protocol.

The thus obtained total RNA (1 $\mu$g) was subjected to reverse transcription using oligo (dT) primers in combination with random nonamers and using the CyScribe First-Strand cDNA Labeling Kit (GE Healthcare, Japan) in accordance with the manufacturer's recommended protocols. Cy3-dUTP (GE Healthcare) was added to total RNA obtained from the kidney cancer tissue, Cy5-dUTP (GE Healthcare) was added to reference total RNA (Stratagene), and cDNA was labeled at the time of reverse transcription in accordance with the manufacturer's recommended protocols. The labeled cDNA was purified using the QIA quick PCR purification Kit (QIAGEN) and then subjected to hybridization.

(3) Preparation of oligo DNA microarray

As the oligo DNA microarrays, the GeneChip® (Human Genome U133 A, Affymetrix) and the DNA chip prepared by the method as described herein were used.

A method for preparing a DNA chip is described below. In order to determine the type of oligo DNA to be loaded at first, genes were determined using the GeneChip® (Affymetrix). The GeneChip® was operated in accordance with the protocol of the Complete GeneChip® Instrument System. As a result of the analysis using the Complete GeneChip®, 8,961 types of genes in total, i.e., the genes whose expression patterns may vary due to kidney cancer and the control genes, were extracted.

Sequences comprising 60-70 residues at sites having high sequence specificity of the extracted 8,961 types of genes were selected and synthesized while avoiding sequence overlapping. The 8,961 types of 60 or 70-mer synthetic oligo DNAs comprising oligo DNAs as shown in SEQ ID NOS: 21 to 40 were separately dissolved in 4 x Solution I (Takara Bio, Japan) to a concentration of 30 $\mu$M. The resulting solutions were spotted on a DMSO-resistant coat glass for Matsunami DNA microarrays (an amino-modified oligo DNA-immobilized coat, type I; Matsunami Glass, Japan) using a spotter (GMS417 arrayer, Affymetrix) under a humidity environment of 50% to 60%.

(4) Hybridization

The labeled cDNA (1 $\mu$g) was dissolved in an antisense oligo cocktail (QIAGEN), the resulting solution was applied to the DNA chip covered by a Gap cover glass (Matsunami Glass), and hybridization was then carried out at 42°C for 16 hours. After hybridization, the DNA chip was washed successively with 2x SSC/0.1% SDS, 1x SSC, and 0.2x SSC.

(5) Determination of gene expression level

The DNA chip that had been subjected to hybridization in the above-described manner was scanned using the Agilent microarray scanner (Agilent) to obtain an image, and the fluorescent intensity was expressed numerically. The statistic procedures were carried out with reference to Speed, T., "Statistical analysis of gene expression microarray data," Chapman & Hall/CRC, and Causton, H. C. et al., "A beginner's guide Microarray gene expression data analysis," Blackwell publishing. Specifically, the data obtained by the image analysis following hybridization were converted into log values, which were then normalized by global normalization and were smoothed by LOWESS (locally weighted scatterplot smoother), and numerical correction was carried out by MAD scaling.

(6) Prediction scoring system

A variety of clinical information concerning all the patients was examined to perform survival analysis, and whether or not differences were observed in the years of life resulting from different clinical information was examined. For example, patients were divided into two groups: a group of patients who were diagnosed to have metastasis to organs other than the kidney at the time of surgery; and a group of patients who were not to diagnosed to have metastasis. The 2 groups were subjected to survival analysis, and the results of analysis were represented by charts by the Kaplan-Meier method (Fig. 1). In this case, significant difference ($p < 0.05$) was observed in the survival curve by the log-rank test between the group of patients who were diagnosed to have metastasis to organs other than the kidney at the time of surgery and the other group. This indicates that prognosis for the former group differs from that for the latter group and that the patients who were diagnosed to have metastasis would have relatively poor prognosis and the other patients would have good prognosis. Thus, influence of the prognosis for the group of patients classified in accordance with the occurrence of metastasis, i.e., the survival curve, on the model was further examined by the Cox proportional hazards model, concerning the expressed genes, in order to examine the significance of the degree of conformity between the gene expression level and the survival curve. As a result, 321 types of expressed genes that would be significantly advantageous for survival and 164 types of expressed genes that would be disadvantageous for survival were obtained with the use of the post-surgery survival curve as the indication (Table 1). Thus, these genes can be used to detect the prognosis for kidney cancer patients and/or the metastasis of kidney cancer.

These genes were used to prepare a discriminant using SVM loaded on the Genomic Profiler (Mitsui Knowledge Industry, Japan). All 31 samples were analyzed by this discriminant to predict the data. All specimens were subjected to analysis. The survivals 5 years after the surgery were used as an indication to compare the kidney cancer lesions from patients with good prognoses or poor prognoses and those from patients with poor prognoses, the data concerning the most expressed genes that would be advantageous for survival were analyzed, and gene expression assayed with the use of the polynucleotides as shown in SEQ ID NOS: 1 to 19 and 48 was examined. Thus, patients with good prognoses or poor prognoses were predicted at a probability of 96% or higher (Fig. 2).

Separately, all specimens were subjected to analysis. The survivals 5 years after the surgery were used as an indication to compare the kidney cancer lesions from patients with good prognoses or poor prognoses and those from patients with poor prognoses, the data concerning the expressed genes at high ranks that would be disadvantageous for survival were analyzed, and gene expression determined with the use of the polynucleotides as shown in SEQ ID NOS: 20 to 47, 49, and 50 was examined. Thus, patients with poor prognoses were predicted at a probability of 87% or higher (Fig. 2).

In addition to the probes used above, the other probes according to the present invention can also be used for predicting the prognosis for kidney cancer .

In the above-described identification of prognosis, the SVM that identifies the kidney cancer tissue from a patient with a good prognosis and the SVM that identifies the kidney cancer tissue from a patient with a poor prognosis were simultaneously applied to the gene expression levels of an analyte tissue, and the analysis was thus performed.

When the two discriminants simultaneously yielded the result that the given analyte tissue was a kidney cancer tissue from a patient with good prognosis, or when the two discriminants yielded the result that the analyte was a kidney cancer tissue from a patient with poor prognosis, the accuracy of each diagnosis was found to be significantly higher than the accuracy attained by conventional diagnostic methods comprising the use of a single discriminant.

<Example 2>

(1) Identification of blood plasma proteins in healthy persons and patients with kidney cancer

EDTA-added blood plasma components were obtained from 5 Japanese patients with kidney cancer at an age of 50s to 70s before kidney cancer extirpation and 1 month after kidney cancer extirpation, i.e., at the healthy state.

The blood plasma was filtered through a filter (pore size 0.22 $\mu$m) to remove contaminants, and the protein concentration was adjusted to 50 mg/ml. The resulting blood plasma was further diluted in 25 mM ammonium bicarbonate solution (pH 8.0) to the concentration of 12.5 mg/ml, and molecular weight fractionation was then carried out using a hollow fiber filter (Toray, Japan). The fractionated blood plasma sample (total amount 1.8 ml, comprising 250 $\mu$g (max) of proteins) was divided into 7 fractions by reversed-phase chromatography (the ProteomeLab® PF2D System (Beckman Coulter)), lyophilized, and then redissolved in 100 $\mu$l of the 25 mM ammonium bicarbonate solution (pH 8.0). This sample was digested with trypsin (1/50 volumes of the protein) at 37°C for 2 to 3 hours for peptidization. Each peptide fraction was further fractionated into 4 fractions on the ion-exchange column (KYA Technologies, Japan).

Each fraction was further fractionated on the reversed-phase column (KYA Technologies), and the eluted peptides were measured using an online-linked mass spectrometer Q-TOF Ultima (Micromass) in a survey scan mode. The resulting data was analyzed using the protein identification software MASCOT (Matrix Science), to perform exhaustive identification of proteins. As a result, about 3,500 types of proteins, which exhibit a MASCOT score of 40 or higher (the number of identified peptides: two or more), were identified from the blood plasma components before surgery and after surgery.

(2) Comparison of the expression of blood plasma proteins of kidney cancer patient before surgery and after surgery

The blood plasma proteins of a kidney cancer patient identified in Example 2 (1) were compared between before surgery and after surgery. Proteins that were not expressed after surgery but were expressed before surgery in 4 or more of 6 patients were found. These proteins were found to be the polypeptides as shown in SEQ ID NOS: 151 to 160, and 191 shown in Table 2, and were found to be useful as the so-called kidney cancer markers, for detecting kidney cancer. Table 2 shows a frequency of the expression in each of the patients. Among the kidney cancer bearing patients before surgery, such expression was detected in 4 or more of 6 patients (indicated by "+") (Table 4).

Thus, the kidney cancer can be detected by measuring the presence or amount of at least one of the above-described polypeptides using, for example, antibodies specific thereto.

Table 4

| SEQ ID NO | Protein No. | Gene Name | Pat 1 | Pat 2 | Pat 3 | Pat 4 | Pat 5 | Pat 6 |
|---|---|---|---|---|---|---|---|---|
| 151 | P54646 | AAK2 | - | + | + | + | + | + |
| 152 | Q8IW52 | SLK4 | - | - | + | + | + | + |
| 153 | Q8TC36 | SP4L | - | + | - | + | + | + |
| 154 | Q96SZ6 | C5P1 | + | - | + | + | - | + |
| 155 | Q16739 | CEGT | + | + | + | + | - | - |
| 156 | Q92185 | SI8A | + | + | - | - | + | + |
| 157 | P13861 | KAP2 | + | + | - | + | - | + |
| 158 | 060825 | F262 | + | + | - | + | + | - |
| 159 | Q99832 | TCPH | - | + | + | - | + | + |
| 160 | P29992 | GB11 | + | - | - | + | + | + |
| 191 | Q9H4Z3 | CT67 | + | - | + | - | + | + |
| Pat: Patient | | | | | | | | |

<Example 3>

(1) Identification of blood plasma proteins in healthy persons and patients with kidney cancer

EDTA-added blood plasma components were obtained from 7 Japanese patients with kidney cancer at an age of 50s to 70s before kidney cancer extirpation and 1 month after kidney cancer extirpation, i.e., at the healthy state.

The blood plasma was filtered through a filter (pore size 0.22 $\mu$m) to remove contaminants, and the protein concentration was adjusted to 50 mg/ml. The resulting blood plasma was further diluted in 25 mM ammonium bicarbonate solution (pH 8.0) to the concentration of 12.5 mg/ml, and molecular weight fractionation was then carried out using a hollow fiber filter (Toray, Japan). The fractionated blood plasma sample (total amount 1.8 ml, comprising 250 $\mu$g (max) of proteins) was divided into 7 fractions by reversed-phase chromatography (the ProteomeLab® PF2D System (Beckman Coulter)), lyophilized, and then redissolved in 100 $\mu$l of the 25 mM ammonium bicarbonate solution (pH 8.0). This sample was digested with trypsin (1/50 volumes of the protein) at 37°C for 2 to 3 hours for peptidization. Each peptide fraction was further fractionated into 4 fractions on the ion-exchange column (KYA Technologies, Japan).

Each fraction was further fractionated on the reversed-phase column (KYA Technologies), and the eluted peptides were measured using an online-linked mass spectrometer Q-TOF Ultima (Micromass) in a survey scan mode. The resulting data was analyzed using the protein identification software MASCOT (Matrix Science), to perform exhaustive identification of proteins. As a result, about 3,500 types of proteins, which exhibit a MASCOT score of 40 or higher (the number of identified peptides: two or more), were identified from the blood plasma components before surgery and after surgery.

(2) Comparison of the expression of blood plasma proteins of kidney cancer patient before surgery and after surgery

The blood plasma proteins of a kidney cancer patient identified in Example 3 (1) were compared between before surgery and after surgery. Proteins that were not expressed after surgery but were expressed before surgery in 3 or more of 7 patients were discovered. These proteins were found to be the polypeptides as shown in SEQ ID NOS: 161 to 182, 192, and 193 shown in Table 1, and were found to be useful as the so-called kidney cancer markers, for detecting kidney cancer. Table 5 shows a frequency of the expression in each of the patients. Among the kidney cancer bearing patients before surgery, such expression was detected in 3 or more of 7 patients (indicated by "+").

Thus, the kidney cancer can be detected by measuring the presence or amount of at least one, and preferably at least 3 to 5, of the above-described polypeptides using, for example, antibodies specific thereto.

Table 5

| SEQ ID NO | Protein No. | Gene Name | Pat 1 | Pat 2 | Pat 3 | Pat 4 | Pat 5 | Pat 6 | Pat 7 |
|---|---|---|---|---|---|---|---|---|---|
| 161 | Q16671 | AMHR2 | - | - | + | + | - | + | - |
| 162 | 095236 | APOL3 | + | - | + | - | - | + | - |
| 163 | Q8WXF8 | DEDD2 | - | - | - | + | + | + | - |
| 164 | P07686 | HEXB | + | - | - | + | - | - | + |
| 165 | P61978 | HNRPK | - | - | + | + | + | - | - |
| 166 | P52294 | KPNA1 | - | - | + | + | - | - | + |
| 167 | Q14847 | LASP1 | - | + | - | - | + | + | - |
| 168 | 043766 | LIAS | - | - | + | | + | + | - |
| 169 | Q8TD91 | MAGEC3 | - | + | - | + | - | - | + |
| 170 | Q14934 | NFATC4 | + | + | + | - | - | - | - |
| 171 | Q96PB7 | OLFM3 | + | - | - | + | - | - | + |
| 172 | Q9H1D9 | POLR3F | + | - | + | - | + | - | - |
| 173 | NP_066955 | PPP3CB | + | - | - | - | - | + | + |
| 174 | Q15637 | SF1 | + | + | - | - | - | - | + |
| 175 | Q9UI40 | SLC24A2 | + | + | + | - | - | - | - |
| 176 | 043511 | SLC26A4 | + | + | - | + | - | - | - |
| 177 | P46721 | SLC01A2 | - | + | - | + | + | - | + |
| 178 | P07951 | TPM2 | - | - | - | + | + | + | - |
| 179 | Q9Y5K5 | UCHL5 | - | + | + | - | + | - | - |
| 180 | Q16880 | UGT8 | + | + | - | - | + | - | - |
| 181 | P52738 | ZNF140 | + | - | + | - | - | - | + |
| 182 | Q96GC6 | ZNF274 | + | + | + | + | - | - | - |
| 192 | P08253 | MMP2 | - | + | - | + | + | + | - |
| 193 | P26842 | TNRSF7 | + | + | - | + | - | + | - |
| Pat: Patient | | | | | | | | | |

(3) Comparison of the expression of blood plasma proteins of kidney cancer patient before surgery and after surgery

The blood plasma proteins of 7 kidney cancer patients identified in (1) above were compared between before surgery and after surgery. Kidney cancer-specific proteins that were not expressed after surgery but were expressed before surgery were discovered by comparison of the same subject. Table 6 shows proteins that were observed commonly in 5 out of 7 patients. These proteins were found to be the polypeptides as shown in SEQ ID NOS: 183 to 190 and 194 to 197 shown in Table 6, and were found to be useful as the so-called kidney cancer markers, for detecting kidney cancer.

Table 6

| SEQ ID NO | Protein No. | Gene Name | Pat 1 | Pat 2 | Pat 3 | Pat 4 | Pat 5 | Pat 6 | Pat 7 |
|---|---|---|---|---|---|---|---|---|---|
| 183 | Q9UJV3 | MID2 | + | + | + | + | - | - | + |
| 184 | Q05469 | LIPE | + | + | - | + | - | + | + |
| 185 | Q9UBN7 | HDAC6 | - | + | + | + | + | + | + |
| 186 | Q99798 | ACO2 | + | + | + | - | + | - | + |

(continued)

| SEQ ID NO | Protein No. | Gene Name | Pat 1 | Pat 2 | Pat 3 | Pat 4 | Pat 5 | Pat 6 | Pat 7 |
|---|---|---|---|---|---|---|---|---|---|
| 187 | P51693 | APLP1 | + | - | + | + | - | + | + |
| 188 | P49757 | NUMB | + | + | + | + | - | + | - |
| 189 | Q14155 | ARHGEF7 | + | - | - | + | + | + | + |
| 190 | P50148 | GNAQ | + | - | - | + | + | + | + |
| 194 | 095263 | PDE8B | - | + | + | + | + | + | - |
| 195 | 075955 | FL0T1 | + | + | - | + | + | + | + |
| 196 | P06127 | CD5 | + | + | + | - | + | + | - |
| 197 | Q16610 | ECM1 | + | + | - | + | + | + | - |
| Pat: Patient | | | | | | | | | |

INDUSTRIAL APPLICABILITY

The present invention can provide a composition, kit, DNA chip, and method for detecting, diagnosing, and predicting metastasis of kidney cancer and/or for predicting the prognosis for kidney cancer patients, with high-specificity and high-sensitivity. Accordingly, the present invention is particularly useful in the pharmaceutical and medical industries.

**Claims**

1. A composition for detecting, identifying, or predicting the presence or metastasis of kidney cancer in a subject *in vitro* comprising one or more probes selected from the probes of the following group I and/or group II:

    group I: polynucleotides consisting of:

       (a) a polynucleotide consisting of a nucleotide sequence as shown in any of SEQ ID NOS: 1 to 10, 12 to 20, and 22 to 47, a mutant thereof, or a fragment comprising at least 15 continuous nucleotides thereof,
       (b) a polynucleotide comprising a nucleotide sequence as shown in any of SEQ ID NOS: 1 to 10, 12 to 20, and 22 to 47,
       (c) a polynucleotide consisting of a nucleotide sequence complementary to a nucleotide sequence as shown in any of SEQ ID NOS: 1 to 10, 12 to 20, and 22 to 47, a mutant thereof, or a fragment comprising at least 15 continuous nucleotides thereof,
       (d) a polynucleotide comprising a nucleotide sequence complementary to a nucleotide sequence as shown in any of SEQ ID NOS: 1 to 10, 12 to 20, and 22 to 47, and
       (e) a polynucleotide hybridizing under stringent conditions to any of the polynucleotides (a) to (d), or a fragment comprising at least 15 continuous nucleotides thereof; and

    group II: antibodies, fragments thereof or chemically modified derivatives thereof consisting of:

       (f) an antibody specifically binding to at least one of a polypeptide comprising an amino acid sequence encoded by a nucleotide sequence as shown in any of SEQ ID NOS: 1 to 10, 12 to 20, and 22 to 47 or a polypeptide having an amino acid sequence as shown in any of SEQ ID NOS: 101 to 110, 112 to 120, and 122 to 147, a mutant thereof, and a fragment thereof; a fragment of the antibody; or a chemically modified derivative of the antibody,
       (g) an antibody specifically binding to at least one of a polypeptide having an amino acid sequence as shown in any of SEQ ID NOS: 151, 153, and 155 to 160, a mutant thereof, and a fragment thereof, a fragment of the antibody, or a chemically modified derivative of the antibody, and
       (h) an antibody specifically binding to at least one of a polypeptide having an amino acid sequence as shown in any of SEQ ID NOS: 161 to 190, a mutant thereof, and a fragment thereof; a fragment of the antibody; or a chemically modified derivative of the antibody.

2. The composition according to claim 1, wherein each of the probes of group I and group II (f) is capable of detecting, identifying, or predicting the presence or metastasis of kidney cancer.

3.  The composition according to claim 1, wherein each of the probes of group II (g) and (h) is capable of detecting, identifying, or predicting the presence of kidney cancer.

4.  The composition according to claim 1, wherein the polynucleotide is DNA or RNA.

5.  The composition according to claim 1, wherein the fragments of group I are each a polynucleotide comprising at least 60 continuous nucleotides.

6.  The composition according to claim 1, wherein the fragments of group I are each a polynucleotide comprising a nucleotide sequence as shown in any of SEQ ID NOS: 51 to 60, 62 to 70, and 72 to 97 in the nucleotide sequence as shown in any of SEQ ID NOS: 1 to 10, 12 to 20, and 22 to 47 or a polynucleotide comprising a nucleotide sequence complementary thereto.

7.  The composition according to claim 1, wherein the fragments of group I are each a polynucleotide comprising a nucleotide sequence as shown in any of SEQ ID NOS: 51 to 60, 62 to 70, and 72 to 97 or a nucleotide sequence complementary thereto.

8.  The composition according to claim 1, which further comprises, as a probe, one or more polynucleotides selected from among a polynucleotide consisting of the nucleotide sequence as shown in any of SEQ ID NOS: 11, 21, and 48 to 50, a polynucleotide consisting of a complementary sequence thereof, a polynucleotide hybridizing under stringent conditions to said polynucleotide, or a fragment comprising at least 15 continuous nucleotides thereof, in addition to the probe or probes of group I.

9.  The composition according to claim 8, wherein the fragment is a polynucleotide comprising at least 60 continuous nucleotides.

10. The composition according to claim 8, wherein the fragment is a polynucleotide comprising the nucleotide sequence as shown in any of SEQ ID NOS: 61, 71, and 98 to 100 in the nucleotide sequence as shown in any of SEQ ID NOS: 11, 21, and 48 to 50 or a polynucleotide comprising a nucleotide sequence complementary thereto.

11. The composition according to claim 8, wherein the fragment is a polynucleotide comprising the nucleotide sequence as shown in any of SEQ ID NOS: 61, 71, and 98 to 100 or a nucleotide sequence complementary thereto.

12. The composition according to claim 1, which further comprises an antibody that binds specifically to at least one of a polypeptide as shown in any of SEQ ID NOS: 111, 121, and 148 to 150, a mutant of the polypeptide, and a fragment of the polypeptide; a fragment of the antibody; or a chemically modified derivative of the antibody, in addition to the probe or probes of group II (f).

13. The composition according to claim 1, which further comprises an antibody that binds specifically to at least one of a polypeptide comprising an amino acid sequence as shown in any of SEQ ID NOS: 152, 154, and 191, a mutant of the polypeptide, and a fragment of the polypeptide; a fragment of the antibody; or a chemically modified derivative of the antibody, in addition to the probe or probes of group II (g).

14. The composition according to claim 1, which further comprises an antibody that binds specifically to at least one of a polypeptide comprising an amino acid sequence as shown in any of SEQ ID NOS: 192 to 197, a mutant of the polypeptide, and a fragment of the polypeptide; a fragment of the antibody; or a chemically modified derivative of the antibody, in addition to the probe or probes of group II (h).

15. The composition according to claim 1, wherein the fragment of the polypeptide or a mutant thereof comprises an epitope having at least 7 amino acids.

16. The composition according to claim 1 above, wherein each of the antibodies is a polyclonal antibody, a monoclonal antibody, a synthetic antibody, a recombinant antibody, a polyspecific antibody, or a single-chain antibody.

17. The composition according to claim 1, which comprises at least two probes selected from the probes of group I or group II (f), (g), or (h) in combination.

18. A kit for detecting, identifying, or predicting the presence or metastasis of kidney cancer in a subject *in vitro,* comprising one or more probes selected from the probes of group I or group II (f), (g), or (h) as defined in claim 1.

19. The kit according to claim 18, which further comprises, as a probe, a polynucleotide consisting of the nucleotide sequence as shown in any of SEQ ID NOS: 11, 21, and 48 to 50, a polynucleotide consisting of a complementary sequence thereof, a polynucleotide hybridizing under stringent conditions to said polynucleotide, or a fragment comprising at least 15 continuous nucleotides thereof.

20. The kit according to claim 18, wherein the probe is a polynucleotide consisting of the nucleotide sequence as shown in any of SEQ ID NOS: 1 to 10, 11, 12 to 20, 21, 22 to 47, and 48 to 50, a polynucleotide consisting of a complementary sequence thereof, a polynucleotide hybridizing under stringent conditions to said polynucleotide, or a fragment comprising at least 15 continuous nucleotides thereof.

21. The kit according to claim 18, wherein the fragment of group I is a polynucleotide comprising at least 60 continuous nucleotides.

22. The kit according to claim 18, wherein the fragment of group I is a polynucleotide comprising the nucleotide sequence as shown in any of SEQ ID NOS: 51 to 60, 61, 62 to 70, 71, 72 to 97, and 98 to 100 and comprising at least 60 continuous nucleotides in the nucleotide sequence as shown in any of SEQ ID NOS: 1 to 10, 11, 12 to 20, 21, 22 to 47, and 48 to 50 or a polynucleotide comprising a nucleotide sequence complementary thereto.

23. The kit according to claim 18, wherein the fragment of group I is a polynucleotide comprising a nucleotide sequence as shown in any of SEQ ID NOS: 51 to 60, 61, 62 to 70, 71, 72 to 97, and 98 to 100 or a nucleotide sequence complementary thereto.

24. The kit according to claim 18, wherein the fragment of group I is a polynucleotide comprising a nucleotide sequence as shown in any of SEQ ID NOS: 51 to 60, 61, 62 to 70, 71, 72 to 97, and 98 to 100.

25. The kit according to claim 18, which comprises at least two or all polynucleotides each comprising a nucleotide sequence as shown in any of SEQ ID NOS: 51 to 60, 61, 62 to 70, 71, 72 to 97, and 98 to 100 or a complementary sequence of any thereof.

26. The kit according to claim 18, which further comprises an antibody that binds specifically to at least one of a polypeptide as shown in any of SEQ ID NOS: 111, 121, and 148 to 150, a mutant of the polypeptide, and a fragment of the polypeptide; a fragment of the antibody; or a chemically modified derivative of the antibody, in addition to a probe or probes of group II (f).

27. The kit according to claim 18, which further comprises an antibody that binds specifically to at least one of a polypeptide as shown in any of SEQ ID NOS: 152, 154, and 191, a mutant of the polypeptide, and a fragment of the polypeptide; a fragment of the antibody; or a chemically modified derivative of the antibody, in addition to a probe or probes of group II (g).

28. The kit according to claim 18, which further comprises an antibody that binds specifically to at least one of a polypeptide as shown in any of SEQ ID NOS: 192 to 197, a mutant of the polypeptide, and a fragment of the polypeptide; a fragment of the antibody; or a chemically modified derivative of the antibody, in addition to a probe or probes of group II (h).

29. The kit according to claim 18, wherein the probes are packaged in different containers, alone or in combination.

30. A DNA chip for detecting, identifying, or predicting the presence or metastasis of kidney cancer in a subject *in vitro,* comprising one or more probes selected from the probes of group I (a) to (e) as defined in claim 1.

31. The DNA chip according to claim 30, which further comprises a polynucleotide consisting of the nucleotide sequence as shown in any of SEQ ID NOS: 11, 21, and 48 to 50, a mutant thereof, and/or a fragment thereof.

32. The DNA chip according to claim 30, which comprises at least two or all of the polynucleotides comprising a nucleotide sequence as shown in any of SEQ ID NOS: 51 to 60, 61, 62 to 70, 71, 72 to 97, and 98 to 100 or complementary sequences thereto.

33. A method for detecting, identifying, or predicting the presence or metastasis of kidney cancer *in vitro,* comprising using a probe or probes selected from the probes of group I and/or group II (f), (g), and (h) as defined in claim 1, to measure *in vitro* the presence, existing amount, or expression level of one or more kidney cancer-associated target nucleic acids in a biological sample from a subject.

34. The method according to claim 33, wherein the measurement is carried out using a DNA chip.

35. The method according to claim 33, wherein the presence or metastasis of kidney cancer is detected, identified, or predicted using changes compared with a control sample as an indication.

36. The method according to claim 33, wherein the measurement is carried out by an immunological method.

37. The method according to claim 36, wherein the measurement by the immunological method is carried out using an antibody of group II (f), (g), or (h), a fragment thereof, or a chemically modified derivative thereof.

38. The method according to claim 37, wherein the antibody, fragment, or chemically modified derivative is labeled.

39. The method according to claim 33, wherein the biological sample is a kidney tissue or a kidney cell, blood, plasma, serum, or urine.

40. A method for detecting, identifying, or predicting the presence or metastasis of kidney cancer *in vitro,* comprising measuring *in vitro* the presence, existing amount, or expression level of one or more kidney cancer-associated target nucleic acids in a biological sample from a subject using the composition according to claim 1, the kit according to claim 18, or the DNA chip according to claim 30.

41. A method for predicting *in vitro* a patient's prognosis and/or metastasis of kidney cancer by comparing the expression level of a target nucleic acid in a biological sample of kidney cancer cells obtained from a subject with that of the target nucleic acid in kidney cancer cells obtained from a patient population with poor prognosis and/or that of the target nucleic acid in kidney cancer cells obtained from a patient population with good prognosis using a probe or probes selected from group I as defined in claim 1, or the composition of claim 1, the kit of claim 18, or the DNA chip of claim 30 comprising the probe or probes, wherein the target nucleic acid can be detected by the polynucleotide contained in the composition, kit, or DNA chip or a mutant or fragment of such polynucleotide, and, when the expression level of the target nucleic acid in the subject is different from that in the patient population with good prognosis and/or that in the patient population with poor prognosis, the subject is identified to have a poor or good prognosis and/or the metastasis of kidney cancer is determined to have or have not occurred.

42. The method according to claim 41, which involves the use of a DNA chip.

43. The method according to claim 41, which comprises the steps of:

   (1) measuring *in vitro* expression levels of target nucleic acids in a plurality of biological samples that are known to be kidney cancer cells obtained from a patient with a poor prognosis and/or kidney cancer cells obtained from a patient with a good prognosis using a probe or probes selected from group I as defined in claim 1, the composition of claim 1, the kit of claim 18, or the DNA chip of claim 30 comprising the probe or probes;
   (2) preparing a discriminant, a support vector machine, using, as training samples, the expression levels of the target nucleic acids as determined in step (1);
   (3) measuring *in vitro* expression levels of the target nucleic acids in a biological sample obtained from the kidney cancer of the subject in the same manner as in step (1); and
   (4) assigning the expression levels of the target nucleic acids identified in step (3) to the discriminant prepared in step (2) and, based on the results obtained with the use of the discriminant, predicting the prognosis for a subject and/or determining whether or not kidney cancer of the subject is metastatic or nonmetastatic, provided that the target nucleic acids can be detected by the polynucleotides, mutants thereof, or fragments thereof, contained in the composition, kit, or DNA chip.

44. Use of a probe or probes selected from the probes of group I and/or group II as defined in claim 1, or of the composition of claim 1, the kit of claim 18, or the DNA chip of claim 30 comprising the probe or probes, in detecting, identifying, or predicting *in vitro* the presence or metastasis of kidney cancer in a biological sample from a subject.

# Fig. 1

# Fig. 2

Prediction of prognosis for kidney cancer patient/number of genes
necessary for detecting metastasis of kidney cancer

## INTERNATIONAL SEARCH REPORT

| International application No. |
| --- |
| PCT/JP2006/317380 |

A. CLASSIFICATION OF SUBJECT MATTER
*C12N15/00*(2006.01)i, *C12N15/09*(2006.01)i, *C12Q1/68*(2006.01)i, *G01N33/53*
(2006.01)i, *G01N33/574*(2006.01)i

According to International Patent Classification (IPC) or to both national classification and IPC

B. FIELDS SEARCHED

Minimum documentation searched (classification system followed by classification symbols)
C12N15/00, C12N15/09, C12Q1/68, G01N33/53, G01N33/574

Documentation searched other than minimum documentation to the extent that such documents are included in the fields searched
Jitsuyo Shinan Koho        1922-1996   Jitsuyo Shinan Toroku Koho   1996-2006
Kokai Jitsuyo Shinan Koho   1971-2006   Toroku Jitsuyo Shinan Koho   1994-2006

Electronic data base consulted during the international search (name of data base and, where practicable, search terms used)
BIOSIS/MEDLINE/WPIDS(STN), CAplus(STN), GenBank/EMBL/DDBJ/GeneSeq,
SwissProt/PIR/Geneseq, JMEDPlus(JDream2), JST7580(JDream2),
JSTPlus(JDream2)

C. DOCUMENTS CONSIDERED TO BE RELEVANT

| Category* | Citation of document, with indication, where appropriate, of the relevant passages | Relevant to claim No. |
| --- | --- | --- |
| X | Masahiro YAO et al., "Idenshi Hatsugen Kaiseki ni Motozuku Tanmei Saibo Jingan no Yogo Yosoku Model no Kochiku", Nihon Gan Gakkai Gakujutsu Sokai Kiji, 64th, 15 August, 2005 (15.08.05), page 291, PA2-0655 | 1-44 |
| X | Yasuo AWAKURA et al., "DNA Chip o Mochiita Tanmei Jinsaibogan ni Okeru Yogo Yosoku Marker no Tansaku", Nihon Gan Gakkai Gakujutsu Sokai Kiji, 64th, 15 August, 2005 (15.08.05), pages 394 to 395, PP2-0951 | 1-44 |
| X | Koichi SHIOI et al., "Tanmei Saibo Jingan ni Okeru Fibronectin Hatsugen to Rinsho Byori·Yogo tono Kankei", The Japanese Cancer Association Sokai Kiji, 63rd, 25 August, 2004 (25.08.04), pages 361 to 362, P-1060 | 1-44 |

☒ Further documents are listed in the continuation of Box C.     ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 22 September, 2006 (22.09.06) | 03 October, 2006 (03.10.06) |

| Name and mailing address of the ISA/ | Authorized officer |
| --- | --- |
| Japanese Patent Office | |
| Facsimile No. | Telephone No. |

Form PCT/ISA/210 (second sheet) (April 2005)

<table>
<tr><td colspan="2" align="center">**INTERNATIONAL SEARCH REPORT**</td><td>International application No.<br>PCT/JP2006/317380</td></tr>
</table>

**C (Continuation). DOCUMENTS CONSIDERED TO BE RELEVANT**

| Category* | Citation of document, with indication, where appropriate, of the relevant passages | Relevant to claim No. |
|---|---|---|
| X | Koichi SHIOI et al., "Tanmei Saibo Jingan ni Okeru VCAM1 no Hatsugen to Rinsho Byori·Yogo tono Kankei", Nihon Gan Gakkai Gakujutsu Sokai Kiji, 64th, 15 August, 2005 (15.08.05), page 395, PP2-0953 | 1-44 |
| X | Masahiro YAO et al., "Jinsaibogan deno ADFP Hatsugen to Rinsho Byori·Yogo tono Kankei", The Japanese Cancer Association Sokai Kiji, 63rd, 25 August, 2004 (25.08.04), page 360, P-1053 | 1-44 |
| X | Yasuyoshi MIYATA et al., "Jinsaibogan Kanja ni Okeru acute-phase reactants, BFP Oyobi IAP ni Tsuite no Rinshoteki Kento", The Japanese Journal of Urology Vol.92 No.2, 20 February, 2001 (20.02.01), 189(237), MP-39 | 1-44 |
| P,X | Yasuo AWAKURA et al., "DNA Chip o Mochiita Tanmei Jinsaibogan ni Okeru Yogo Marker no Tansaku", The 94th Annual Meeting of the Japanese Urological Association, 10 March, 2006 (10.03.06), Vol.97, No.2, 163(231), APP-017 | 1-44 |
| A | Lam JS et al.,Tissue array-based predictions of pathobiology, prognosis, and response to treatment for renal cell carcinoma therapy., Clin Cancer Res. 2004 Sep 15, Vol.10(18 Pt 2): 6304S-9S. | 1-44 |
| A | JP 2004-531713 A (Merck Patent GmbH), 14 October, 2004 (14.10.04), & EP 1373900 A & WO 02/082076 A2 & CA 2442957 A & BR 208603 A & CZ 20032787 A & HU 303749 A & SK 12872003 A & PL 363009 A & CN 1630819 A | 1-44 |
| A | WO 02/096943 A1 (Asahi Kasei Corp.), 05 December, 2002 (05.12.02), SEQ ID NO 120 & US 2003/0092616 A1 | 1-44 |

Form PCT/ISA/210 (continuation of second sheet) (April 2005)

**INTERNATIONAL SEARCH REPORT**

International application No.

PCT/JP2006/317380

Continuation of B. FIELDS SEARCHED
  Electronic data base consulted during the international search (name of data base and, where practicable, search terms used)

Igaku·Yakugaku Yokoshu Zenbun Database

Form PCT/ISA/210 (extra sheet) (April 2005)

## INTERNATIONAL SEARCH REPORT

International application No.

PCT/JP2006/317380

**Box No. II      Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:
  (See extra sheet.)

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

| **INTERNATIONAL SEARCH REPORT** | International application No. |
|---|---|
| | PCT/JP2006/317380 |

Continuation of Box No.III of continuation of first sheet(2)

The matter common to claims 1 to 44 resides in a probe which recognizes a specific sequence to thereby detect kidney cancer *in vitro* in a subject.

However, the above common matter was publicly known on the priority date of the present case (see, if necessary, documents 3 to 6).

As a result, the common matter as described above falls within the category of prior art and, therefore, this common matter cannot be considered as a special technical feature.

Such being the case, the inventions as set forth in the claims are classified into those having special technical features respectively in the base sequences represented by SEQ ID NOS:1 to 10, 12 to 20 and 22 to 47 and the amino acid sequences represented by SEQ ID NOS:151, 153, 155 to 160 and 161 to 190.

Although the claims have 83 general inventive concepts corresponding to the SEQ ID NOS. as cited above, there is seemingly no novel special technical feature common to these general inventive concepts. Thus, it appears that the present case does not comply with the requirement of unity of invention (Rules for the Enforcement of the Law, Article 13 (PCT Rules 13.1, 13.2 and 13.3)).

3. Koichi SHIOI et al., "Tanmei Saibo Jingan ni Okeru Fibronectin Hatsugen to Rinsho Byori·Yogo tono Kankei", The Japanese Cancer Association Sokai Kiji, 63rd, 25 August, 2004 (25.08.04), p.361-362, P-1060
4. Koichi SHIOI et al., "Tanmei Saibo Jingan ni Okeru VCAM1 no Hatsugen to Rinsho Byori·Yogo tono Kankei", Nihon Gan Gakkai Gakujutsu Sokai Kiji, 64th, 15 August, 2005 (15.08.05), p.395, PP2-0953
5. Masahiro YAO et al., "Jinsaibogan deno ADFP Hatsugen to Rinsho Byori·Yogo tono Kankei", The Japanese Cancer Association Sokai Kiji, 63rd, 25 August, 2004 (25.08.04), p.360, P-1053
6. Yasuyoshi MIYATA et al., "Jinsaibogan Kanja ni Okeru acute-phase reactants, BFP Oyobi IAP ni Tsuite no Rinshoteki Kento", The Japanese Journal of Urology vol.92, No.2, 20 February, 2001 (20.02.01), MP-39

Form PCT/ISA/210 (extra sheet) (April 2005)

## REFERENCES CITED IN THE DESCRIPTION

**Patent documents cited in the description**

- WO 2005024603 A
- WO 2004042077 A
- WO 2004048933 A
- US 6303765 B
- JP 2005520536 A
- JP 2005507997 A
- WO 200206339 A
- JP 2004518402 A
- WO 200524603 A


**Non-patent literature cited in the description**

- **LATIF, F. et al.** *Science,* 1993, vol. 260, 1317-1320
- **MAXWELL, P. et al.** *Nature,* 1999, vol. 399, 271-275
- **NAGASE, Y. et al.** *the Japanese Journal of Urology,* 1991, vol. 82, 1781-1789
- **LEIN, M. et al.** *International Journal of Cancer,* 2000, vol. 85, 801-804
- **NAKATSUJI, T.** *Clinical and Experimental Medicine,* 2003, vol. 2, 192-196
- **MIYAKE, H. et al.** *Cancer Research,* vol. 56, 2440-2445
- **KITAGAWA, Y. et al.** *Journal of Urology,* 1999, vol. 162, 905-909
- **FREEMAN, M. R. et al.** *Cancer Research,* 1989, vol. 49, 6221-6225
- **KARLIN, S. et al.** *Proceedings of the National Academic Sciences, U.S.A.,* 1993, vol. 90, 5873-5877, http://www.ncbi.nlm.nih.gov/BLAST
- **KARLIN, S. et al.** *Proceedings of the National Academic Sciences, U.S.A.,* 1993, vol. 90, 5873-5877
- **ALTSCHUL, S. F. et al.** *Journal of Molecular Biology,* 1990, vol. 215, 403-410
- **PEARSON, W. R. et al.** *Proceedings of the National Academic Sciences, U.S.A.,* 1988, vol. 85, 2444-2448
- *BMJ,* 1998, vol. 317, 1572
- **BRAIS, B. et al.** *Nature genetics,* 1998, vol. 18, 164-167
- **UNOKI, M. et al.** *Oncogene,* 2001, vol. 20, 4457-4465
- **TOMASETTO, C. et al.** *EMBO Journal,* 1990, vol. 9, 407-414
- **METHOT, N. et al.** *Journal of Biological Chemistry,* 1997, vol. 272, 1110-1116
- **WAGNER, A. et al.** *Proceedings of the National Academic Sciences, U. S.A.,* 1992, vol. 89, 3111-3115
- **NAGASE, T. et al.** *DNA Research,* 1997, vol. 4, 141-150
- **ROSENFELD, M. et al.** *Annals of Neurology,* 1993, vol. 33, 113-120
- **HATEBOER, G et al.** *EMBO Journal,* 1995, vol. 14, 3159-3169
- **BANERJI, J. et al.** *Proceedings of the National Academic Sciences, U.S.A.,* 1990, vol. 87, 2374-2378
- **HOOPER, J. D. et al.** *Genomics,* 2000, vol. 66, 113-118
- **TAKEI, Y. et al.** *Journal of Biological Chemistry,* 1998, vol. 273, 22177-22180
- **SOLOMON, E. et al.** *Proceedings of the National Academic Sciences, U.S.A.,* 1985, vol. 82, 3330-3334
- **FUKUNAGA, R. et al.** *EMBO Journal,* 1997, vol. 16, 1921-1933
- **YU, J. et al.** *Molecular Cell,* 2001, vol. 7, 673-682
- **SUPERTI-FRUGA, A. et al.** *Genomics,* 1993, vol. 17, 463-467
- **DU, M. et al.** *Biochemical Biophysical Research Communication,* 1997, vol. 235, 779-783
- **GAUDET, S. et al.** *Proceedings of the National Academic Sciences, U.S.A.,* 2000, vol. 97, 5167-5172
- **STURANY, S. et al.** *Journal of Biological Chemistry,* 2001, vol. 276, 3310-3318
- **BADER, B. et al.** *EMBO Journal,* 1986, vol. 5, 1865-1875
- **BEAUSOLEIL, S. A. et al.** *Proceedings of the National Academic Sciences, U.S.A.,* 2004, vol. 101, 12130-12135
- **KESTILA, M. et al.** *Molecular Cell,* 1998, vol. 1, 575-582
- **KOIKE, G et al.** *Biochemical Biophysical Research Communication,* 1994, vol. 203, 1842-1850

- **LOVENBERG, T. W. et al.** *Proceedings of the National Academic Sciences, U.S.A.,* 1993, vol. 90, 2184-2188
- **DANIELSON, K. G et al.** *Genomics,* 1993, vol. 15, 146-160
- **VISCONTI, P. E. et al.** *Genomics,* 2001, vol. 77, 163-170
- **MATSUDA, L.A. et al.** *Nature,* 1990, vol. 346, 561-564
- **XIAO, B. et al.** *Neuron,* 2001, vol. 21, 707-716
- **MARCHESE, A. et al.** *Genomics,* 1994, vol. 23, 609-618
- **OTA, T. et al.** *Nature,* 2004, vol. 36, 40-45
- **BANERJI, S. et al.** *Journal of Cellular Biology,* 1999, vol. 144, 1789-801
- **STRAUSBERG, R. L. et al.** *Proceedings of the National Academic Sciences, U.S.A.,* 2002, vol. 99, 16899-16903
- **BURGESON, R. E. et al.** *Proceedings of the National Academic Sciences, U.S.A.,* 2002, vol. 73, 2579-2583
- **NOMURA, N. L. et al.** *DNA Research,* 1994, vol. 1, 223-229
- **SMITH, A. N. et al.** *Nature Genetics,* 2000, vol. 26, 71-75
- **CHAKRAVARTI, A. et al.** *Proceedings of the National Academic Sciences, U.S.A.,* 2002, vol. 99, 4755-4756
- **CHAN, J. et al.** *Oncogene,* 1991, vol. 6, 1057-1061
- **SU, L. et al.** *Genes Development,* 1993, vol. 7, 735-748
- **TAANMAN, J-W. et al.** *Nucleic Acids Research,* 1989, vol. 17, 1766
- **OPPENHEIM, F. G. et al.** *Biochemistry,* 1971, vol. 10, 4233-4238
- **ADRA, C. N. et al.** *Genomics,* 1996, vol. 35, 328-337
- **PIERRAT, B. et al.** *Journal of Biological Chemistry,* 1998, vol. 273, 29661-29671
- **EICHMULLER, S. et al.** *Proceedings of the National Academic Sciences, U.S.A.,* 2001, vol. 98, 629-634
- **ABRAHAM, J. et al.** *Science,* 1986, vol. 233, 545-548
- **MIYAKE, H. et al.** *Cancer Research,* 1996, vol. 56, 2440-2445
- **SATO, H. et al.** *Nature,* 1994, vol. 370, 61-65
- **YANG-FENG, T. L. et al.** *Citogenetic Cell Genetics,* 1985, vol. 40, 784
- **AUSUBEL et al.** Current Protocols in Molecular Biology. John Willey & Sons, 1993
- **SAMBROOK et al.** Molecular Cloning: A Laboratory Manual. Cold Spring Harbor Laboratory Press, 1989
- **SAMBROOK, J. ; RUSSEL, D.** Molecular Cloning, A LABORATORY MANUAL. Cold Spring Harbor Laboratory Press, 15 January 2001, vol. 1, 7.42-7.45
- MOLECULAR CLONING, A LABORATORY MANUAL. vol. 2, 8.9-8.17
- Monoclonal Antibodies, Hybridomas: A New Dimension in Biological Analyses. Plenum Press, 1980
- **J. B. LAMTURE et al.** *Nucleic. Acids. Research,* 1994, vol. 22, 2121-2125
- **Z. GUO et al.** *Nucleic. Acids. Research,* 1994, vol. 22, 5456-5465
- **G. YERSHOV et al.** *Proceedings of the National Academic Sciences, U.S.A.,* 1996, vol. 94, 4913
- **SOSNOWSKI et al.** *Proceedings of the National Academic Sciences, U.S.A.,* 1997, vol. 94, 1119-1123
- The Nature of Statistical Leaning Theory. Springer, 1995
- Pataan ninshiki to gakushu no toukeigaku. **HIDEKI ASOU et al.** Toukei kagaku no furontia. Iwanami Shoten Publishers, 11 April 2003, vol. 6, 107-138
- Current protocols in Protein Sciences. John Wiley & Sons Inc, 1995
- Current protocols in Immunology. John Wiley & Sons Inc, 2001